# EUROPEAN PATENT APPLICATION

(11) **EP 3 915 976 A1**
(43) Date of publication of application: **01.12.2021**
(21) Application number: 20744803.6
(22) Date of filing: 08.01.2020
(51) Int. Cl.: C07D 217/26, C07D 401/04, C07D 471/16, C07D 471/22

(54) **COMPOUND AND METHOD FOR PRODUCING COMPOUND**

(30) Priority: 23.01.2019 JP 2019009079
(71) Applicant: FUJIFILM Corporation, Tokyo 106-8620 (JP)
(72) Inventor: NAKAMURA Koki, kami-gun, Kanagawa 258-8577 (JP); TASAKI Yasutaka, kami-gun, Kanagawa 258-8577 (JP); TAKAHASHI Motomasa, kami-gun, Kanagawa 258-8577 (JP)
(74) Representative: HGF
(86) International application number: PCT/JP2020/000247
(87) International publication number: WO 2020/153121

(57) **Abstract**

The present invention provides an intermediate compound for easily producing a compound having an azaperylene skeleton. Another object of the present invention is to provide a method for easily producing a compound having an azaperylene skeleton.

The compound of the present invention is a compound represented by Formula (I), or a compound represented by Formula (II).

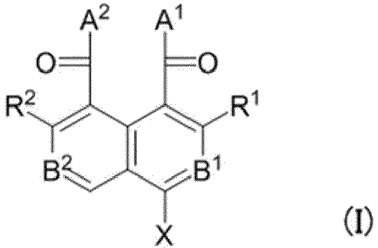

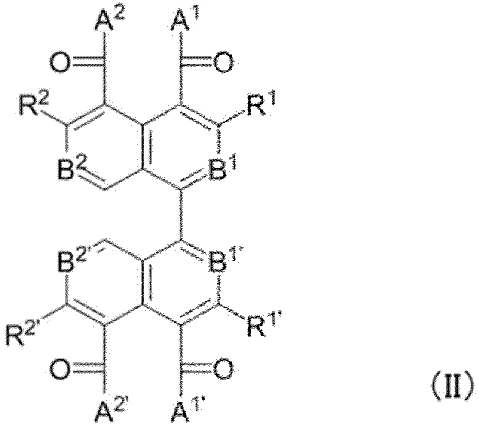

## Description

### BACKGROUND OF THE INVENTION

### 1. Field of the Invention

The present invention relates to a compound and a method for producing a compound.

### 2. Description of the Related Art

Since it is possible to be made lighter, cheaper, and more flexible, as a device which uses a logic circuit including a field effect transistor (FET), radio frequency identifier (RFID; RF tag), and memory, which are used in liquid crystal displays and organic electric luminescence (EL) displays, an organic thin film transistor (organic TFT) having an organic semiconductor film (organic semiconductor layer) has been used.

As a compound for forming such an organic semiconductor film, JP2018-6745A discloses an organic semiconductor compound having an azaperylene skeleton.

### SUMMARY OF THE INVENTION

The present inventors have studied a method for producing a compound having an azaperylene skeleton disclosed in JP2018-6745A, and have found that there is room for improvement in the ease of the production method.

Therefore, an object of the present invention is to provide an intermediate compound for easily producing a compound having an azaperylene skeleton.

Another object of the present invention is to provide a method for easily producing a compound having an azaperylene skeleton.

As a result of intensive studies on the above-described objects, the present inventors have found that the above-described objects can be achieved by the following configurations, and have completed the present invention.

[1] A compound represented by Formula (I) described later.
[2] The compound according to [1],
   in which A¹ and A² each independently represent -OR³, or are integrated to form -O- or -N(R⁶)-.
[3] The compound according to [1] or [2],
   in which R¹ and R² represent hydrogen atoms.
[4] A compound represented by Formula (II) described later.
[5] The compound according to [4],
   in which A¹, A², A^{1'}, and A^{2'} each independently represent -OR³, or A¹ and A², and A^{1'} and A^{2'} are each independently integrated to form -O- or -N(R⁶)-.
[6] The compound according to [4] or [5],
   in which R¹, R², R^{1'}, and R^{2'} represent hydrogen atoms.
[7] The compound according to any one of [4] to [6],
   in which the compound represented by Formula (II) is a compound represented by any one of Formula (II-1), (II-2), (II-3), (II-4), (II-5), (II-6), (II-7), or (II-8) described later.
[8] A method for producing a compound, comprising:
   a step of ring-closing the compound represented by Formula (II) according to any one of [4] to [7] to produce an azaperylene compound represented by Formula (IV) described later.
[9] The production method according to [8],
   in which the compound represented by Formula (II) is ring-closed in the presence of a base.
[10] The production method according to [8] or [9], further comprising:
   a step of reacting a compound represented by Formula (I) described later with a compound represented by Formula (III) described later in the presence of a transition metal catalyst to produce the compound represented by Formula (II) described later.
[11] A method for producing a compound, comprising:
   a step of reacting a compound represented by Formula (I) described later with a compound represented by Formula (III) described later in the presence of a transition metal catalyst to produce the compound represented by Formula (II) according to any one of [4] to [6].
[12] The production method according to [10] or [11],
   in which, in a case where X in Formula (I) represents a leaving group, X' in Formula (III) represents a hydrogen atom or an atomic group having a metal atom or a metalloid atom, which is bonded to a carbon atom in an azanaphthalene ring, and
   in a case where X in Formula (I) represents an atomic group having a metal atom or a metalloid atom, which is bonded to a carbon atom in an azanaphthalene ring, X' in Formula (III) represents a leaving group.

As shown below, according to the present invention, it is possible to provide an intermediate compound for easily producing an organic semiconductor compound having an azaperylene skeleton.

In addition, according to the present invention, it is possible to provide a method for easily producing an organic semiconductor compound having an azaperylene skeleton.

### BRIEF DESCRIPTION OF THE DRAWINGS

Fig. 1 is a schematic cross-sectional view showing a structure of a bottom gate-bottom contact type organic thin film transistor which is an example of an organic thin film transistor.
Fig. 2 is a schematic cross-sectional view showing a structure of a bottom gate-top contact type organic thin film transistor which is another example of the organic thin film transistor.
Fig. 3A is a schematic view showing an example of a method of forming an organic semiconductor film of the organic thin film transistor.
Fig. 3B is a schematic view showing an example of a method of forming an organic semiconductor film of the organic thin film transistor.
Fig. 3C is a schematic view showing an example of a method of forming an organic semiconductor film of the organic thin film transistor.
Fig. 4A is a schematic view showing another example of the method of forming an organic semiconductor film of the organic thin film transistor.
Fig. 4B1 is a schematic view showing another example of the method of forming an organic semiconductor film of the organic thin film transistor.

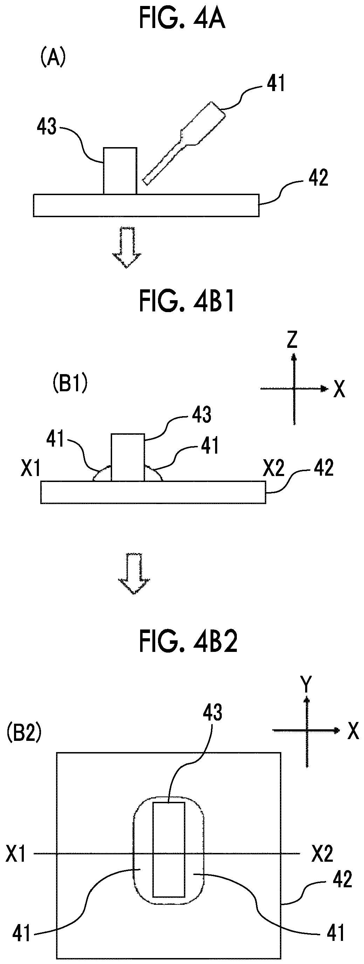

Fig. 4B2 is a schematic view showing another example of the method of forming an organic semiconductor film of the organic thin film transistor.

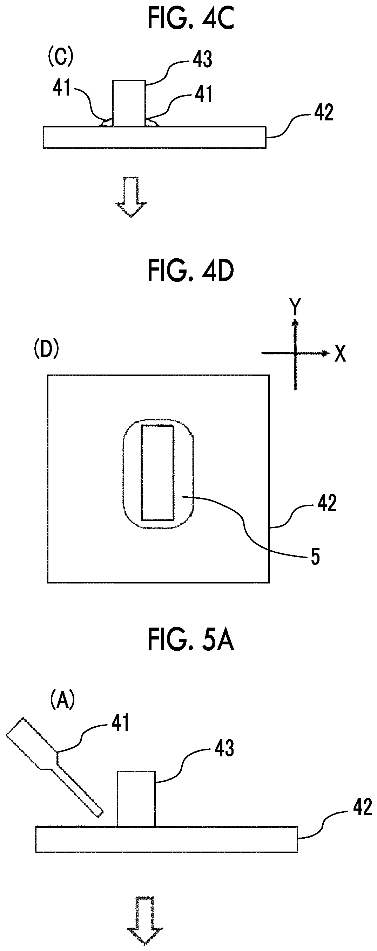

Fig. 4C is a schematic view showing another example of the method of forming an organic semiconductor film of the organic thin film transistor.
Fig. 4D is a schematic view showing another example of the method of forming an organic semiconductor film of the organic thin film transistor.
Fig. 5A is a schematic view showing still another example of a method of forming an organic semiconductor film of the organic thin film transistor.

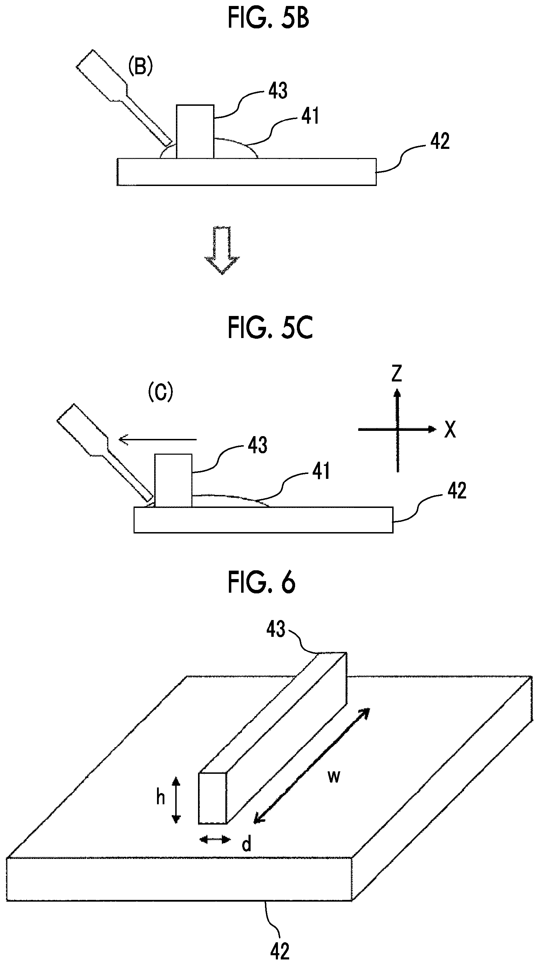

Fig. 5B is a schematic view showing still another example of the method of forming an organic semiconductor film of the organic thin film transistor.
Fig. 5C is a schematic view showing still another example of the method of forming an organic semiconductor film of the organic thin film transistor.
Fig. 6 is a schematic view showing an example of a substrate and a member used in the method of forming an organic semiconductor film of the organic thin film transistor.

### DESCRIPTION OF THE PREFERRED EMBODIMENTS

Hereinafter, the present invention will be described in detail. In the present specification, the numerical range expressed by using "to" means a range including numerical values described before and after "to" as a lower limit value and an upper limit value.

In the present specification, unless otherwise specified, the "aliphatic hydrocarbon group", "alkyl group", "alkenyl group", and "alkynyl group" include those having a linear, branched, or cyclic structure.

The present invention relates to a compound represented by Formula (I) described later and a compound represented by Formula (II) described later, which are intermediates useful for producing an azaperylene compound represented by Formula (IV) described later; a method for producing an azaperylene compound represented by Formula (IV) by ring-closing the compound represented by Formula (II); and a method for producing a compound represented by Formula (II) by ring-closing the compound represented by Formula (I).

The present inventors have studied the method for producing a compound having an azaperylene skeleton, which is disclosed in JP2018-6745A, and have found that a compound having a specific anthraquinone skeleton is used as an intermediate, and this intermediate compound is insoluble in an organic solvent. As a result, the present inventors have found that there is room for improvement in this production method, and by using, as the intermediate, a compound represented by Formula (I) described later and a compound represented by Formula (II) described later, a compound having an azaperylene skeleton can be more easily produced.

More specifically, compared with the intermediate compound having an anthraquinone skeleton disclosed in JP2018-6745A, since both the compound represented by Formula (I) and the compound represented by Formula (II) have excellent solubility in an organic solvent, an amount of solvent used for synthesizing the compound represented by Formula (IV) having an azaperylene skeleton can be reduced.

In addition, in a case of synthesizing the compound represented by Formula (IV) using the compound represented by Formula (I) and the compound represented by Formula (II), unlike the case of using an intermediate compound having an anthraquinone skeleton, since it is easy to change, in the synthesis process, the position and number of nitrogen atoms constituting the azaperylene skeleton, and the position, number, and type of substituents, it has an advantage that various kinds of compounds can be produced without major changes from the basic synthesis method, and the degree of freedom in designing a target compound is high.

Hereinafter, the azaperylene compound represented by Formula (IV), the compound represented by Formula (I), and the compound represented by Formula (II) are also described as "specific azaperylene compound", "intermediate I", and "intermediate II", respectively.

### [Compound (intermediate I) represented by Formula (I)]

First, the intermediate I used for producing the specific azaperylene compound will be described. The intermediate I is a compound represented by Formula (I).

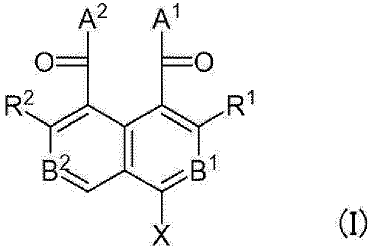

A¹ and A² each independently represent -OR³ or -NR⁴R⁵. R³ to R⁵ each independently represent a hydrogen atom, an aliphatic hydrocarbon group which may have a substituent, an aromatic hydrocarbon group which may have a substituent, or a heterocyclic group which may have a substituent.

In addition, A¹ and A² may be integrated to form -O- or -N(R⁶)-. In other words, A¹ and A² may be bonded to each other to form -O- or -N(R⁶)-. R⁶ represents an aliphatic hydrocarbon group which may have a hydrogen atom or a substituent, an aromatic hydrocarbon group which may have a substituent, or a heterocyclic group which may have a substituent.

The aliphatic hydrocarbon group which may have a substituent, represented by R³ to R⁶, may be any one of an alkyl group, an alkenyl group, or an alkynyl group.

The number of carbon atoms in the aliphatic hydrocarbon group is not particularly limited, but in a case of a linear or branched aliphatic hydrocarbon group, the number of carbon atoms in the linear or branched aliphatic hydrocarbon group is preferably 1 to 30 and more preferably 1 to 20. In addition, in a case of a cyclic aliphatic hydrocarbon group, the number of carbon atoms in the cyclic aliphatic hydrocarbon group is preferably 3 to 30 and more preferably 3 to 20.

Examples of the alkyl group represented by R³ to R⁶ include a methyl group, an ethyl group, a propyl group, a 2-methylpropyl group, a butyl group, an amyl group, a pentyl group, a 2,2-dimethylpropyl group, a hexyl group, a heptyl group, an octyl group, a nonyl group, a decyl group, an undecyl group, a dodecyl group, a tridecyl group, a tetradecyl group, a pentadecyl group, a 2,6-dimethyloctyl group, an icosyl group, a 2-decyltetradecyl group, a 2-hexyldodecyl group, a 2-ethyloctyl group, a 2-decyltetradecyl group, a 2-butyldecyl group, a 1-octylnonyl group, a 2-ethyloctyl group, a 2-octyldecyl group, a 2-octyldodecyl group, a 7-hexylpentadecyl group, a 2-octyltetradecyl group, a 2-ethylhexyl group, a cyclopropyl group, a cyclobutyl group, a cyclopentyl group, a cyclohexyl group, and an adamantyl group.

Examples of the alkenyl group represented by R³ to R⁶ include a vinyl group, an allyl group, a 2-butenyl group, a 1-pentenyl group, and a 4-pentenyl group.

Examples of the alkynyl group represented by R³ to R⁶ include an ethynyl group, a propargyl group, and a 1-pentynyl group.

The number of carbon atoms in the aromatic hydrocarbon group (hereinafter, also referred to as an "aryl group") represented by R³ to R⁶ is not particularly limited, but is preferably 6 to 30, more preferably 6 to 20, and still more preferably 6 to 10.

Examples of the aryl group represented by R³ to R⁶ include a phenyl group, a naphthyl group, a phenanthryl group, and an anthryl group, and a phenyl group or a naphthyl group is preferable, and a phenyl group is more preferable.

Examples of the heterocyclic group (hereinafter, also referred to as a "hetero ring group") represented by R³ to R⁶ include heterocyclic groups which have 3 or more atoms constituting the ring and include at least one or more heteroatoms and 1 to 30 carbon atoms as the atoms constituting the ring. In addition, the heterocyclic group includes an aromatic heterocyclic group (heteroaryl group) and an aliphatic heterocyclic group.

Examples of the heteroatom constituting the ring include a nitrogen atom, an oxygen atom, and a sulfur atom, and the number thereof is not particularly limited, but is, for example, 1 or 2. The number of carbon atoms constituting the ring is preferably 3 to 20 and more preferably 5 to 12.

As the heterocyclic group, a 5-membered ring, a 6-membered ring, or a group of a fused ring of these rings is preferable.

Examples of the heterocyclic group include a thienyl group, a thiazolyl group, an imidazolyl group, a pyridyl group, a pyrimidinyl group, a quinolyl group, a furanyl group, a selenophenyl (C₄H₃Se) group, a piperidyl group, a morpholino group, a benzoxazolyl group, a benzimidazolyl group, a benzthiazolyl group, a 2-hexylfuranyl group, a pyranyl group, and a 2-tetrahydropyranyl group.

The substituent which may be included in each of the aliphatic hydrocarbon group, the aromatic hydrocarbon group, and the heterocyclic group represented by R³ to R⁶ is not particularly limited, and examples thereof include a group selected from the following substituent group Z.

The number of substituents in the aliphatic hydrocarbon group, the aromatic hydrocarbon group, and the heterocyclic group represented by R³ to R⁶ is not particularly limited, but is preferably 1 to 6 and more preferably 1 to 3.

### -Substituent group Z-

Examples of the substituent group Z include a halogen atom, an alkyl group, an alkenyl group, an alkynyl group, an aryl group, a heterocyclic group, a silyl group, an alkoxy group, an amino group, an aryloxy group, an acyl group, an alkoxycarbonyl group, an aryloxycarbonyl group, an acyloxy group, an acylamino group, an aminocarbonylamino group, an alkoxycarbonylamino group, an aryloxycarbonylamino group, an alkylsulfonylamino group, an arylsulfonylamino group, an alkylthio group, an arylthio group, an alkylsulfinyl group, an arylsulfinyl group, an alkylsulfonyl group, an arylsulfonyl group, a silyloxy group, a heterocyclic oxy group, a carbamoyl group, a carbamoyloxy group, a heterocyclic thio group, a sulfamoyl group, an arylazo group, a heterocyclic azo group, an imide group, a phosphino group, a phosphinyl group, a phosphinyloxy group, a phosphinylamino group, a hydrazino group, an imino group, a cyano group, a hydroxy group, a nitro group, a mercapto group, a sulfo group, a carboxy group, a hydroxamic acid group, a sulfino group, a boronate group (-B(OH)₂), a phosphato group (-OPO(OH)₂), a phosphono group (-PO(OH)₂), and a sulfate group (-OSO₃H).

The group selected from the substituent group Z may further have a substituent.

Examples of the halogen atom included in the substituent group Z include a fluorine atom, a chlorine atom, a bromine atom, and an iodine atom, and a fluorine atom or a chlorine atom is preferable.

The alkyl group included in the substituent group Z is not particularly limited, but an alkyl group having 1 (3) to 30 carbon atoms is preferable, an alkyl group having 1 (3) to 20 carbon atoms is more preferable, and an alkyl group having 4 to 20 carbon atoms is still more preferable. The numbers in parentheses represent the number of carbon atoms in a case of a cycloalkyl group.

Examples of the alkyl group included in the substituent group Z, which may have a substituent, include a methyl group, an ethyl group, a propyl group, a 2-methylpropyl group, a butyl group, an amyl group, a pentyl group, a 2,2-dimethylpropyl group, a hexyl group, a heptyl group, an octyl group, a nonyl group, a decyl group, an undecyl group, a dodecyl group, a tridecyl group, a tetradecyl group, a pentadecyl group, a 2,6-dimethyloctyl group, an icosyl group, a 2-decyltetradecyl group, a 2-hexyldodecyl group, a 2-ethyloctyl group, a 2-decyltetradecyl group, a 2-butyldecyl group, a 1-octylnonyl group, a 2-ethyloctyl group, a 2-octyldecyl group, a 2-octyldodecyl group, a 7-hexylpentadecyl group, a 2-octyltetradecyl group, a 2-ethylhexyl group, a cyclopropyl group, a cyclobutyl group, a cyclopentyl group, a cyclohexyl group, an adamantyl group, a benzyl group, a p-chlorobenzyl group, a trifluoromethyl group, a perfluoroethyl group, 2,2,3,3,4,4,4,-heptafluorobutyl group, C₅F₁₁C₂H₄-, a 3-aminopropyl group, a 4-aminobutyl group, a 5-ethoxypentyl group, a (meth)acryloxypropyl group, a (meth)acryloxypentyl group, a 4-hydroxybutyl group, a 4-sulfobutyl group, a 10-phosphonodecyl group, a 2-hydroxyethoxymethyl group, a 2-imidazolylethoxymethyl group, and a 4-(N,N-dimethylamino)butyl) group.

The alkenyl group included in the substituent group Z is not particularly limited, but an alkenyl group having 2 to 20 carbon atoms is preferable, an alkenyl group having 2 to 12 carbon atoms is more preferable, and an alkenyl group having 2 to 8 carbon atoms is still more preferable.

Examples of the alkenyl group included in the substituent group Z, which may have a substituent, include a vinyl group, an allyl group, a 2-butenyl group, a 1-pentenyl group, and a 4-pentenyl group.

The alkynyl group included in the substituent group Z is not particularly limited, but an alkynyl group having 2 to 20 carbon atoms is preferable, an alkynyl group having 2 to 12 carbon atoms is more preferable, and an alkynyl group having 2 to 8 carbon atoms is still more preferable.

Examples of the alkynyl group included in the substituent group Z, which may have a substituent, include an ethynyl group, a propargyl group, a 1-pentynyl group, a trimethylsilylethynyl group, a triethylsilylethynyl group, a tri-i-propylsilylethynyl group, and a 2-p-propylphenylethynyl group.

The aryl group included in the substituent group Z is not particularly limited, but an aryl group having 6 to 20 carbon atoms is preferable and an aryl group having 6 to 12 carbon atoms is more preferable.

Examples of the aryl group included in the substituent group Z, which may have a substituent, include a phenyl group, a naphthyl group, a 2,4,6-trimethylphenyl group, a p-(t-butyl)phenyl group, a 4-methyl-2,6-dipropylphenyl group, a 4-fluorophenyl group, a 4-trifluoromethylphenyl group, a p-pentylphenyl group, a 3,4-dipentylphenyl group, a p-heptoxyphenyl group, and a 3,4-diheptoxyphenyl group.

Examples of the heterocyclic group included in the substituent group Z include heterocyclic groups which have 3 or more atoms constituting the ring and include at least one or more heteroatoms and 1 to 30 carbon atoms as the atoms constituting the ring. In addition, the heterocyclic group includes an aromatic heterocyclic group (heteroaryl group) and an aliphatic heterocyclic group.

Examples of the heteroatom constituting the ring include a nitrogen atom, an oxygen atom, and a sulfur atom, and the number thereof is not particularly limited, but is, for example, 1 or 2. The number of carbon atoms constituting the ring is preferably 3 to 20 and more preferably 5 to 12.

As the heterocyclic group, a 5-membered ring, a 6-membered ring, or a group of a fused ring of these rings is preferable.

Examples of the heterocyclic group included in the substituent group Z include a thienyl group, a thiazolyl group, an imidazolyl group, a pyridyl group, a pyrimidinyl group, a quinolyl group, a furanyl group, a selenophenyl group, a piperidyl group, a morpholino group, a benzoxazolyl group, a benzimidazolyl group, a benzthiazolyl group, a 2-hexylfuranyl group, and a pyranyl group.

The silyl group included in the substituent group Z, which may have a substituent, is not particularly limited, but a silyl group which has a group selected from an alkyl group and an aryl group as a substituent and has 3 to 40 (more preferably 3 to 30 and still more preferably 3 to 24) carbon atoms is preferable.

Examples of the silyl group included in the substituent group Z, which may have a substituent, include a trimethylsilyl group, a triphenylsilyl group, and a dimethylphenylsilyl group.

The alkoxy group included in the substituent group Z is not particularly limited, but an alkoxy group having 1 to 20 carbon atoms is preferable, an alkoxy group having 1 to 12 carbon atoms is more preferable, and an alkoxy group having 1 to 8 carbon atoms is still more preferable.

Examples of the alkoxy group included in the substituent group Z include a methoxy group, an ethoxy group, and a butoxy group.

The amino group included in the substituent group Z, which may have a substituent, is not particularly limited, but an amino group or an amino group which have a group selected from an alkyl group and an aryl group as a substituent and has 1 to 20 (more preferably 1 to 10 and still more preferably 1 to 6) carbon atoms is preferable.

Examples of the amino group included in the substituent group Z, which may have a substituent, include an amino group, a methylamino group, a dimethylamino group, a diethylamino group, a dibenzylamino group, and an anilino group.

The aryloxy group included in the substituent group Z is not particularly limited, but an aryloxy group having 6 to 20 carbon atoms is preferable, an aryloxy group having 6 to 16 carbon atoms is more preferable, and an aryloxy group having 6 to 12 carbon atoms is still more preferable.

Examples of the aryloxy group included in the substituent group Z include a phenyloxy group and a 2-naphthyloxy group.

The acyl group included in the substituent group Z is not particularly limited, but an acyl group having 1 to 20 carbon atoms is preferable, an acyl group having 1 to 16 carbon atoms is more preferable, and an acyl group having 1 to 12 carbon atoms is still more preferable.

Examples of the acyl group included in the substituent group Z, which may have a substituent, include an acetyl group, a hexanoyl group, a benzoyl group, a formyl group, and a pivaloyl group.

The alkoxycarbonyl group included in the substituent group Z is not particularly limited, but an alkoxycarbonyl group having 2 to 20 carbon atoms is preferable, an alkoxycarbonyl group having 2 to 16 carbon atoms is more preferable, an alkoxycarbonyl group having 2 to 12 carbon atoms is still more preferable, and a methoxycarbonyl group or an ethoxycarbonyl group is particularly preferable.

The aryloxycarbonyl group included in the substituent group Z is not particularly limited, but an aryloxycarbonyl group having 7 to 20 carbon atoms is preferable, an aryloxycarbonyl group having 7 to 16 carbon atoms is more preferable, an aryloxycarbonyl group having 7 to 10 carbon atoms is still more preferable, and a phenyloxycarbonyl group is particularly preferable.

The acyloxy group included in the substituent group Z is not particularly limited, but an acyloxy group having 2 to 20 carbon atoms is preferable, an acyloxy group having 2 to 16 carbon atoms is more preferable, and an acyloxy group having 2 to 10 carbon atoms is still more preferable.

Examples of the acyloxy group included in the substituent group Z, which may have a substituent, include an acetoxy group, a benzoyloxy group, and a (meth)acryloyloxy group.

The acylamino group included in the substituent group Z is not particularly limited, but an acylamino group having 2 to 20 carbon atoms is preferable, an acylamino group having 2 to 16 carbon atoms is more preferable, and an acylamino group having 2 to 10 carbon atoms is still more preferable.

Examples of the acylamino group included in the substituent group Z include an acetylamino group and a benzoylamino group.

The aminocarbonylamino group included in the substituent group Z is not particularly limited, but an aminocarbonylamino group having 2 to 20 carbon atoms is preferable, an aminocarbonylamino group having 2 to 16 carbon atoms is more preferable, an aminocarbonylamino group having 2 to 12 carbon atoms is still more preferable, and a ureido group is particularly preferable.

The alkoxycarbonylamino group included in the substituent group Z is not particularly limited, but an alkoxycarbonylamino group having 2 to 20 carbon atoms is preferable, an alkoxycarbonylamino group having 2 to 16 carbon atoms is more preferable, an alkoxycarbonylamino group having 2 to 12 carbon atoms is still more preferable, and a methoxycarbonylamino group is particularly preferable.

The aryloxycarbonylamino group included in the substituent group Z is not particularly limited, but an aryloxycarbonylamino group having 7 to 20 carbon atoms is preferable, an aryloxycarbonylamino group having 7 to 16 carbon atoms is more preferable, an aryloxycarbonylamino group having 7 to 12 carbon atoms is still more preferable, and a phenyloxycarbonylamino group is particularly preferable.

The alkylthio group included in the substituent group Z is not particularly limited, but an alkylthio group having 1 to 20 carbon atoms is preferable, an alkylthio group having 1 to 16 carbon atoms is more preferable, and an alkylthio group having 1 to 12 carbon atoms is still more preferable. Examples of the alkylthio group included in the substituent group Z include a methylthio group, an ethylthio group, and an octylthio group.

The arylthio group included in the substituent group Z is not particularly limited, but an arylthio group having 6 to 20 carbon atoms is preferable, an arylthio group having 6 to 16 carbon atoms is more preferable, an arylthio group having 6 to 12 carbon atoms is still more preferable, and a phenylthio group is particularly preferable.

Specific examples of the alkyl group represented by R³ to R⁶, which have a substituent, include an alkyl group having, as a substituent, a group selected from the group consisting of a halogen atom, an alkyl group, an aryl group, a heterocyclic group (heteroaryl group), an alkoxy group (including a hydroxyalkoxy group, a halogenated alkoxy group, and a heteroarylalkoxy group), an amino group, an acyloxy group, a hydroxy group, a sulfate group, and a phosphono group.

Specific examples of the aromatic hydrocarbon group represented by R³ to R⁶, which have a substituent, include an aryl group having a halogen atom and an aryl group having an alkyl group or a fluorinated alkyl group.

The number of carbon atoms included in R³ to R⁶ is not particularly limited, but from the viewpoint of carrier mobility of an organic semiconductor film in an organic thin film transistor, is preferably 30 or less and more preferably 20 or less, respectively. In addition, the lower limit of the number of carbon atoms in R⁶ is not particularly limited, but from the viewpoint of solubility in an organic solvent, is preferably 3 or more and more preferably 5 or more.

As R³ to R⁶, a hydrogen atom, an alkyl group having 1 to 30 carbon atoms, which may have a substituent, or an aryl group having 6 to 30 carbon atoms, which may have a substituent, is preferable; a hydrogen atom, an alkyl group having 1 to 20 carbon atoms, which may have, as a substituent, at least one selected from the group consisting of a halogen atom, an aryl group having 6 to 12 carbon atoms, and an aromatic heterocyclic group having 5 to 12 constituent atoms, or an aryl group having 6 to 20 carbon atoms, which may have, as a substituent, at least one selected from the group consisting of a halogen atom and an alkyl group having 1 to 20 carbon atoms, which may have a halogen atom, is more preferable; and a hydrogen atom or an alkyl group having 1 to 20 carbon atoms, which may have at least one selected from the group consisting of a fluorine atom and a phenyl group, is still more preferable.

In the intermediate I, it is preferable that both A¹ and A² represent -OR³, or that A¹ and A² are integrated to form -O- or -N(R⁶)-, and it is more preferable that A¹ and A² are integrated to form -N(R⁶)-.

In this case, as R³ in -OR³ and R⁶ in -N(R⁶)-, the above-described preferred groups are preferable.

In Formula (I), B¹ and B² each independently represent -CH= or -N=. However, at least one of B¹ or B² represents -N=. In addition, as described later, B¹ may be bonded to R¹ to form a ring, and B² may be bonded to R² to form a ring.

The intermediate I is a compound represented by any one of Formula (I-1), (1-2), or (1-3).

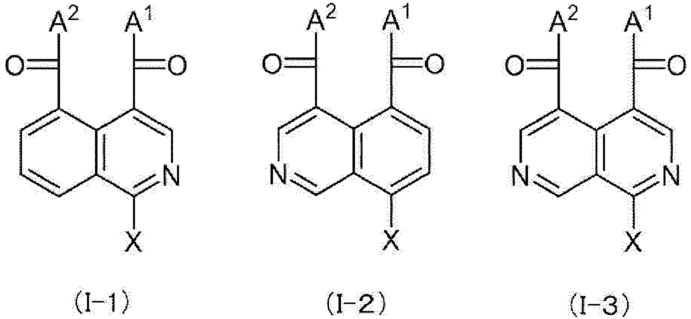

A¹, A², and X in Formulae (I-1) to (1-3) are the same as A¹, A², and X in Formula (I).

In Formula (I), R¹ and R² each independently represent a hydrogen atom or a substituent. In addition, R¹ and B¹, and R² and B² may be bonded to each other to form a ring.

The substituent represented by R¹ and R² is not particularly limited, and examples thereof include a group selected from the above-described substituent group Z. The group selected from the substituent group Z may further have a substituent, and examples of such a substituent include a group selected from the substituent group Z.

The ring formed by R¹ and B¹, and R² and B² is not particularly limited, and in a case where R¹ and B¹ are bonded to each other to form a ring, examples of the ring include a 5-membered ring, a 6-membered ring, and a fused ring of these rings, which are formed by R¹ and B¹ together with the carbon atom to which R¹ is bonded.

The ring formed by R¹ and B¹, and R² and B² may be a hetero ring. Examples of a heteroatom constituting the hetero ring include a nitrogen atom, an oxygen atom, and a sulfur atom, and a sulfur atom is preferable.

Specific examples of the ring formed by R¹ and B¹, and R² and B² include a benzene ring, a thiophene ring, a furan ring, an azole ring, a pyridine ring, a pyrazine ring, an imidazoline ring, a 1,2-thiazole ring, and a 1,2-oxazole ring. Among these, a benzene ring, a thiophene ring, or a furan ring is preferable.

The ring formed by R¹ and B¹, and R² and B² may have a substituent. The substituent in this case is not particularly limited, and examples thereof include a group selected from the above-described substituent group Z.

As R¹ and R², a hydrogen atom, an alkyl group, an alkenyl group, an alkoxycarbonyl group, an aryl group, an alkoxy group, a heterocyclic group, an amino group, a halogen atom, a cyano group, a carboxy group, a nitro group, or a mercapto group is preferable, a hydrogen atom, an alkyl group, an aryl group, a heteroaryl group, a halogen atom, or a cyano group is more preferable, and a hydrogen atom is still more preferable.

Among these, it is particularly preferable that both R¹ and R² are hydrogen atoms.

In Formula (I), X represents a leaving group or an atomic group (hereinafter, also referred to as an "atomic group N") having a metal atom or a metalloid atom, which is bonded to a carbon atom in an azanaphthalene ring.

The leaving group means a group which can be replaced with another group by a substitution reaction, and a leaving group used in a cross-coupling reaction can be used.

Examples of the leaving group include a halogen atom, an alkylsulfonyloxy group which may have a substituent, an arylsulfonyloxy group which may have a substituent, an alkylsulfonyl group which may have a substituent, an arylsulfonyl group which may have a substituent, a methoxy group, an arylester group, and a nitro group.

Examples of the halogen atom include a fluorine atom, a chlorine atom, a bromine atom, and an iodine atom, and a chlorine atom, a bromine atom, or an iodine atom is preferable.

As the alkylsulfonyloxy group which may have a substituent, an alkylsulfonyloxy group having 1 to 4 carbon atoms, which may have a halogen atom, is preferable, and a trifluoromethylsulfonyloxy group or a methylsulfonyloxy group is more preferable.

As the arylsulfonyloxy group which may have a substituent, a phenylsulfonyloxy group which may have a substituent is preferable, and a phenylsulfonyloxy group, a p-toluenesulfonyloxy group, a p-chlorophenylsulfonyloxy group, or an o-nitrophenylsulfonyloxy group is more preferable.

As the alkylsulfonyl group which may have a substituent, an alkylsulfonyl group having 1 to 4 carbon atoms, which may have a halogen atom, is preferable, and a trifluoromethylsulfonyl group or a methylsulfonyl group is more preferable.

As the arylsulfonyl group which may have a substituent, a phenylsulfonyl group which may have a substituent is preferable, and a phenylsulfonyl group, a p-toluenesulfonyl group, a p-chlorophenylsulfonyl group, or an o-nitrophenylsulfonyl group is more preferable.

As the leaving group, a halogen atom, a triflate group, a mesylate group, a phenylsulfonyloxy group, a p-toluenesulfonyloxy group, or an o-nitrophenylsulfonyloxy group is preferable; a chlorine atom, a bromine atom, or an iodine atom is more preferable; and a bromine atom is still more preferable.

The atomic group N represented by X is not particularly limited as long as at least one of atoms constituting the atomic group is a metal atom or a metalloid atom, which is bonded to the carbon atom in the azanaphthalene ring. In addition, the atomic group N may be constituted only of a metal atom or a metalloid atom, which is bonded to the carbon atom in the azanaphthalene ring.

Examples of the metal atom bonded to the carbon atom in the azanaphthalene ring include a lithium atom, a tin atom, a magnesium atom, a zinc atom, an aluminum atom, and a copper atom.

Examples of the metalloid atom bonded to the carbon atom in the azanaphthalene ring include a boron atom, a silicon atom, and a selenium atom.

Examples of the atomic group N having a lithium atom include a lithium atom (lithio group).

Examples of the atomic group N having a tin atom include a group represented by -Sn(Y¹)₃ (in the formula, Y¹ represents an alkyl group).

As Y¹ in the group represented by -Sn(Y¹)₃, an alkyl group having 1 to 6 carbon atoms is preferable, an alkyl group having 1 to 4 carbon atoms is more preferable, and a methyl group, an ethyl group, or an n-butyl group is still more preferable.

As the group represented -Sn(Y¹)₃, -Sn(CH₃)₃, -Sn(C₂H₅)₃, or -Sn(n-C₄H₉)₃ is preferable, and -Sn(n-C₄H₉)₃ is more preferable.

Examples of the atomic group N having a magnesium atom include a group represented by ―MgY² (in the formula, Y² represents a halogen atom).

As the group represented by -MgY², -MgCl or -MgBr is preferable, and -MgCl is more preferable.

Examples of the atomic group N having a zinc atom include a group represented by ―ZnY² (in the formula, Y² represents a halogen atom).

As the group represented by -ZnY², -ZnCl is preferable.

Examples of the atomic group N having an aluminum atom include a group represented by -Al(Y¹)₂ (in the formula, Y¹ represents an alkyl group).

As Y¹ in the group represented by -Al(Y¹)₂, an alkyl group having 1 to 6 carbon atoms is preferable, an alkyl group having 1 to 4 carbon atoms is more preferable, and a methyl group or an ethyl group is still more preferable. As the group represented by -Al(Y¹)₂, -AlSn(CH₃)₂ is preferable.

Examples of the atomic group N having a boron atom include a group represented by -B(OY³)₂ (in the formula, Y³ represents a hydrogen atom, a halogen atom, or an alkyl group; two Y³'s may be bonded to each other to form a ring).

It is preferable that Y³ in the group represented by -B(OY³)₂ represents a hydrogen atom or an alkyl group having 1 to 4 carbon atoms, or that two Y³'s are bonded to each other to form a ring represented by any one of Formulae (B-1), (B-2), or (B-3) together with a boron atom and two oxygen atoms.

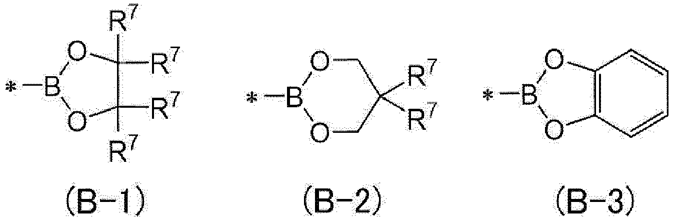

In the formulae, R⁷ represents a hydrogen atom or an alkyl group having 1 to 4 carbon atoms. As R⁷, a hydrogen atom or a methyl group is preferable, and a methyl group is more preferable.

As -B(OY³)₂, -B(OH)₂, -B(OCH₃)₂, -B(OC₂H₅)₃, a boronic acid ethylene glycol ester group (group represented by Formula (B-1), where all R⁷'s are hydrogen atoms), a boronic acid pinacol ester group (group represented by Formula (B-1), where all R⁷'s are methyl groups), a boronic acid trimethylene glycol ester group (group represented by Formula (B-2), where both R⁷'s are methyl groups), or a boronic acid catechol ester group (group represented by Formula (B-3) is preferable, and -B(OH)₂, a boronic acid pinacol ester group, a boronic acid trimethylene glycol ester group, or a boronic acid catechol ester group is more preferable.

Examples of the atomic group N having a silicon atom include a group represented by ―Si(Y⁴)₃ (in the formula, Y⁴ represents a halogen atom, an alkyl group, a hydroxy group, or an alkoxy group).

As Y⁴ in the group represented by -Si(Y⁴)₃, a halogen atom, a methyl group, an ethyl group, a hydroxy group, a methoxy group, or an ethoxy group is preferable, and a chlorine atom, a methyl group, a hydroxy group, or a methoxy group is more preferable.

As the group represented -Si(Y⁴)₃, -Si(CH₃)₃, -SiCH₃Cl₂, -Si(OCH₃)₃, or -Si(CH₃)₂OH is preferable, and -Si(OCH₃)₃ is more preferable.

As the atomic group N, a group represented by -Sn(Y¹)₃, a group represented by -B(OY³)₂, or a group represented by ―Si(Y⁴)₃ is preferable, a group represented by -B(OY³)₂ is more preferable.

As X, a halogen atom, an alkylsulfonyloxy group which may have a substituent, an arylsulfonyloxy group which may have a substituent, a group represented by -Sn(Y¹)₃, a group represented by -B(OY³)₂, or a group represented by ―Si(Y⁴)₃ is preferable, a chlorine atom, a bromine atom, an iodine atom, -B(OH)₂, a boronic acid pinacol ester group, a boronic acid trimethylene glycol ester group, or a boronic acid catechol ester group is more preferable, and a bromine atom or a boronic acid pinacol ester group is still more preferable.

Specific examples of the intermediate I are shown below, but the intermediate I is not limited to these specific examples.

In addition to the following specific examples, examples thereof include compounds in which one or both of -OR^{3a} and -OR^{3b} in the following compound I-A are replaced with -NR⁴R⁵. Here, examples of R⁴ and R⁵ in -NR⁴R⁵ include the same groups as R^{3a} or R^{3b} in the following specific examples.

In the following specific examples, TIPS represents a triisopropylsilyl group, and ^{∗} represents a bonding site.

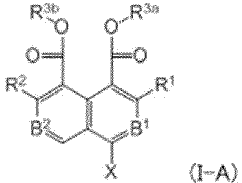

| No. | B¹ | B² | R¹ | R² | R^{3a} | R^{3b} | X |
|---|---|---|---|---|---|---|---|
| I -A-1 | N | CH | H | H | H | H | Cl |
| I -A-2 | N | CH | H | H | CH₃ | CH₃ | Br |
| I -A-3 | CH | N | H | H | CH₃ | CH₃ | Br |
| I -A-4 | N | N | H | H | C₂H₃ | C₂H₃ | I |
| I -A- 5 | N | CH | H | H | C₂H₃ | CH₃ | 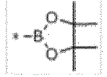 |
| I -A-6 | CH | N | H | H | -isoPropyl | -isoPropyl | 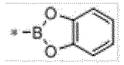 |
| I -A-7 | N | N | H | H | -tert-Butyl | -tert-Butyl | 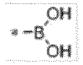 |
| I -A-8 | N | CH | H | H | -Phenyl | -Phenyl | 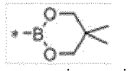 |
| I -A-9 | CH | N | H | H | C₂H₃ | CH₃ | 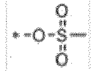 |
| I -A-10 | N | N | H | H | C₂H₃ | CH₃ | 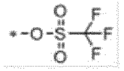 |
| I -A-11 | N | CH | H | H | -isoPropyl | CH₃ | 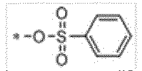 |
| I -A-12 | CH | N | H | H | -tert-Butyl | CH₃ | 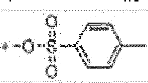 |
| I -A-13 | N | N | H | H | -Phenyl | CH₃ | 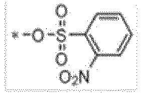 |
| I -A-14 | N | CH | H | H | CH₃ | -Phenyl | 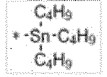 |
| I -A-15 | CH | N | H | H | nC₈H₁₃ | nC₈H₁₃ | 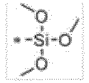 |
| I -A-16 | N | N | H | F | nC₂₀H₄₁ | nC₁₃H₂₇ | Cl |
| I -A-1 7 | N | CH | H | CN | nC₂₀H₄₁ | nC₂₀H₄₁ | Br |
| I -A-18 | CH | N | H | 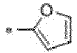 | -Cyclo hexyl | -Cyclohexyl | I |
| I -A-19 | N | N | CN | Ph | -Cyclopentyl | -Cyclopentyl | 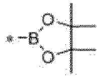 |
| I -A-20 | N | CH | CH₃ | H | -Cyclobutyl | -Cyclo butyl | 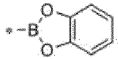 |
| I -A-21 | CH | N | F | F | -Cyclopropyl | -Cyclopropyl | 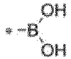 |
| I -A-22 | N | N | NO₂ | CN | CH₂C₃F₇ | CH₂C₃F₇ | 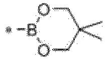 |
| I -A-23 | N | CH | N(CH₃)₂ | 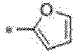 | -isoPropyl | -isoPropyl | 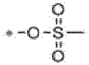 |
| I -A-24 | CH | N | COOH | Ph | -tert-Butyl | -tert-Butyl | 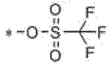 |
| I -A-25 | N | N | CO₂CH₃ | H | C₂H₄C₅F₁₁ | C₂H₄C₅F₁₁ | 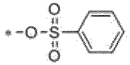 |
| I -A-26 | N | CH | OCH₃ | 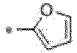 | -Phenyl | -Phenyl | 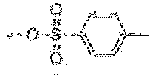 |
| I -A-27 | CH | N | 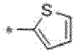 | CN | -Benzyl | -Benzyl | 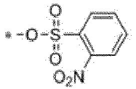 |
| I -A-28 | N | N | 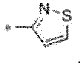 | 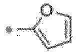 | CH₂CF₃ | CH₂CF₃ | 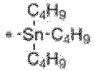 |
| I -A-29 | N | CH | H | Ph | 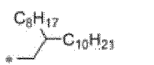 | 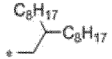 | 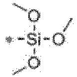 |
| I -A-30 | CH | N | CN | 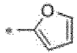 | 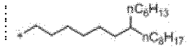 | 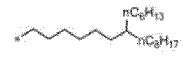 | Cl |
| I -A-31 | N | N | CH₃ | F | 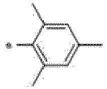 | 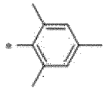 | Br |
| I -A-32 | N | CH | F | CN | 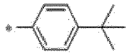 | 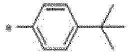 | I |
| I -A-33 | CH | N | NO₂ | 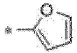 | 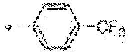 | 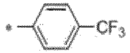 | 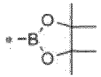 |
| I -A-34 | N | N | N(CH₃)₂ | 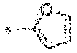 | 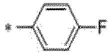 | 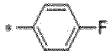 | 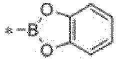 |
| I -A-35 | N | CH | COOH | H | 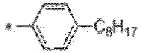 | 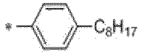 | 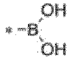 |
| I -A-36 | CH | N | CO₂CH₃ | F | 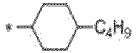 | 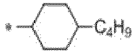 | 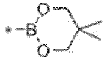 |
| I -A-37 | N | N | OCH₃ | CN | 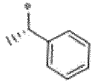 | 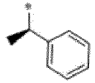 | 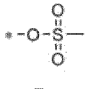 |
| I -A-38 | N | CH | 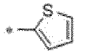 | 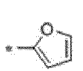 | 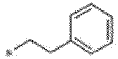 | 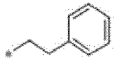 | 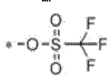 |
| I -A-39 | CH | N | 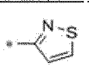 | Ph | 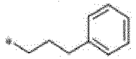 | 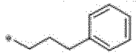 | 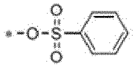 |
| I -A-40 | N | N | H | H | 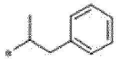 | 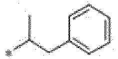 | 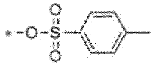 |
| I -A-41 | N | CH | CN | 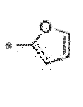 | 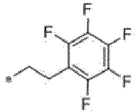 | 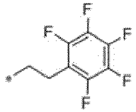 | 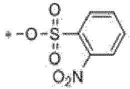 |
| I -A-42 | CH | N | CH₃ | 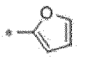 | 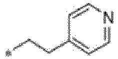 | 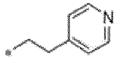 | 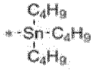 |
| I -A-43 | N | N | 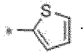 | 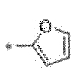 | 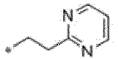 | 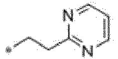 | 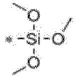 |
| I -A-44 | N | CH | 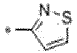 | 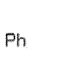 | 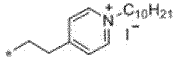 | 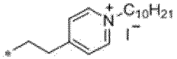 | Cl |
| I -A-45 | CH | N | N(CH₃)₂ | 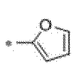 | 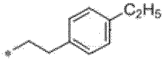 | 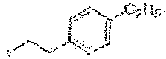 | Br |
| I -A-46 | N | N | COOH | 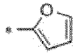 | 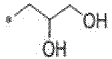 | 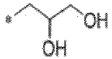 | I |
| I -A-47 | N | CH | CO₂CH₃ | CN | 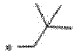 | 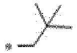 | 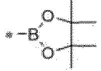 |
| I -A-48 | CH | N | OCH₃ | 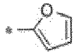 | 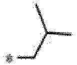 | 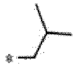 | 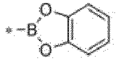 |
| I -A-49 | N | N | 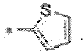 | Ph | 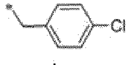 | 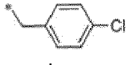 | 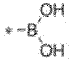 |
| I -A-50 | N | CH | 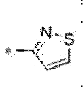 | 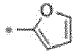 | 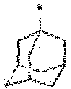 | 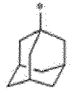 | 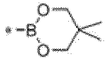 |
| I -A-51 | CH | N | H | F | 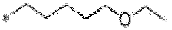 | 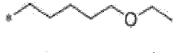 | 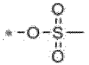 |
| I -A-52 | N | N | CN | CN | 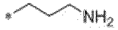 | 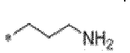 | 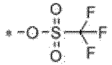 |
| I -A-53 | N | CH | CH₃ | 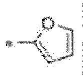 | 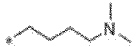 | 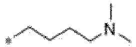 | 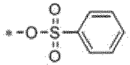 |
| I -A-54 | CH | N | 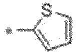 | 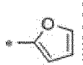 | 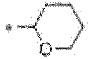 | 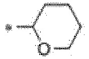 | 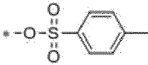 |
| I -A-55 | N | N | 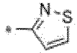 | H | 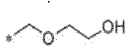 | 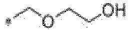 | 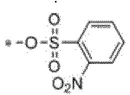 |
| I -A-56 | N | CH | N(CH₃)₂ | 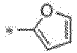 | 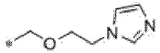 | 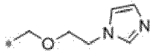 | 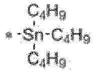 |
| I -A-57 | CH | N | COOH | CN | 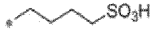 | 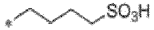 | 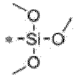 |
| I -A-58 | N | N | CO₂CH₃ | 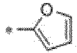 | 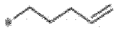 | 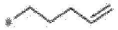 | Cl |
| I -A-59 | N | CH | OCH₃ | Ph | 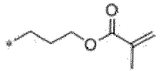 | 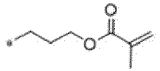 | Br |
| I -A-60 | CH | N | 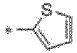 | 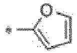 | 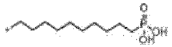 | 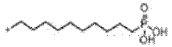 | I |
| I -A-61 | N | N | 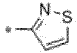 | F | 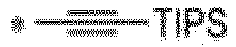 | 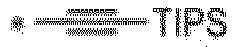 | 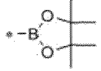 |

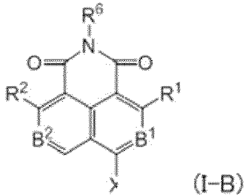

| No. | B¹ | B² | R¹ | R² | R⁵ | X |
|---|---|---|---|---|---|---|
| I -B-1 | N | CH | H | H | H | Cl |
| I -B-2 | CH | N | H | H | CH₃ | Br |
| I -B-3 | N | N | H | H | C₂H₅ | I |
| I -B-4 | N | CH | H | H | 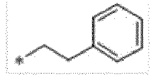 | Cl |
| I -B-5 | N | CH | H | H | 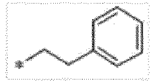 | Br |
| I -B-6 | CH | N | H | H | 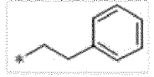 | Cl |
| I -B-7 | CH | N | H | H | 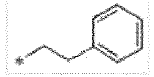 | Br |
| I -B-8 | N | N | H | H | 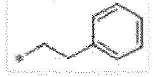 | Cl |
| I -B-9 | N | N | H | H | 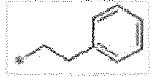 | Br |
| 1 -B-10 | N | CH | H | H | nC₈H₁₇ | Cl |
| I -B-11 | N | CH | H | H | nC₈H₁₇ | Br |
| I -B-12 | CH | N | H | H | nC₈H₁₇ | Cl |
| I- B-13 | CH | N | H | H | nC₈H₁₇ | Br |
| I -B-14 | N | N | H | H | nC₈H₁₇ | Cl |
| I -B-15 | N | N | H | H | nC₈H₁₇ | Br |
| I -B-16 | N | N | H | H | 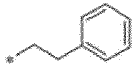 | I |
| I -B-17 | N | CH | H | H | nC₈H₁₇ | 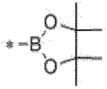 |
| I -B-18 | CH | N | H | H | 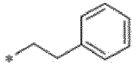 | 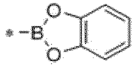 |
| I -B-19 | N | N | H | H | nC₈H₁₇ | 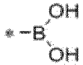 |
| I -B-20 | N | CH | H | H | 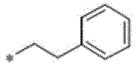 | 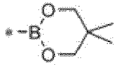 |
| I -B-21 | CH | N | H | H | nC₈H₁₇ | 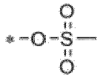 |
| I -B-22 | N | N | H | H | 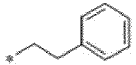 | 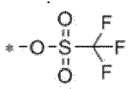 |
| I -B-23 | N | CH | H | H | nC₈H₁₇ | 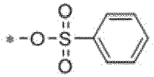 |
| I -B-24 | CH | N | H | H | 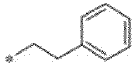 | 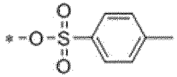 |
| I -B-25 | N | N | H | H | nC₈H₁₇ | 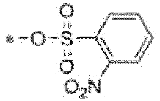 |
| I -B-26 | N | CH | H | H | 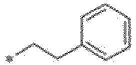 | 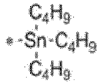 |
| I -B-27 | CH | N | H | H | nC₈H₁₇ | 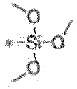 |
| I -B-28 | N | N | H | F | nC₁₃H₂₇ | Cl |
| I -B-28 | N | CH | H | CN | nC₂₀H₄₁ | Br |
| I -B-30 | CH | N | H | 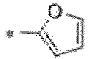 | -Cyclohexyl | I |
| I -B-31 | N | N | CN | Ph | -Cyclopentyl | 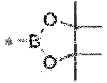 |
| I -B-32 | N | CH | CH₃ | H | -Cyclobutyl | 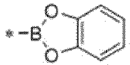 |
| I -B-33 | CH | N | F | F | -Cyclopropyl | 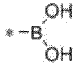 |
| I -B-34 | N | N | NO₂ | CN | CH₂C₃F₇ | 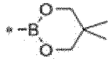 |
| I -B-35 | N | CH | N(CH₃)₂ | 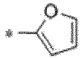 | -isoPropyl | 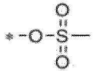 |
| I -B-36 | CH | N | COOH | Ph | -tert-Butyl | 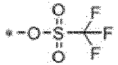 |
| I -B-37 | N | N | CO₂CH₃ | H | C₂H₄C₅F₁₁ | 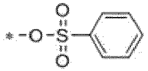 |
| I -B-38 | N | CH | OCH₃ | F | -Phenyl | 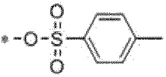 |
| I -B-39 | CH | N | 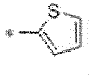 | CN | -Benzyl | 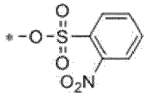 |
| I -B-40 | N | N | 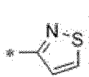 | 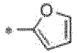 | CH₂CF₃ | 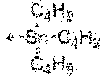 |
| I -B-41 | N | CH | H | Ph | 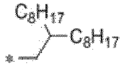 | 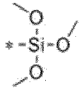 |
| I -B-42 | CH | N | CN | H | 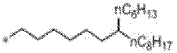 | Cl |
| I -B-43 | N | N | CH₃ | F | 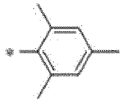 | Br |
| I -B-44 | N | CH | F | CN | 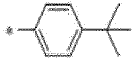 | I |
| I -B-45 | CH | N | NO₂ | 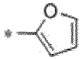 | 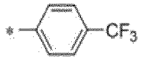 | 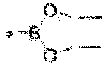 |
| 1 -B-46 | N | N | N(CH₃)₂ | Ph | 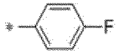 | 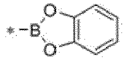 |
| I -B-47 | N | CH | COOH | H | 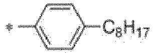 | 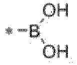 |
| I -B-48 | CH | N | CO₂CH₃ | F | 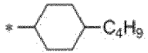 | 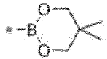 |
| I -B-49 | N | N | OCH₃ | CN | 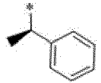 | 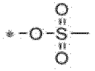 |
| I -B-50 | N | CH | 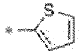 | 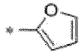 | 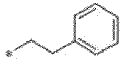 | 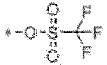 |
| I -B-51 | CH | N | 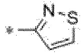 | Ph | 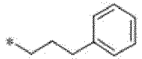 | 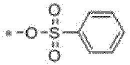 |
| I -B-52 | N | N | H | H | 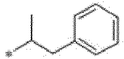 | 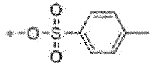 |
| I -B-53 | N | CH | CN | F | 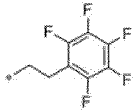 | 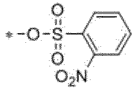 |
| I -B-54 | CH | N | CH₃ | CN | 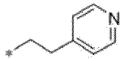 | 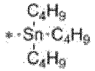 |
| I -B-55 | N | N | F | 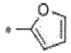 | 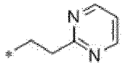 | 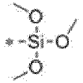 |
| I -B-56 | N | CH | NO₂ | Ph | 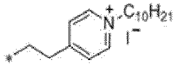 | Cl |
| I -B-57 | CH | N | N(CH₃)₂ | H | 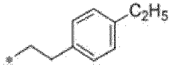 | Br |
| I -B-58 | N | N | COOH | 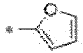 | 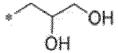 | I |
| I -B-59 | N | CH | CO₂CH₃ | C N | 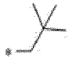 | 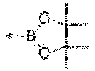 |
| I -B-60 | CH | N | OCH₃ | 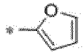 | 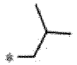 | 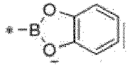 |
| I -B-61 | N | N | 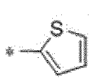 | Ph | 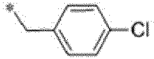 | 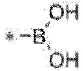 |
| I -B-62 | N | CH | 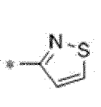 | H | 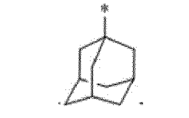 | 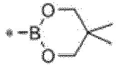 |
| I -B-63 | CH | N | H | F | 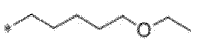 | 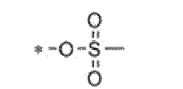 |
| I -B-64 | N | N | CN | CN | 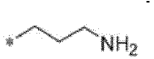 | 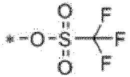 |
| I -B-65 | N | CH | CH₃ | 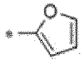 | 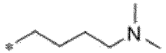 | 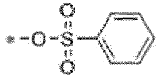 |
| I -B-66 | CH | N | F | Ph | 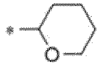 | 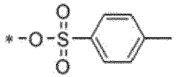 |
| I -B-67 | N | N | NO₂ | H | 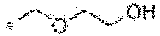 | 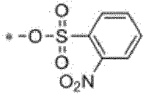 |
| I -B-68 | N | CH | N(CH₃)₂ | F | 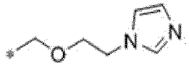 | 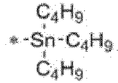 |
| I -B-69 | CH | N | COOH | CN | 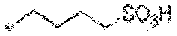 | 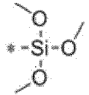 |
| I -B-70 | N | N | CO₂CH₃ | 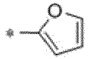 | 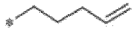 | Cl |
| I -B-71 | N | CH | OCH₃ | Ph | 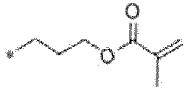 | Br |
| I -B-72 | CH | N | 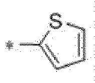 | H | 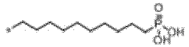 | I |
| I -B-73 | N | N | 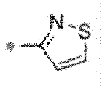 | F | 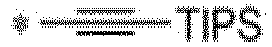 | 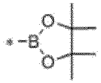 |

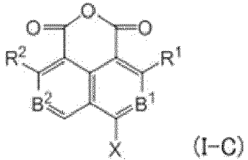

| No. | B¹ | B² | R¹ | R² | X |
|---|---|---|---|---|---|
| I -C-1 | N | CH | H | H | Cl |
| I -C-2 | CH | N | H | H | Br |
| I -C-3 | N | N | H | H | I |
| I -C-4 | N | CH | H | H | 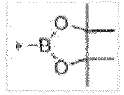 |
| I -C-5 | CH | N | H | H | 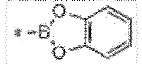 |
| 1 -C-6 | N | N | H | H | 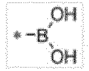 |
| I -C-7 | N | CH | H | H | 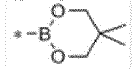 |
| I -C-8 | CH | N | H | H | 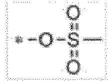 |
| I -C-9 | N | N | H | H | 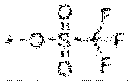 |
| I -C-10 | N | CH | H | H | 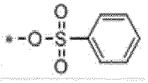 |
| I -C-11 | CH | N | H | H | 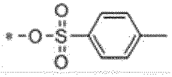 |
| I -C-12 | N | N | H | H | 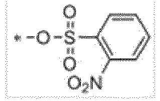 |
| 1 -C-13 | N | CH | H | H | 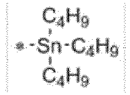 |
| I -C-14 | CH | N | H | H | 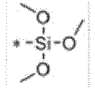 |

### <Method for producing intermediate I and use of intermediate I>

The method for producing the intermediate I is not particularly limited, and the intermediate I can be synthesized with reference to a known method. For example, the intermediate I can be synthesized according to the method described in Examples described later.

As described later, the intermediate I can be used to produce the intermediate II by reacting the intermediate I with an intermediate III in the presence of a transition metal catalyst.

### [Compound (intermediate II) represented by Formula (II)]

Next, the intermediate II used for producing the specific azaperylene compound will be described. The intermediate II is a compound represented by Formula (II).

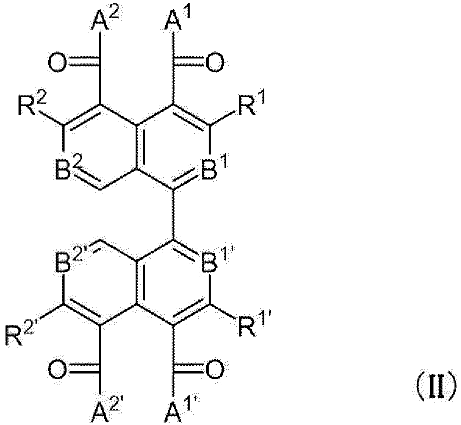

A¹, A², A^{1'}, and A^{2'} each independently represent -OR³ or -NR⁴R⁵. R³ to R⁵ each independently represent a hydrogen atom, an aliphatic hydrocarbon group which may have a substituent, an aromatic hydrocarbon group which may have a substituent, or a heterocyclic group which may have a substituent.

A¹ and A², and A^{1'} and A^{2'} may be integrated to form -O- or -N(R⁶)-. R⁶ represents an aliphatic hydrocarbon group which may have a hydrogen atom or a substituent, an aromatic hydrocarbon group which may have a substituent, or a heterocyclic group which may have a substituent.

B¹, B², B^{1'}, and B^{2'} each independently represent -CH= or -N=. However, at least one of B¹ or B² represents -N=.

R¹, R², R^{1'}, and R^{2'} each independently represent a hydrogen atom or a substituent.

In addition, R¹ and B¹, R² and B², R^{1'} and B^{1'}, and R^{2'} and B^{2'} may be bonded to each other to form a ring.

Suitable aspects of A¹, A², A^{1'}, and A^{2'} in Formula (II) are the same as the suitable aspects of A¹ and A² in Formula (I), respectively.

Suitable aspects of R³ to R⁶ in Formula (II) are the same as the suitable aspects of R³ and R⁶ in Formula (I), respectively.

Suitable aspects of B¹ and B^{1'}, B² and B^{2'}, R¹ and R^{1'}, and R² and R^{2'} in Formula (II) are respectively the same as the suitable aspects of B¹, B², R¹, and R² in Formula (I), except that both R^{1'} and R^{2'} may represent -CH=.

The number of -N='s represented in B¹, B², B^{1'}, and B^{2'} in Formula (II) is preferably 1 to 3 and more preferably 1 or 2.

As the intermediate II, a compound represented by any one of Formula (II-1), (II-2), (II-3), (II-4), (II-5), (II-6), (II-7), or (II-8) is preferable, and a compound represented by any one of Formula (II-1), (II-2), (II-3), (II-4), or (II-5) is more preferable.

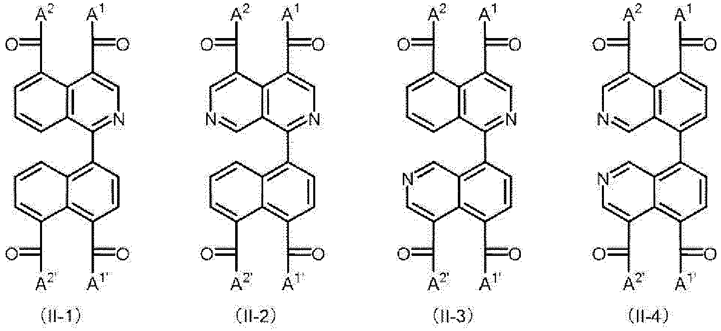

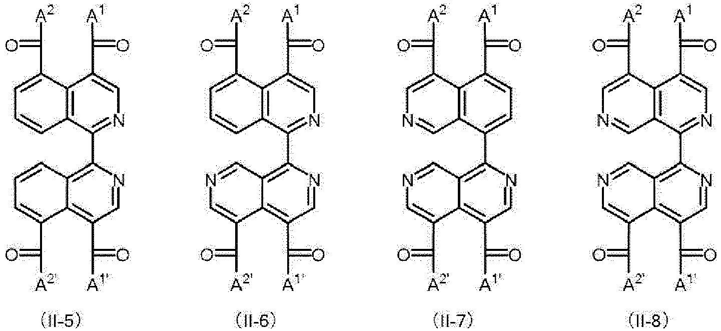

Each of A¹, A², A^{1'}, and A^{2'} in the above formulae are the same as A¹, A², A^{1'}, and A^{2'} in Formula (II), and the suitable aspects thereof are also the same.

Specific examples of the Intermediate II are shown below, but the Intermediate II is not limited to these specific examples.

In addition to the following specific examples, examples thereof include compounds in which at least one of -OR^{3a}, -OR^{3b}, -OR^{3c}, or -OR^{3d} in the following compounds II-A to II-C are replaced with -NR⁴R⁵. Here, examples of R⁴ and R⁵ in -NR⁴R⁵ include the same groups as R^{3a}, R^{3b}, R^{3c}, or R^{3d} in the following specific examples.

In the following specific examples, TIPS represents a triisopropylsilyl group, and ^{∗} represents a bonding site.

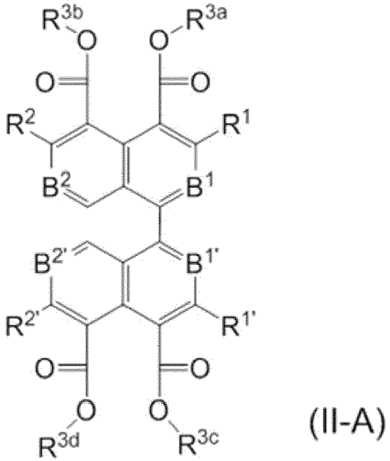

| No. | B¹ | B² | B^{1'} | B^{2'} | R¹ | R² | R^{1'} | R^{2'} |
|---|---|---|---|---|---|---|---|---|
| II -A-1 | N | CH | CH | CH | H | H | H | H |
| II -A-2 | N | CH | CH | CH | H | H | H | H |
| II -A-3 | N | N | CH | CH | H | H | H | H |
| II -A-4 | CH | N | CH | N | H | H | H | H |
| II -A-5 | N | N | N | CH | H | H | H | H |
| II -A-6 | N | N | CH | N | H | H | H | H |
| II -A-7 | N | N | N | N | H | H | H | H |
| II -A-8 | CH | N | CH | N | H | H | H | H |
| II -A-9 | N | N | N | CH | H | H | H | H |
| II -A-10 | N | N | CH | N | H | H | H | H |
| II -A-11 | N | N | N | N | H | H | H | H |
| II -A-12 | N | CH | CH | CH | H | H | H | H |
| II -A-13 | N | N | CH | CH | H | H | H | H |
| II -A-14 | CH | N | CH | N | H | H | H | H |
| II -A-15 | N | N | N | CH | H | H | H | H |
| II -A-16 | N | N | CH | N | H | H | H | H |
| II -A-17 | N | N | N | N | H | H | H | H |
| II -A-18 | N | CH | CH | CH | H | H | H | H |
| II -A-19 | N | N | CH | CH | H | H | H | H |
| II -A-20 | CH | N | CH | N | H | F | H | F |
| II -A-21 | N | N | N | CH | H | ON | H | ON |
| II -A-22 | N | N | CH | N | H | 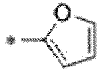 | H | 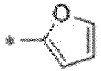 |
| II -A-23 | N | N | N | N | CN | Ph | ON | Ph |
| II -A-24 | N | CH | CH | CH | CH₃ | H | CH₃ | H |
| II -A-25 | N | N | CH | CH | F | F | F | F |

| No. | R^{5a} | R^{3b} | R^{3c} | R^{3d} |
|---|---|---|---|---|
| II -A-1 | H | H | H | H |
| II -A-2 | CH₃ | CH₃ | CH₃ | CH₃ |
| II -A-3 | CH₃ | CH₃ | CH₃ | CH₃ |
| II -A-4 | CH₃ | CH₃ | CH₃ | CH₃ |
| II -A-5 | CH₃ | CH₃ | CH₃ | CH₃ |
| II -A-6 | CH₃ | CH₃ | CH₃ | CH₃ |
| II -A-7 | CH₃ | CH₃ | CH₃ | CH₃ |
| II -A-8 | C₂H₅ | C₂H₅ | C₂H₅ | C₂H₅ |
| II -A-9 | CH₃ | C₂H₅ | CH₃ | C₂H₅ |
| II -A-10 | -isoPropyl | -isoPropyl | -isoPropyl | -isoPropyl |
| II -A-11 | -tert-Butyl | -tert-Butyl | -tert-Butyl | -tert-Butyl |
| II -A-12 | -Phenyl | -Phenyl | -Phenyl | -Phenyl |
| II -A-13 | CH₃ | C₂H₅ | CH₃ | C₂H₅ |
| II -A-14 | CH₃ | C₂H₅ | CH₃ | C₂H₅ |
| II -A-15 | CH₃ | -isoPropyl | CH₃ | -isoPropyl |
| II -A-16 | CH₃ | -tert-Butyl | CH₃ | -tert-Butyl |
| II -A-17 | CH₃ | -Phenyl | CH₃ | -Phenyl |
| II -A-18 | -Phenyl | CH₃ | -Phenyl | CH₃ |
| II -A-19 | nC₆H₁₃ | nC₆H₁₃ | nC₆H₁₃ | nC₆H₁₃ |
| II -A-20 | nC₁₃H₂₇ | nC₂₀H₄₁ | nC₁₃H₁₇ | nC₂₀H₄₁ |
| II -A-21 | nC₂₀H₄₁ | nC₂₀H₄₁ | nC₂₀H₄₁ | nC₂₀H₄₁ |
| II -A-22 | -Cyclohexyl | -Cyclohexyl | -Cyclohexyl | -Cyclohexyl |
| II -A-23 | -Cyclopentyl | -Cyclopentyl | -Cyclopentyl | -Cyclopentyl |
| II -A-24 | -Cyclobutyl | -Cyclobutyl | -Cyclobutyl | -Cyclobutyl |
| II -A-25 | -Cyclopropyl | -Cyclopropyl | -Cyclopropyl | -Cyclopropyl |

| No. | B¹ | B² | B^{1'} | B^{2'} | R¹ | R² | R^{1'} | R^{2'} |
|---|---|---|---|---|---|---|---|---|
| II -A-26 | CH | N | CH | N | NO₂ | CN | NO₂ | CN |
| II -A-27 | N | N | N | CH | N(CH₃)₂ | 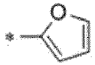 | N(CH₃)₂ | 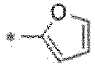 |
| II -A-28 | N | N | CH | N | COOH | Ph | COOH | Ph |
| II -A-29 | N | N | N | N | CO₂CH₃ | H | CO₂CH₃ | H |
| II -A-30 | N | CH | CH | CH | OCH₃ | 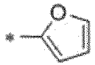 | OCH₃ | 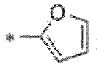 |
| II -A-31 | N | N | CH | CH | 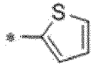 | CN | 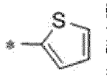 | CN |
| II -A-32 | CH | N | CH | N | 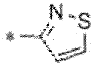 | 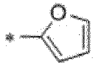 | 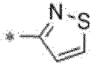 | 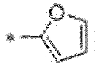 |
| II -A-33 | N | N | N | CH | H | Ph | H | Ph |
| II -A-34 | N | N | CH | N | CN | 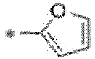 | CN | 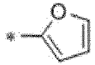 |
| II -A-35 | N | N | N | N | CH₃ | F | CH₃ | F |
| II -A-36 | N | CH | CH | CH | F | CN | F | CN |
| II -A-37 | N | N | CH | CH | NO₂ | 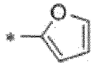 | NO₂ | 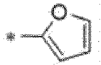 |
| II -A-38 | CH | N | CH | N | N(CH₃)₂ | 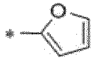 | N(CH₃)₂ | 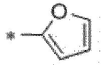 |
| II -A-39 | N | N | N | CH | COOH | H | COOH | H |
| II -A-40 | N | N | CH | N | CO₂CH₃ | F | CO₂CH₃ | F |

| No. | R^{5a} | R^{3b} | R^{3c} | R^{3d} |
|---|---|---|---|---|
| II -A-26 | CH₂C₃F₇ | CH₂C₃F₇ | CH₂C₃F₇ | CH₂C₃F₇ |
| II -A-27 | -isoPropyl | -isoPropyl | -isoPropyl | -isoPropyl |
| II -A-28 | -tert-Butyl | -tert-Butyl | -tert-Butyl | -tert-Butyl |
| II -A-29 | C₂H₄C₅F₁₁ | C₂H₄C₅F₁₁ | C₂H₄C₅F₁₁ | C₂H₄C₅F₁₁ |
| II -A-30 | -Phenyl | -Phenyl | -Phenyl | -Phenyl |
| II -A-31 | -Benzyl | -Benzyl | -Benzyl | -Benzyl |
| II -A-32 | CH₂CF₃ | CH₂CF₃ | CH₂CF₃ | CH₂CF₃ |
| II -A-33 | 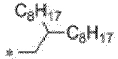 | 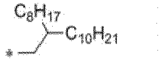 | 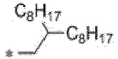 | 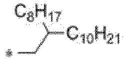 |
| II -A-34 | 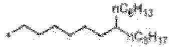 | 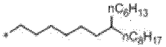 | 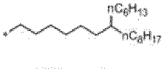 | 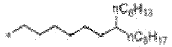 |
| II -A-35 | 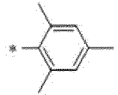 | 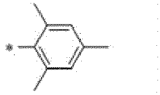 | 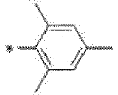 | 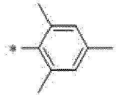 |
| II -A-36 | 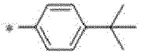 | 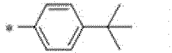 | 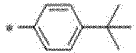 | 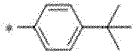 |
| II -A-37 | 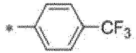 | 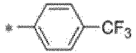 | 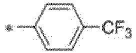 | 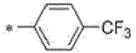 |
| II -A-38 | 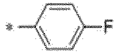 | 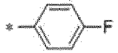 | 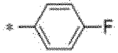 | 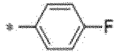 |
| II -A-39 | 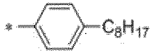 | 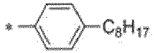 | 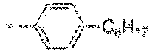 | 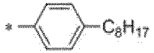 |
| II -A-40 | 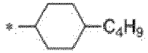 | 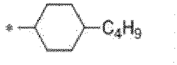 | 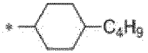 | 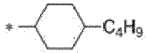 |

| No. | B¹ | B² | B^{1'} | B^{2'} | R¹ | R² | R^{1'} | R^{2'} |
|---|---|---|---|---|---|---|---|---|
| II -A-41 | N | N | N | N | OCH₃ | CN | OCH₃ | CN |
| II -A-42 | N | CH | CH | CH | 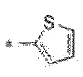 | 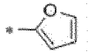 | 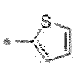 | 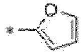 |
| II -A-43 | N | N | CH | CH | 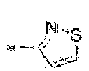 | Ph | 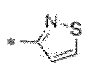 | Ph |
| II -A-44 | CH | N | CH | N | H | H | H | H |
| II -A-45 | N | N | N | CH | CN | 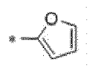 | CN | 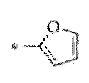 |
| II -A-46 | N | N | CH | N | CH₃ | 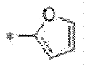 | CH₃ | 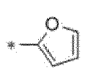 |
| II -A-47 | N | N | N | N | 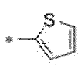 | 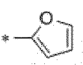 | 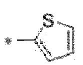 | 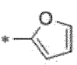 |
| II -A-48 | N | CH | CH | CH | 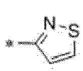 | Ph | 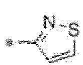 | Ph |
| II -A-49 | N | N | CH | CH | N(CH₃)₂ | 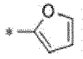 | N(CH₃)₂ | 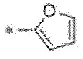 |
| II -A-50 | CH | N | CH | N | COOH | 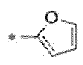 | COOH | 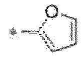 |
| II -A-51 | N | N | N | CH | CO₂CH₃ | CN | CO₂CH₃ | CN |
| II -A-52 | N | N | CH | N | OCH₃ | 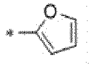 | OCH₃ | 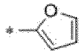 |
| II -A-53 | N | N | N | N | 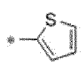 | Ph | 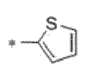 | Ph |
| II -A-54 | N | CH | CH | CH | 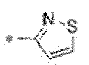 | 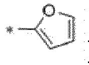 | 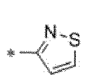 | 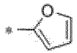 |
| II -A-55 | N | N | CH | CH | H | F | H | F |

| No. | R^{5a} | R^{3b} | R^{3c} | R^{3d} |
|---|---|---|---|---|
| II -A-41 | 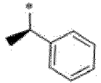 | 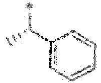 | 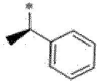 | 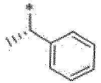 |
| II -A-42 | 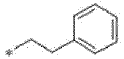 | 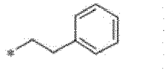 | 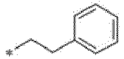 | 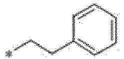 |
| II -A-43 | 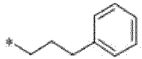 | 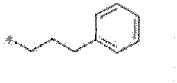 | 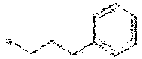 | 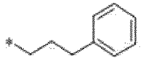 |
| II -A-44 | 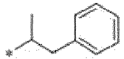 | 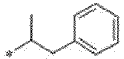 | 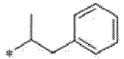 | 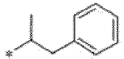 |
| II -A-45 | 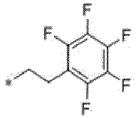 | 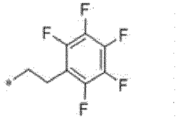 | 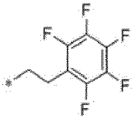 | 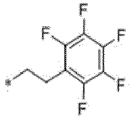 |
| II -A-46 | 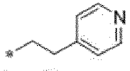 | 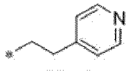 | 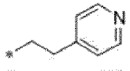 | 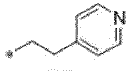 |
| II -A-47 | 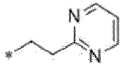 | 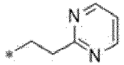 | 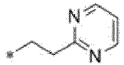 | 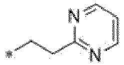 |
| II -A-48 | 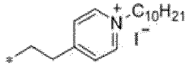 | 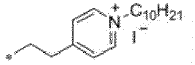 | 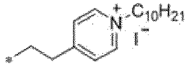 | 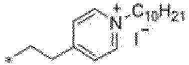 |
| II -A-49 | 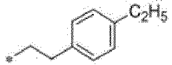 | 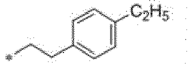 | 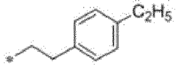 | 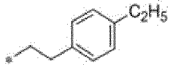 |
| II -A-50 | 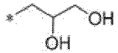 | 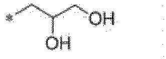 | 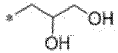 | 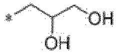 |
| II -A-51 | 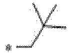 | 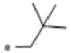 | 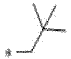 | 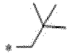 |
| II -A-52 | 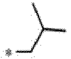 | 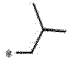 | 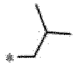 | 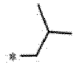 |
| II -A-53 | 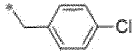 | 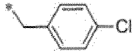 | 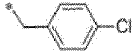 | 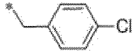 |
| II -A-54 | 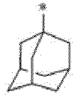 | 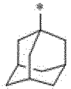 | 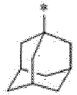 | 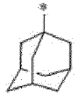 |
| II -A-55 | 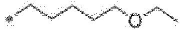 | 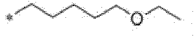 | 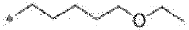 | 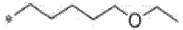 |

| No. | B¹ | B² | B^{1'} | B^{2'} | R¹ | R² | R^{1'} | R^{2'} |
|---|---|---|---|---|---|---|---|---|
| II -A-56 | CH | N | CH | N | CN | ON | CN | CN |
| II -A-57 | N | N | N | CH | CH₃ | 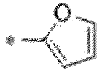 | CH₃ | 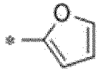 |
| II -A-58 | N | N | CH | N | 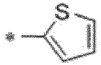 | 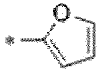 | 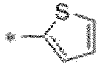 | 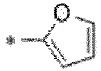 |
| II -A-59 | N | N | N | N | 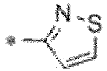 | H | 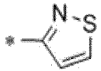 | H |
| II -A-60 | N | CH | CH | CH | N(C)H₃)₂ | 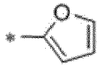 | N(CH₃)₂ | 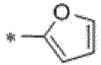 |
| II -A-61 | N | N | CH | CH | COOH | CN | COOH | CN |
| II -A-62 | CH | N | CH | N | CO₂CH₃ | 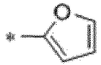 | CO₂CH₃ | 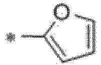 |
| II -A-63 | N | N | N | CH | OCH₃ | Ph | OCH₃ | Ph |
| II -A-64 | N | N | CH | N | 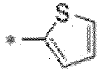 | 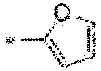 | 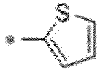 | 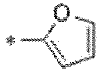 |
| II -A-65 | N | N | N | N | 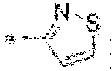 | F | 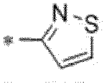 | F |

| No. | R^{5a} | R^{3b} | R^{3c} | R^{3d} |
|---|---|---|---|---|
| II -A-56 | 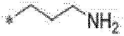 | 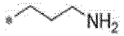 | 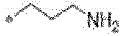 | 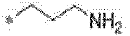 |
| II -A-57 | 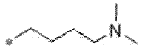 | 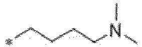 | 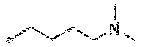 | 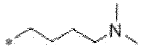 |
| II -A-58 | 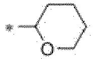 | 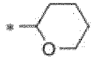 | 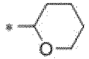 | 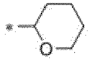 |
| II -A-59 | 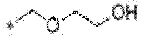 | 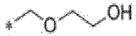 | 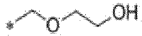 | 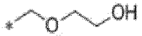 |
| II -A-60 | 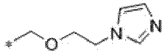 | 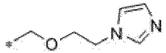 | 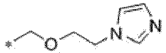 | 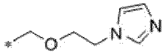 |
| II -A-61 | 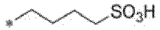 | 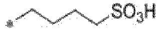 | 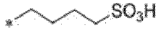 | 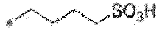 |
| II -A-62 | 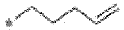 | 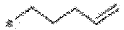 | 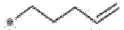 | 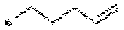 |
| II -A-63 | 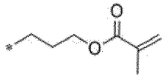 | 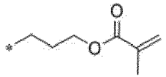 | 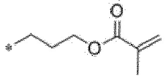 | 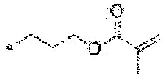 |
| II -A-64 | 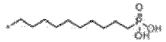 | 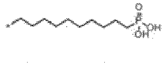 | 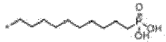 | 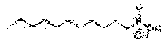 |
| II -A-65 | 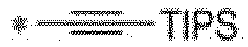 | 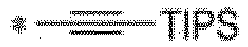 | 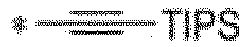 | 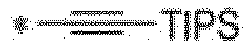 |

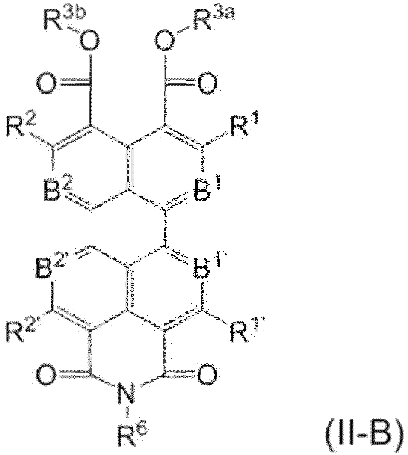

| No. | B¹ | B² | B^{1'} | B^{2'} | R¹ | R² | R^{1'} | R^{2'} |
|---|---|---|---|---|---|---|---|---|
| II -B-1 | N | CH | CH | CH | H | H | H | H |
| II -B-2 | N | N | CH | CH | H | H | H | H |
| II -B-3 | CH | N | CH | N | H | H | H | H |
| II -B-4 | N | CH | CH | CH | H | H | H | H |
| II -B-5 | N | N | CH | CH | H | H | H | H |
| II -B-6 | CH | N | CH | N | H | H | H | H |
| II -B-7 | N | N | N | CH | H | H | H | H |
| II -B-8 | N | N | CH | N | H | H | H | H |
| II -B-9 | N | N | N | N | H | H | H | H |
| II -B-10 | N | CH | CH | CH | H | H | H | H |
| II -B-11 | N | N | CH | CH | H | H | H | H |
| II -B-12 | CH | N | CH | N | H | H | H | H |
| II -B-13 | N | N | N | CH | H | H | H | H |
| II -B-14 | N | N | CH | N | H | H | H | H |
| II -B-15 | N | N | N | N | H | H | H | H |
| II -B-16 | N | N | CH | N | H | H | H | H |
| II -B-17 | N | N | N | N | H | H | H | H |
| II -B-18 | N | CH | CH | CH | H | H | H | H |
| II -B-19 | N | N | CH | CH | H | H | H | H |
| II -B-20 | CH | N | CH | N | H | H | H | H |

| No. | R^{3a} | R^{3d} | R⁶ |
|---|---|---|---|
| II -B-1 | H | H | H |
| II -B-2 | CH₃ | CH₃ | CH₃ |
| II -B-3 | C₂H₅ | C₂H₅ | C₂H₅ |
| II -B-4 | CH₃ | C₂H₅ | 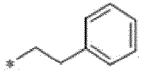 |
| II -B-5 | CH₃ | C₂H₅ | 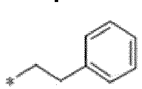 |
| II -B-6 | CH₃ | C₂H₅ | 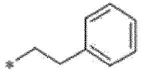 |
| II -B-7 | CH₃ | C₂H₅ | 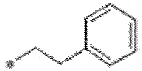 |
| II -B-8 | CH₃ | C₂H₅ | 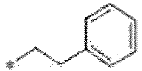 |
| II -B-9 | CH₃ | C₂H₅ | 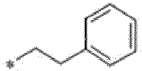 |
| II -B-10 | CH₃ | CH₃ | nC₈H₁₇ |
| II -B-11 | CH₃ | CH₃ | nC₈H₁₇ |
| II -B-12 | CH₃ | CH₃ | nC₈H₁₇ |
| II -B-13 | CH₃ | CH₃ | nC₈H₁₇ |
| II -B-14 | CH₃ | CH₃ | nC₈H₁₇ |
| II -B-15 | CH₃ | CH₃ | nC₈H₁₇ |
| II -B-16 | -isoPropyl | -isoPropyl | nC₈H₁₇ |
| II -B-17 | -tert-Butyl | -tert-Butyl | 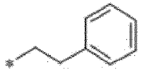 |
| II -B-18 | -Phenyl | -Phenyl | nC₈H₁₇ |
| II -B-19 | CH₃ | C₂H₅ | 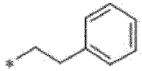 |
| II -B-20 | CH₃ | C₂H₅ | nC₈H₁₇ |

| No. | B¹ | B² | B^{1'} | B^{2'} | R¹ | R² | R^{1'} | R^{2'} |
|---|---|---|---|---|---|---|---|---|
| II -B-21 | N | N | N | CH | H | H | H | H |
| II -B-22 | N | N | CH | N | H | H | H | H |
| II -B-23 | N | N | N | N | H | H | H | H |
| II -B-24 | N | CH | CH | CH | H | H | H | H |
| II -B-25 | N | N | CH | CH | H | H | H | H |
| II -B-26 | CH | N | CH | N | H | F | H | F |
| II -B-27 | N | N | N | CH | H | CN | H | CN |
| II -B-28 | N | N | CH | N | H | 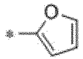 | H | 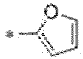 |
| II -B-29 | N | N | N | N | CN | Ph | CN | Ph |
| II -B-30 | N | CH | CH | CH | CH₃ | H | CH₃ | H |
| II -B-31 | N | N | CH | CH | F | F | F | F |
| II -B-32 | CH | N | CH | N | NO₂ | ON | NO₂ | CN |
| II -B-33 | N | N | N | CH | N(CH₃)₂ | 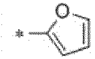 | N(CH₃)₂ | 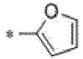 |
| II -B-34 | N | N | CH | N | COOH | Ph | COOH | Ph |
| II -B-35 | N | N | N | N | CO₂CH₃ | H | CO₃CH₃ | H |
| II -B-36 | N | CH | CH | CH | OCH₃ | 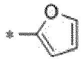 | OCH₃ | 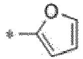 |
| II -B-37 | N | N | CH | CH | 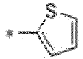 | CN | 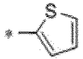 | CN |
| II -B-38 | CH | N | CH | N | 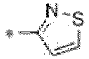 | 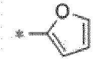 | 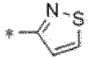 | 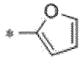 |
| II -B-39 | N | N | N | CH | H | Ph | H | Ph |
| II -B-40 | N | N | CH | N | CN | 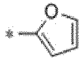 | ON | 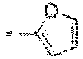 |

| No. | R^{3a} | R^{3d} | R⁶ |
|---|---|---|---|
| II -B-21 | CH₃ | -isoPropyl | 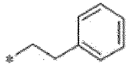 |
| II -B-22 | CH₃ | -tert-Butyl | nC₈H₁₇ |
| II -B-23 | CH₃ | -Phenyl | 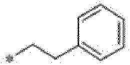 |
| II -B-24 | -Phenyl | CH₃ | nC₈H₁₇ |
| II -B-25 | nC₆H₁₃ | nC₆H₁₃ | 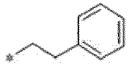 |
| II -B-26 | nC₁₃H₂₇ | nC₂₀H₄₁ | nC₈H₁₇ |
| II -B-27 | nC₂₀H₄₁ | nC₂₀H₄₁ | 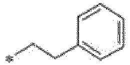 |
| II -B-28 | -Cyclo hexyl | -Cyclohexyl | nC₈H₁₇ |
| II -B-29 | -Cyclopentyl | -Cyclopentyl | nC₁₃H₂₇ |
| II -B-30 | -Cyclobutyl | -Cyclobutyl | nC₂₀H₄₁ |
| II -B-31 | -Cyclopropyl | -Cyclopropyl | -Cyclohexyl |
| II -B-32 | CH₂C₃F₇ | CH₂C₃F₇ | -Cyclopentyl |
| II -B-33 | -isoPropyl | -isoPropyl | -Cyclobutyl |
| II -B-34 | -tert-Butyl | -tert-Butyl | -Cyclopropyl |
| II -B-35 | C₂H₄C₅F₁₁ | C₂H₄C₅F₁₁ | CH₂C₃F₇ |
| II -B-36 | -Phenyl | -Phenyl | -isoPropyl |
| II -B-37 | -Benzyl | -Benzyl | -tert-Butyl |
| II -B-38 | CH₂CF₃ | CH₂CF₃ | C₂H₄C₅F₁₁ |
| II -B-39 | 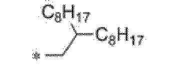 | 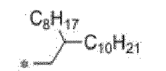 | -Phenyl |
| II -B-40 | 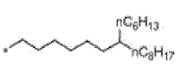 | 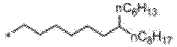 | -Benzyl |

| No. | B¹ | B² | B^{1'} | B^{2'} | R¹ | R² | R^{1'} | R^{2'} |
|---|---|---|---|---|---|---|---|---|
| II -B-41 | N | N | N | N | CH₃ | F | CH₃ | F |
| II -B-42 | N | CH | CH | CH | F | CN | F | CN |
| II -B-43 | N | N | CH | CH | NO₂ | 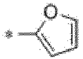 | NO₂ | 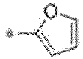 |
| II -B-44 | CH | N | CH | N | N(CH₃)₂ | 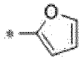 | N(CH₃)₂ | 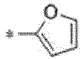 |
| II -B-45 | N | N | N | CH | COOH | H | COOH | H |
| II -B-46 | N | N | CH | N | CO₂CH₃ | F | CO₂CH₃ | F |
| II -B-47 | N | N | N | N | OCH₃ | CN | OCH₃ | CN |
| II -B-48 | N | CH | CH | CH | 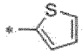 | 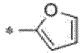 | 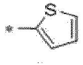 | 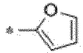 |
| II -B-49 | N | N | CH | CH | 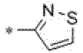 | Ph | 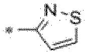 | Ph |
| II -B-50 | CH | N | CH | N | H | H | H | H |
| II -B-51 | N | N | N | CH | CN | 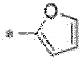 | CN | 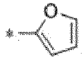 |
| II -B-52 | N | N | CH | N | CH₃ | 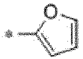 | CH₃ | 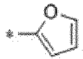 |
| II -B-53 | N | N | N | N | 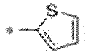 | 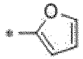 | 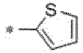 | 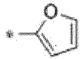 |
| II -B-54 | N | CH | CH | CH | 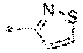 | Ph | 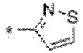 | Ph |
| II -B-55 | N | N | CH | CH | N(CH₃)₂ | 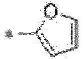 | N(CH₃)₂ | 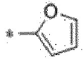 |

| No. | R^{3a} | R^{3d} | R⁶ |
|---|---|---|---|
| II -B-41 | 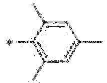 | 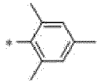 | CH₂CF₃ |
| II -B-42 | 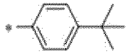 | 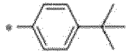 | 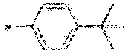 |
| II -B-43 | 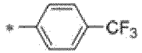 | 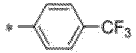 | 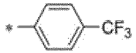 |
| II -B-44 | 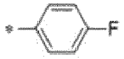 | 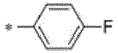 | 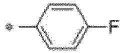 |
| II -B-45 | 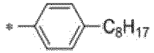 | 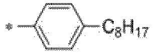 | 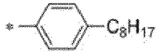 |
| II -B-46 | 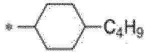 | 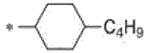 | 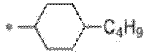 |
| II -B-47 | 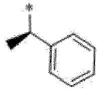 | 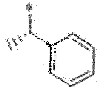 | 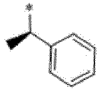 |
| II -B-48 | 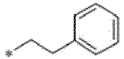 | 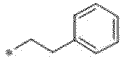 | 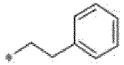 |
| II -B-49 | 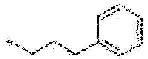 | 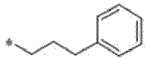 | 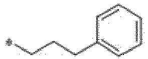 |
| II -B-50 | 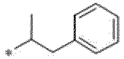 | 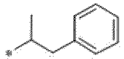 | 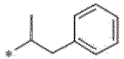 |
| II -B-51 | 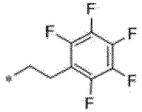 | 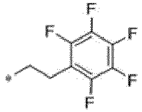 | 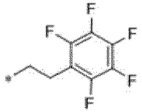 |
| II -B-52 | 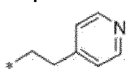 | 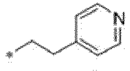 | 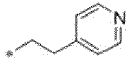 |
| II -B-53 | 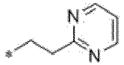 | 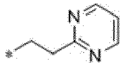 | 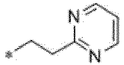 |
| II -B-54 | 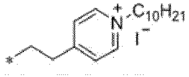 | 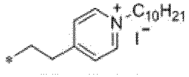 | 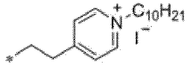 |
| II -B-55 | 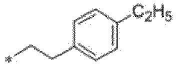 | 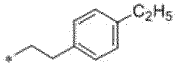 | 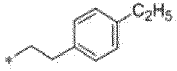 |

| No. | B¹ | B² | B^{1'} | B^{2'} | R¹ | R² | R^{1'} | R^{2'} |
|---|---|---|---|---|---|---|---|---|
| II -B-56 | CH | N | CH | N | COOH | 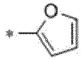 | COOH | 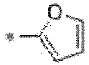 |
| II -B-57 | N | N | N | CH | CO₂CH₃ | CN | CO₂CH₃ | CN |
| II -B-58 | N | N | CH | N | OCH₃ | 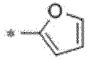 | OCH₃ | 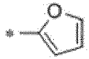 |
| II -B-59 | N | N | N | N | 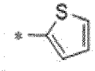 | Ph | 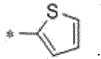 | Ph |
| II -B-60 | N | CH | CH | CH | 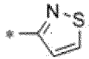 | 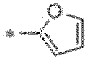 | 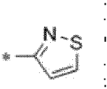 | 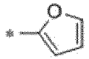 |
| II -B-61 | N | N | CH | CH | H | F | H | F |
| II -B-62 | CH | N | CH | N | CN | CN | CN | CN |
| II -B-63 | N | N | N | CH | CH₃ | 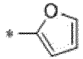 | CH₃ | 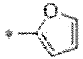 |
| II -B-64 | N | N | CH | N | 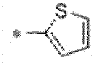 | 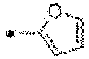 | 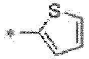 | 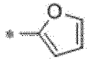 |
| II -B-65 | N | N | N | N | 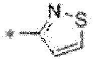 | H | 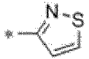 | H |
| II -B-66 | N | CH | CH | CH | N(CH₃)₂ | 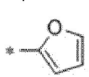 | N(CH₃)₂ | 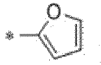 |
| II -B-67 | N | N | CH | CH | COOH | CN | COOH | CN |
| II -B-68 | CH | N | CH | N | CO₂CH₃ | 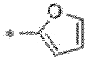 | CO₄CH₃ | 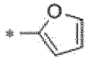 |
| II -B-69 | N | N | N | CH | OCH₃ | Ph | OCH₃ | Ph |
| II -B-70 | N | N | CH | N | 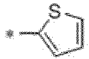 | 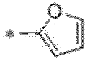 | 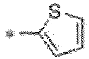 | 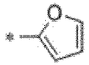 |
| II -B-71 | N | N | N | N | 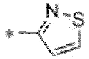 | F | 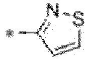 | F |

| No. | R^{3a} | R^{3d} | R⁶ |
|---|---|---|---|
| II -B-56 | 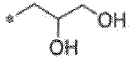 | 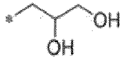 | 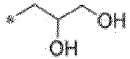 |
| II -B-57 | 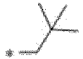 | 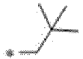 | 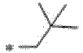 |
| II -B-58 | 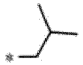 | 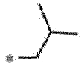 | 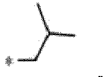 |
| II -B-59 | 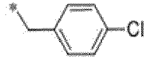 | 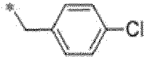 | 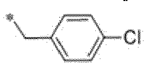 |
| II -B-60 | 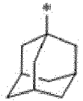 | 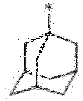 | 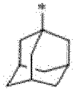 |
| II -B-61 | 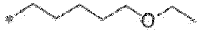 | 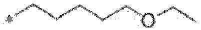 | 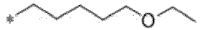 |
| II -B-62 | 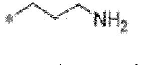 | 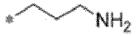 | 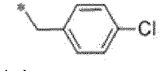 |
| II -B-63 | 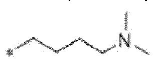 | 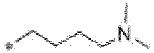 | 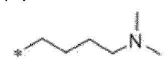 |
| II -B-64 | 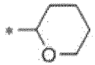 | 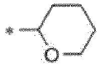 | 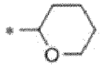 |
| II -B-65 | 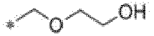 | 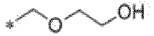 | 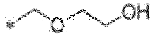 |
| II -B-66 | 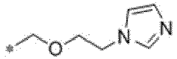 | 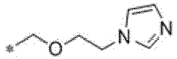 | 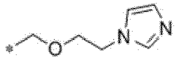 |
| II -B-67 | 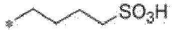 | 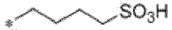 | 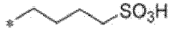 |
| II -B-68 | 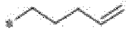 | 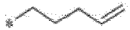 | 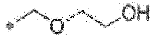 |
| II -B-69 | 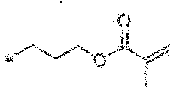 | 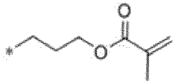 | 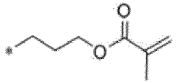 |
| II -B-70 | 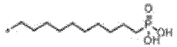 | 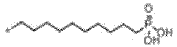 | 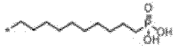 |
| II -B-71 | 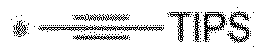 | 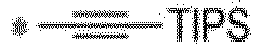 | 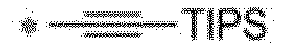 |

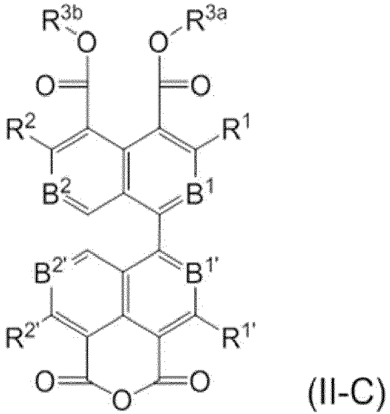

| No. | B¹ | B² | B^{1'} | B^{2'} | R¹ | R² | R^{1'} | R^{2'} | R^{3a} | R^{3b} |
|---|---|---|---|---|---|---|---|---|---|---|
| II -C-1 | N | CH | CH | CH | H | H | H | H | H | H |
| II -C-2 | N | N | CH | CH | H | H | H | H | CH₃ | CH₃ |
| II -C-3 | CH | N | CH | N | H | H | H | H | C₂H₅ | C₂H₅ |
| II -C-4 | N | N | N | CH | H | H | H | H | CH₃ | C₂H₅ |
| II -C-5 | N | N | CH | N | H | H | H | H | -isoPropyl | -isoPropyl |
| II -C-6 | N | N | N | N | H | H | H | H | -tert-Butyl | -tert-Butyl |
| II -C-7 | N | CH | CH | CH | H | H | H | H | -Phenyl | -Phenyl |
| II -C-8 | N | N | CH | CH | H | H | H | H | CH₃ | C₂H₅ |
| II -C-9 | CH | N | CH | N | H | H | H | H | CH₃ | C₂H₅ |
| II -C-10 | N | N | N | CH | H | H | H | H | CH₃ | -isoPropyl |
| II -C-11 | N | N | CH | N | H | H | H | H | CH₃ | -tert-Butyl |
| II -C-12 | N | N | N | N | H | H | H | H | CH₃ | -Phenyl |
| II -C-13 | N | CH | CH | CH | H | H | H | H | -Phenyl | CH₃ |
| II -C-14 | N | N | CH | CH | H | H | H | H | nC₆H₁₃ | nC₆H₁₃ |
| II -C-15 | CH | N | CH | N | H | F | H | F | nC₁₃H₂₇ | nC₂₀H₄₁ |
| II -C-16 | N | N | N | CH | H | CN | H | CN | nC₂₀H₄₁ | nC₂₀H₄₁ |
| II -C-17 | N | N | CH | N | H | 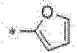 | H | 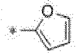 | -Cyclohexyl | -Cyclohexyl |
| II -C-18 | N | N | N | N | CN | Ph | CN | Ph | -Cyclopentyl | -Cyclopentyl |
| II -C-19 | N | CH | CH | CH | CH₃ | H | CH₃ | H | -Cyclobutyl | -Cyclobutyl |
| II -C-20 | N | N | CH | CH | F | F | F | F | -Cyclopropyl | -Cyclopropyl |
| II -C-21 | CH | N | CH | N | NO₂ | CN | NO₂ | CN | CH₂C₃F₇ | CH₂C₃F₇ |
| II -C-22 | N | N | N | CH | N(CH₃)₂ | 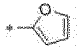 | N(CH₃)₂ | 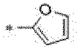 | -isoPropyl | -isoPropyl |
| II -C-23 | N | N | CH | N | COOH | Ph | COOH | Ph | -tert-Butyl | -tert-Butyl |
| II -C-24 | N | N | N | N | CO₂CH₃ | H | CO₂CH₃ | H | C₂H₄C₅F₁₁ | C₂H₄C₅F₁₁ |
| II -C-25 | N | CH | CH | CH | OCH₃ | 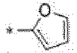 | OCH₃ | 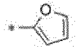 | -Phenyl | -Phenyl |
| II -C-26 | N | N | CH | CH | 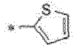 | CN | 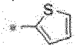 | CN | -Benzyl | -Benzyl |
| II -C-27 | CH | N | CH | N | 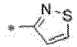 | 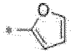 | 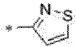 | 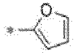 | CH₂CF₃ | CH₂CF₃ |
| II -C-28 | N | N | N | CH | H | Ph | H | Ph | 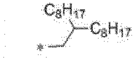 | 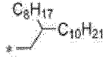 |
| II -C-29 | N | N | CH | N | CN | 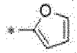 | CN | 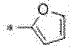 | 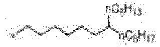 | 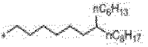 |
| II -C-30 | N | N | N | N | CH₃ | F | CH₃ | F | 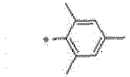 | 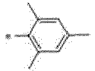 |
| II -C-31 | N | CH | CH | CH | F | CN | F | CN | 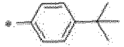 | 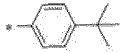 |
| II -C-32 | N | N | CH | CH | NO₂ | 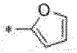 | NO₂ | 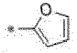 | 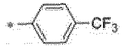 | 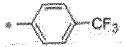 |
| II -C-33 | CH | N | CH | N | N(CH₃)₂ | 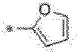 | N(CH₃)₂ | 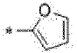 | 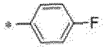 | 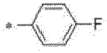 |
| II -C-34 | N | N | N | CH | COOH | H | COOH | H | 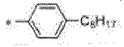 | 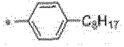 |
| II -C-35 | N | N | CH | N | CO₂OH₃ | F | CO₂CH₃ | F | 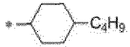 | 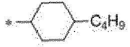 |
| II -C-36 | N | N | N | N | OCH₃ | CN | OCH₃ | CN | 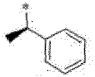 | 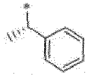 |
| II -C-37 | N | CH | CH | CH | 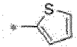 | 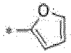 | 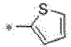 | 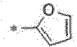 | 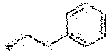 | 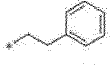 |
| II -C-36 | N | N | CH | CH | 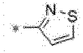 | Ph | 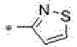 | Ph | 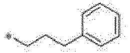 | 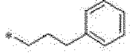 |
| II -C-39 | CH | N | CH | N | H | H | H | H | 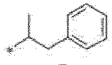 | 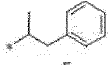 |
| II -C-40 | N | N | N | CH | CN | 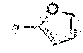 | CN | 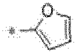 | 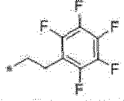 | 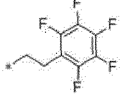 |
| II -C-41 | N | N | CH | N | CH₃ | 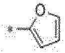 | CH₃ | 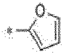 | 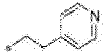 | 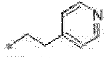 |
| II -C-42 | N | N | N | N | 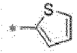 | 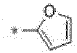 | 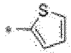 | 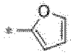 | 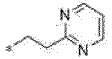 | 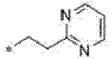 |
| II -C-43 | N | CH | CH | CH | 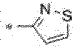 | Ph | 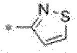 | Ph | 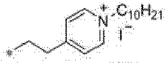 | 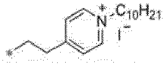 |
| II -C-44 | N | N | CH | CH | N(CH₃)₂ | 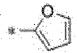 | N(CH₃)₂ | 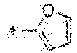 | 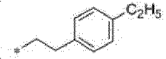 | 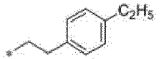 |
| II -C-45 | CH | N | CH | N | COOH | 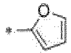 | COOH | 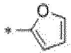 | 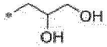 | 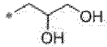 |
| II -C-46 | N | N | N | CH | CO₂CH₃ | CN | CO₂CH₃ | CN | 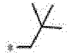 | 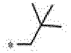 |
| II -C-47 | N | N | CH | N | OCH₃ | 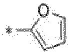 | OCH₃ | 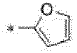 | 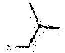 | 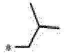 |
| II -C-48 | N | N | N | N | 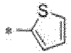 | Ph | 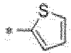 | Ph | 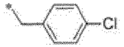 | 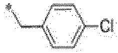 |
| II -C-49 | N | CH | CH | CH | 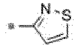 | 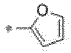 | 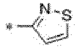 | 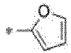 | 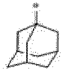 | 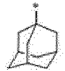 |
| II -C-50 | N | N | CH | CH | H | F | H | F | 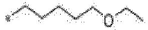 | 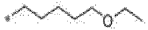 |
| II -C-51 | CH | N | CH | N | CN | CN | CN | CN | 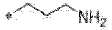 | 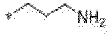 |
| II -C-52 | N | N | N | CH | CH₃ | 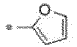 | CH₃ | 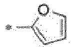 | 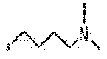 | 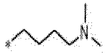 |
| II -C-53 | N | N | CH | N | 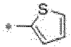 | 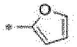 | 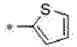 | 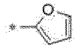 | 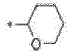 | 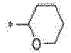 |
| II -C-54 | N | N | N | N | 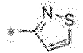 | H | 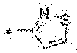 | H | 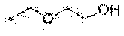 | 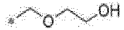 |
| II -C-55 | N | CH | CH | CH | N(CH₃)₂ | 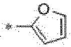 | N(CH₃)₂ | 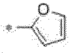 | 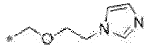 | 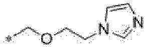 |
| II -C-56 | N | N | CH | CH | COOH | CN | COOH | CN | 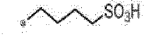 | 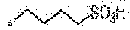 |
| II -C-57 | CH | N | CH | N | CO₂CH₃ | 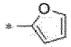 | CO₂CH₃ | 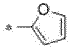 | 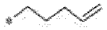 | 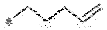 |
| II -C-58 | N | N | N | CH | OCH₃ | Ph | OCH₃ | Ph | 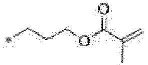 | 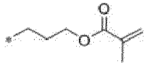 |
| II -C-59 | N | N | CH | N | 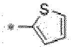 | 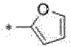 | 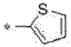 | 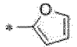 | 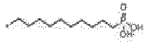 | 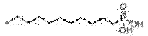 |
| II -C-60 | N | N | N | N | 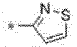 | F | 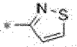 | F | 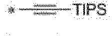 | 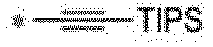 |

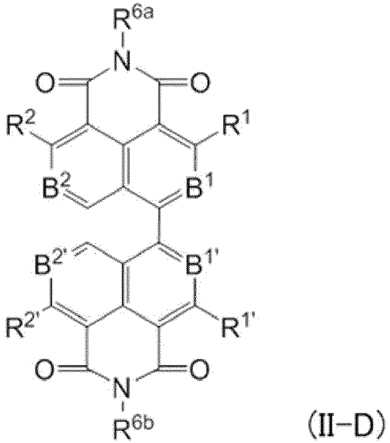

| No. | B¹ | B⁵ | B^{1'} | B^{2'} | R¹ | R² | R^{1'} | R^{2'} | R^{6a} | R^{6b} |
|---|---|---|---|---|---|---|---|---|---|---|
| II -D-1 | N | CH | CH | CH | H | H | H | H | H | H |
| II -D-2 | N | N | CH | CH | H | H | H | H | CH₃ | CH₃ |
| II-D-3 | CH | N | CH | N | H | H | H | H | C₂H₅ | C₂H₅ |
| II-D-4 | N | CH | CH | CH | H | H | H | H | 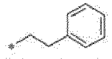 | 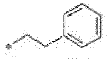 |
| II-D-5 | N | N | CH | CH | H | H | H | H | 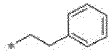 | 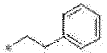 |
| II -D-6 | CH | N | CH | N | H | H | H | H | 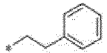 | 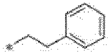 |
| II-D-7 | N | N | N | CH | H | H | H | H | 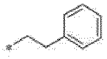 | 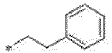 |
| II-D-8 | N | N | CH | N | H | H | H | H | 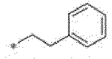 | 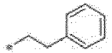 |
| II-D-9 | N | N | N | N | H | H | H | H | 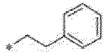 | 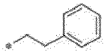 |
| II-D-10 | N | CH | CH | CH | H | H | H | H | nC₃H₁₇ | nC₃H₁₇ |
| II-D-11 | N | N | CH | CH | H | H | H | H | nC₈H₁₇ | nC₈H_{I7} |
| II-D-12 | CH | N | CH | N | H | H | H | H | nC₈H₁₇ | nC₈H₁₇ |
| II-D-13 | N | N | N | CH | H | H | H | H | nC₈H₁₇ | nC₈H₁₇ |
| II-D-14 | N | N | CH | N | H | H | H | H | NC₈H₁₇ | nC₈H₁₇ |
| II-D-15 | N | N | N | N | H | H | H | H | nC₈H₁₇ | nO₈H₁₇ |
| II-D-16 | N | CH | CH | CH | H | H | H | H | nC₈H₁₇ | nC₈H₁₇ |
| II-D-17 | N | N | CH | CH | H | H | H | H | 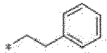 | nC₈H₁₇ |
| II-D-18 | CH | N | CH | N | H | H | H | H | 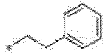 | nC₈H₁₇ |
| II-D-19 | N | N | N | CH | H | H | H | H | nC₈H₁₇ | 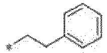 |
| II-D-20 | N | N | CH | N | H | H | H | H | nC₈H₁₇ | 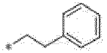 |
| II-D-21 | N | N | N | N | H | H | H | H | nC₈H₁₇ | 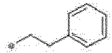 |
| II-D-22 | N | CH | CH | CH | H | H | H | H | nC₈H₁₇ | nC₈H₁₇ |
| II-D-23 | CH | N | CH | N | H | F | H | F | nC₆H₁₃ | nC₆H₁₃ |
| II-D-24 | N | N | N | CH | H | CN | H | CN | 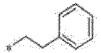 | 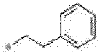 |
| II-D-25 | N | N | CH | N | H | 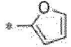 | H | 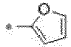 | nC₆H₁₃ | nC₆H₁₃ |
| II-D-26 | N | N | N | N | CN | Ph | CN | Ph | nC₁₃H₂₇ | nC₁₃H₂₇ |
| II-D-27 | N | CH | CH | CH | CH₃ | H | CH₃ | H | nC₂₀H₄₁ | nC₂₀H₄₁ |
| II-D-28 | N | N | CH | CH | F | F | F | F | -Cyclohexyl | -Cyclohexyl |
| II-D-29 | CH | N | CH | N | NO₂ | CN | NO₂ | CN | -Cyclopentyl | -Cyclopentyl |
| II-D-30 | N | N | N | CH | N(CH₃)₂ | 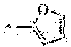 | N(CH₃)₂ | 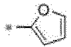 | -Cyctobutyl | -Cyctobutyl |
| II-D-31 | N | N | CH | N | COOH | Ph | COOH | Ph | -Cyclopropyl | -Cyclopropyl |
| II-D-32 | N | N | N | N | CO₂CH₃ | H | CO₂CR₃ | H | CH₂C₃F₇ | CH₂C₃F₇ |
| II-D-33 | N | CH | CH | CH | OCH₃, | 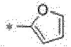 | OCH₃ | 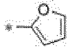 | -isoPropyl | -isoPropyl |
| II-D-34 | N | N | CH | CH | 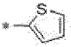 | CN | 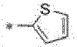 | CN | -tert-Buty) | -tert-Butyl |
| II-D-35 | CH | N | CH | N | 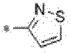 | 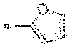 | 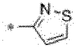 | 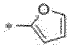 | C₂H₄C₅F₁₁ | C₂H₄C₅F₁₁ |
| II-D-36 | N | N | N | CH | H | Ph | H | Ph | -Phenyl | -Phenyl |
| II-D-37 | N | N | CH | N | CN | 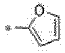 | CN | 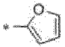 | -Benzyl | -Benzyl |
| II-D-3B | N | N | N | N | CH₃ | F | CH₃ | F | CH₂CF₃ | CH₂CF₃ |
| II-D-39 | N | CH | CH | CH | F | CN | F | CN | 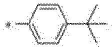 | 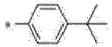 |
| II-D-40 | N | N | CH | CH | NO₂ | 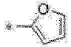 | NO₂ | 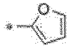 | 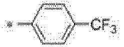 | 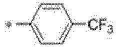 |
| II-D-41 | CH | N | CH | N | N(CH₃)₂ | 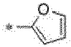 | N(CH₃)₂ | 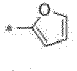 | 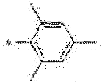 | 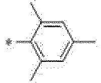 |
| II-D-42 | N | N | N | CH | COOH | H | COOH | H | 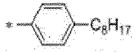 | 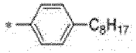 |
| II-D-43 | N | N | CH | N | CO₅CH₃ | F | CO₂CH₃ | F | 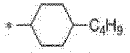 | 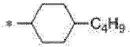 |
| II-D-44 | N | N | N | N | OCH₃ | CN | OCH₃ | CN | 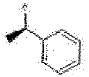 | 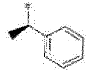 |
| II-D-45 | N | CH | CH | CH | 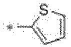 | 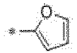 | 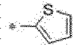 | 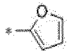 | 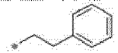 | 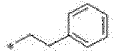 |
| II-D-46 | N | N | CH | CH | 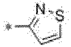 | Ph | 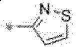 | Ph | 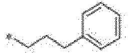 | 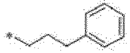 |
| II-D-47 | CH | N | CH | N | H | H | H | H | 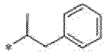 | 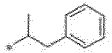 |
| II-D-48 | N | N | N | CH | CN | 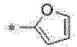 | CN | 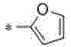 | 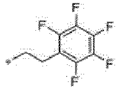 | 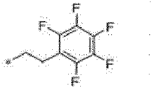 |
| II-D-49 | N | N | CH | N | CH₃ | 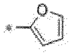 | CH₃ | 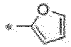 | 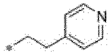 | 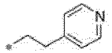 |
| II-D-50 | N | N | N | N | 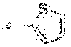 | 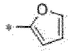 | 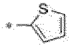 | 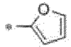 | 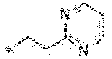 | 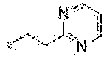 |
| II-D-51 | N | CH | CH | CH | 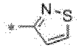 | Ph | 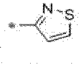 | Ph | 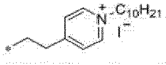 | 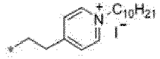 |
| II-D-52 | N | N | CH | CH | N(CH₃)₂ | 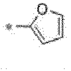 | N(CH₃)₂ | 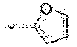 | 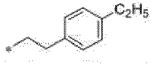 | 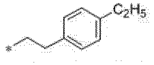 |
| II-D-53 | CH | N | CH | N | COOH | 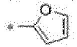 | COOH | 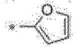 | 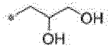 | 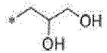 |
| II-D-54 | N | N | N | CH | CO₂CH₃ | CN | CO₂CH₃ | CN | 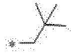 | 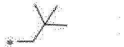 |
| II-D-55 | N | N | CH | N | OCH₃ | 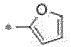 | OCH₃ | 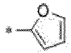 | 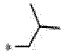 | 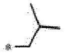 |
| II-D-56 | N | N | N | N | 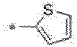 | Ph | 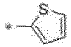 | Ph | 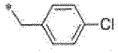 | 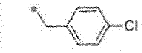 |
| II-D-57 | N | CH | CH | CH | 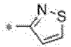 | 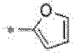 | 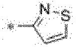 | 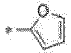 | 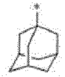 | 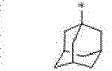 |
| II-D-58 | N | N | CH | CH | H | F | H | F | 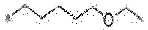 | 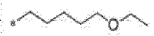 |
| II-D-59 | CH | N | CH | N | CN | CN | CN | CN | 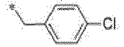 | 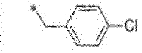 |
| II-D-60 | N | N | N | CH | CH₃ | 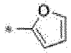 | CH₃ | 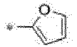 | 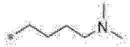 | 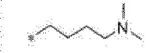 |
| II-D-61 | N | N | CH | N | 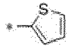 | 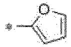 | 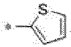 | 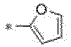 | 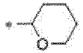 | 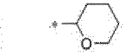 |
| II-D-62 | N | N | N | N | 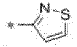 | H | 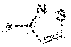 | H | 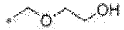 | 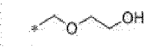 |
| II-D-63 | N | CH | CH | CH | N(CH₃)₂ | 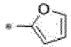 | N(CH₃)₂ | 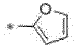 | 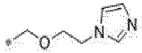 | 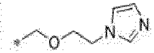 |
| II-D-64 | N | N | CH | CH | COOH | CN | COOH | CN | 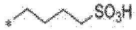 | 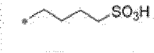 |
| II-D-65 | CH | N | CH | N | CO₅CH₃ | 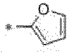 | CO₂CH₃ | 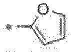 | 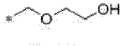 | 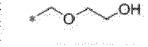 |
| II-D-66 | N | N | N | CH | OCH₃ | Ph | OCH₃ | Ph | 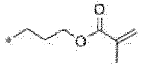 | 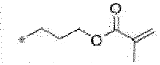 |
| II-D-67 | N | N | CH | N | 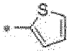 | 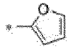 | 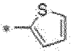 | 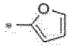 | 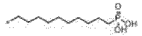 | 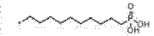 |
| II-D-68 | N | N | N | N | 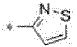 | F | 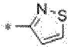 | F | 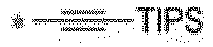 | 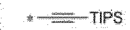 |

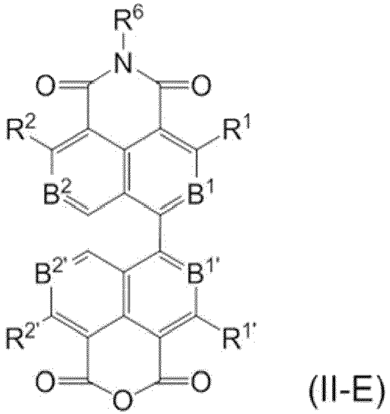

| No. | B¹ | B² | B^{1'} | B^{2'} | R¹ | R² | R^{1'} | R^{2'} | R⁶ |
|---|---|---|---|---|---|---|---|---|---|
| II-E-1 | N | CH | CH | CH | H | H | H | H | H |
| II-E-2 | N | N | CH | CH | H | H | H | H | CH₃ |
| II-E-3 | CH | N | CH | N | H | H | H | H | C₂H₅ |
| II-E-4 | N | CH | CH | CH | H | H | H | H | 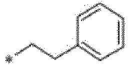 |
| II-E-5 | N | N | CH | OH | H | H | H | H | 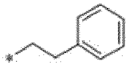 |
| II-E-6 | CH | N | CH | N | H | H | H | H | 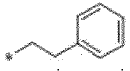 |
| II-E-7 | N | N | N | CH | H | H | H | H | 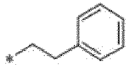 |
| II -E-8 | N | N | CH | N | H | H | H | H | 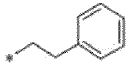 |
| II-E-9 | N | N | N | N | H | H | H | H | 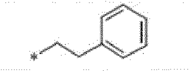 |
| II-E-10 | N | CH | CH | CH | H | H | H | H | nC₈H₁₇ |
| II-E-11 | N | N | CH | CH | H | H | H | H | nC₈H₁₇ |
| II-E-12 | CH | N | CH | N | H | H | H | H | nC₈H₁₇ |
| II-E-13 | N | N | N | CH | H | H | H | H | nC₈H₁₇ |
| II-E-14 | N | N | CH | N | H | H | H | H | nC₈H₁₇ |
| II-E-15 | N | N | N | N | H | H | H | H | nC₈H₁₇ |
| II-E-16 | CH | N | CH | N | H | F | H | F | nC₈H₁₇ |
| II-E-17 | N | N | N | CH | H | CN | H | CN | 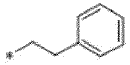 |
| II-E-18 | N | N | CH | N | H | 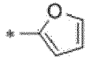 | H | 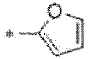 | nC₈H₁₇ |
| II-E-19 | N | N | N | N | ON | Ph | CN | Ph | nC₁₃H₂₇ |
| II-E-20 | N | CH | CH | CH | CH₃ | H | CH₃ | H | nC₂₀H₄₁ |
| II-E-21 | N | N | CH | CH | F | F | F | F | -Cyclohexyl |
| II-E-22 | CH | N | CH | N | NO₂ | ON | NO₂ | CN | -Cyclopentyl |
| II-E-23 | N | N | N | CH | N(CH₃)₂ | 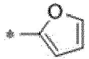 | N(CH₃)₂ | 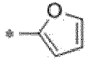 | -Cyctabutyl |
| II-E-24 | N | N | CH | N | COOH | Ph | COOH | Ph | -Cyclopropyl |
| II-E-25 | N | N | N | N | OO₂OH₃ | H | CO₂CH₃ | H | CH₂C₃F₇ |
| II-E-26 | N | CH | CH | CH | OCH₃ | 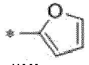 | OCH₃ | 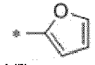 | -isoPropyl |
| II-E-27 | N | N | CH | CH | 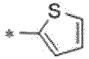 | CN | 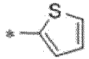 | CN | -tert-Butyl |
| II-E-28 | CH | N | CH | N | 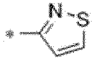 | 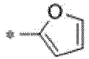 | 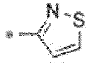 | 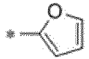 | C₂H₄C₅F₁₁ |
| II-E-29 | N | N | N | CH | H | Ph | H | Ph | -Phenyl |
| II-E-30 | N | N | CH | N | ON | 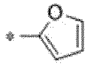 | CN | 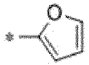 | -Benzyl |
| II-E-31 | N | N | N | N | CH₃ | F | CH₃ | F | CH₂CF₃ |
| II-E-32 | N | CH | CH | CH | F | CN | F | CN | 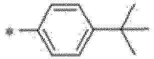 |
| II-E-33 | N | N | CH | CH | NO₂ | 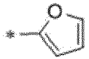 | NO₂ | 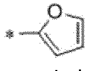 | 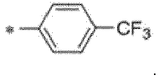 |
| II-E-34 | CH | N | CH | N | N(CH₃)₂ | 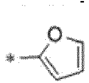 | N(CH₃)₂ | 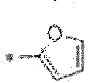 | 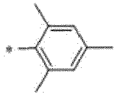 |
| II-E-35 | N | N | N | CH | COOH | H | COOH | H | 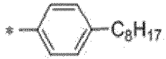 |
| II-E-36 | N | N | CH | N | CO₂CH₃ | F | CO₂CH₃ | F | 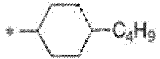 |
| II-E-37 | N | N | N | N | OCH₃ | CN | OCH₃ | CN | 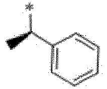 |
| II-E-38 | N | CH | CH | CH | 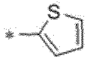 | 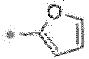 | 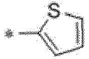 | 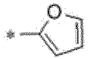 | 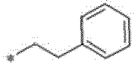 |
| II-E-39 | N | N | CH | CH | 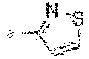 | Ph | 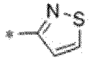 | Ph | 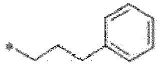 |
| II-E-40 | CH | N | CH | N | H | H | H | H | 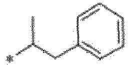 |
| II-E-41 | N | N | N | CH | CN | 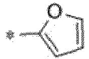 | CN | 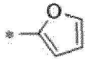 | 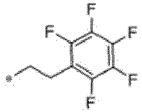 |
| II-E-42 | N | N | CH | N | CH₃ | 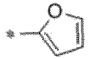 | CH₃ | 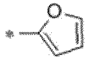 | 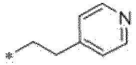 |
| II-E-43 | N | N | N | N | 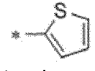 | 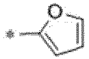 | 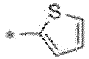 | 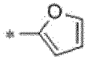 | 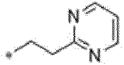 |
| II-E-44 | N | CH | CH | CH | 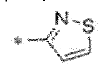 | Ph | 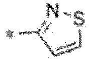 | Ph | 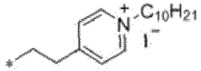 |
| II-E-45 | N | N | CH | CH | N(CH₃)₂ | 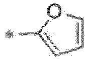 | N(CH₃)₂ | 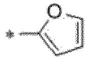 | 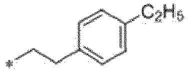 |
| II-E-46 | CH | N | CH | N | COOH | 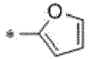 | COOH | 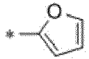 | 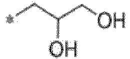 |
| II-E-47 | N | N | N | CH | CO₂CH₃ | CN | CO₂CH₃ | CN | 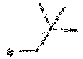 |
| II-E-48 | N | N | CH | N | OCH₃ | 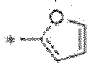 | OCH₃ | 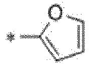 | 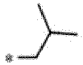 |
| II-E-49 | N | N | N | N | 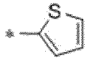 | Ph | 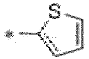 | Ph | 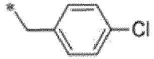 |
| II-E-50 | N | CH | CH | CH | 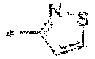 | 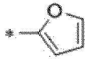 | 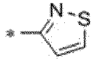 | 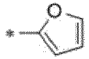 | 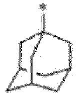 |
| II-E-51 | N | N | CH | CH | H | F | H | F | 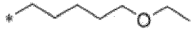 |
| II-E-52 | CH | N | CH | N | CN | CN | CN | CN | 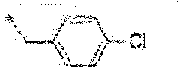 |
| II-E-53 | N | N | N | CH | CH₃ | 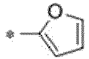 | CH₃ | 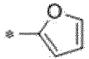 | 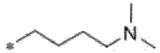 |
| II-E-54 | N | N | CH | N | 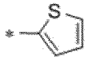 | 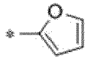 | 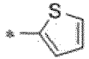 | 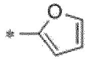 | 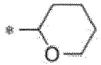 |
| II-E-55 | N | N | N | N | 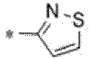 | H | 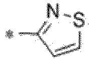 | H | 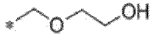 |
| II-E-56 | N | CH | CH | CH | N(CH₃)₂ | 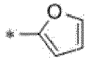 | N(CH₃)₂ | 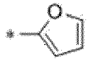 | 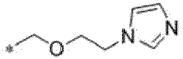 |
| II-E-57 | N | N | CH | CH | C OOH | CN | C OOH | CN | 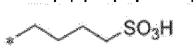 |
| II-E-58 | CH | N | CH | N | CO₂CH₃ | 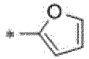 | CO₂CH₃ | 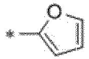 | 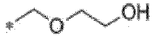 |
| II-E-59 | N | N | N | CH | OCH₃ | Ph | OCH₃ | Ph | 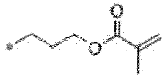 |
| II-E-60 | N | N | CH | N | 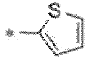 | 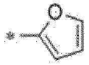 | 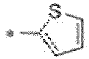 | 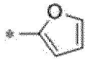 | 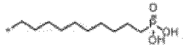 |
| II-E-61 | N | N | N | N | 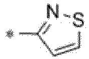 | F | 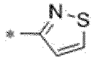 | F | 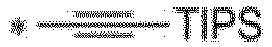 |

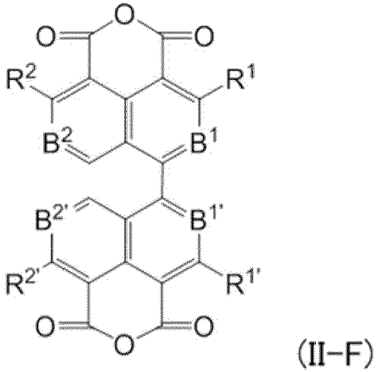

| No. | B¹ | B² | B^{1'} | B^{2'} | R¹ | R² | R^{1'} | R^{2'} |
|---|---|---|---|---|---|---|---|---|
| II-F-1 | N | CH | CH | CH | H | H | H | H |
| II-F-2 | N | N | CH | CH | H | H | H | H |
| II-F-3 | CH | N | CH | N | H | H | H | H |
| II-F-4 | N | N | N | CH | H | H | H | H |
| II-F-5 | N | N | CH | N | H | H | H | H |
| II -F-6 | N | N | N | N | H | H | H | H |

From the viewpoint of improved carrier mobility and durability of the organic semiconductor film formed by using the specific azaperylene compound, and material stability, the molecular weight of the intermediate II is preferably 350 or more, more preferably 400 or more, and still more preferably 500 or more. In addition, from the viewpoint of more excellent solubility, the molecular weight of the intermediate II is preferably 3000 or less, more preferably 2000 or less, and still more preferably 1000 or less.

### <Method for producing intermediate II>

The method for producing the intermediate II is not particularly limited, and the intermediate II can be synthesized with reference to a known method.

For example, the intermediate II can be produced by performing a step of reacting (coupling-reacting) the intermediate I with a compound (hereinafter, also referred to as an "intermediate III") represented by Formula (III) in the presence of a transition metal catalyst to produce the intermediate II.

Hereinafter, the method for producing the intermediate II will be described by taking the coupling reaction between the intermediate I and the intermediate III as an example.

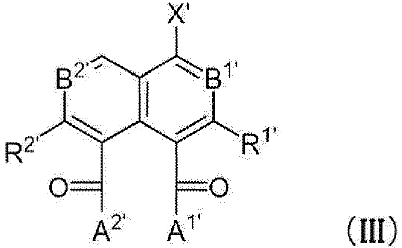

A^{1'}, A^{2'}, R^{1'}, R^{2'}, B^{1'}, and B^{2'} in Formula (III) are respectively the same as A^{1'}, A^{2'}, R^{1'}, R^{2'}, B^{1'}, and B^{2'} in Formula (II), and the suitable aspects thereof are also the same.

X' in Formula (III) represents a hydrogen atom, a leaving group, or an atomic group (hereinafter, also referred to as an "atomic group N'") having a metal atom or a metalloid atom, which is bonded to a carbon atom in an azanaphthalene ring. Here, the leaving group and atomic group N' represented by X' in Formula (III) are the same as the leaving group and atomic group N represented by X in Formula (II), and the suitable aspects thereof are the same.

As is clear from the provisions of Formula (I) and Formula (III), the intermediate III corresponds to a superordinate concept including the intermediate I. Therefore, in the present specification, the description of "producing the intermediate II by reacting the intermediate I with the intermediate III" includes a case where two identical or different compounds included in the intermediate I are reacted to produce the intermediate II.

In the coupling reaction, it is preferable that at least one of X in the intermediate I or X' in the intermediate III is a leaving group, and it is more preferable that only one thereof is a leaving group.

In other words, in a case where X in the intermediate I is a leaving group, X' in the intermediate III is preferably a hydrogen atom or the atomic group N', and in a case where X in the intermediate I is the atomic group N, X' in the intermediate III is preferably a leaving group.

As a specific combination of X in the intermediate I and X' in the intermediate III, a combination in which X is a halogen atom, an alkylsulfonyloxy group which may have a substituent, or an arylsulfonyloxy group which may have a substituent, and X' is a hydrogen atom, a group represented by -Sn(Y¹)₃, a group represented by -B(OY³)₂, or a group represented by -Si(Y⁴)₃, or a combination in which X is a group represented by -Sn(Y¹)₃, a group represented by -B(OY³)₂, or a group represented by -Si(Y⁴)₃, and X' is a halogen atom, an alkylsulfonyloxy group which may have a substituent, or an arylsulfonyloxy group which may have a substituent is preferable; and a combination in which X is a chlorine atom, a bromine atom, or an iodine atom, and X' is a hydrogen atom, -B(OH)₂, a boronic acid pinacol ester group, a boronic acid trimethylene glycol ester group, or a boronic acid catechol ester group, or a combination in which X is -B(OH)₂, a boronic acid pinacol ester group, a boronic acid trimethylene glycol ester group, or a boronic acid catechol ester group, and X' is a chlorine atom, a bromine atom, or an iodine atom is more preferable.

The type of the coupling reaction between the intermediate I and the intermediate III performed in the presence of a transition metal catalyst is not particularly limited, and the intermediate II can be synthesized by referring to a cross-coupling method well known in the field of organic chemistry.

The amounts of the intermediate I and the intermediate III used in the coupling reaction are not particularly limited. For example, in a case where only one of the intermediate I and the intermediate III has a leaving group, among the intermediate I and the intermediate III, the ratio of blending amount of a compound having no leaving group to a compound having the leaving group is preferably 1.0 to 3.0 molar equivalents, more preferably 1.0 to 2.0 molar equivalents, and still more preferably 1.1 to 1.5 molar equivalents.

The type of the transition metal catalyst used in the coupling reaction is appropriately selected according to X in the intermediate I and X' in the intermediate III.

The transition metal catalyst is not particularly limited, and examples thereof include a palladium catalyst (palladium(0) catalyst or palladium(II) catalyst), a nickel catalyst (nickel(0) catalyst), an iron catalyst (iron(III) catalyst), a cobalt catalyst (cobalt(II) catalyst), and an iridium catalyst (iridium(0) catalyst). Among these, a palladium catalyst or a nickel catalyst is preferable, and a palladium catalyst is more preferable.

The palladium catalyst is not particularly limited, and examples thereof include palladium acetate, tris(dibenzylideneacetone)dipalladium(0) chloroform complex, tetrakis(triphenylphosphine)palladium(0), and dichlorobis[di-tert-butyl(4-dimethylaminophenyl)phosphine]palladium(II).

The amount of the transition metal catalyst used in the coupling reaction is not particularly limited as long as the transition metal catalyst acts as a catalyst, and is preferably 0.001 to 2 mol and more preferably 0.01 to 0.5 mol with respect to 1 mol of the compound having the leaving group, among the intermediate I and the intermediate III.

The coupling reaction may be performed in the presence of a base, depending on the types of the intermediate I and the intermediate III.

Examples of the base include an organic base and an inorganic base, and an inorganic base is preferable. Examples of the inorganic base include hydroxides, carbonates, phosphates, and acetates. More specific examples thereof include sodium hydroxide, potassium hydroxide, cesium hydroxide, sodium carbonate, potassium carbonate, cesium carbonate, sodium phosphate, potassium phosphate, and sodium acetate.

The base may be used alone or in combination of two or more thereof.

The amount of the base used is not particularly limited, and is preferably 0.1 to 10 mol and more preferably 1 to 5 mol with respect to 1 mol of the compound having the leaving group, among the intermediate I and the intermediate III.

The coupling reaction may be performed in the presence of a solvent. The solvent may be used alone or in combination of two or more thereof.

Examples of the solvent include aromatic hydrocarbon solvents including toluene and xylene, ether solvents including diethyl ether, dibutyl ether, dioxane, and tetrahydrofuran, aliphatic saturated hydrocarbon solvents including pentane and hexane, alcohol solvents including methanol and ethanol, and water.

The coupling reaction may be performed under an inert gas atmosphere. Examples of the inert gas include nitrogen, helium, and argon.

The reaction temperature of the coupling reaction is not particularly limited and varies depending on the types of the intermediate I and the intermediate III, but is preferably 0°C to 100°C and more preferably 20°C to 70°C.

The reaction time of the coupling reaction varies depending on the solvent used and reaction conditions including the reaction temperature, but is, for example, 1 to 24 hours, preferably 3 to 20 hours.

After completion of the coupling reaction, as necessary, the obtained product of the intermediate II may be purified by a separation and purification method including washing, extraction, drying, filtration, concentration, recrystallization, and column purification.

### [Azaperylene compound (specific azaperylene compound) represented by Formula (IV)]

Subsequently, the specific azaperylene compound produced by using the intermediate II will be described. The specific azaperylene compound is an azaperylene compound represented by Formula (IV).

In the present specification, the "azaperylene compound" means a compound having an azaperylene skeleton in which at least one of carbon atoms at 1, 6, 7, and 12 positions of perylene is replaced with a nitrogen atom.

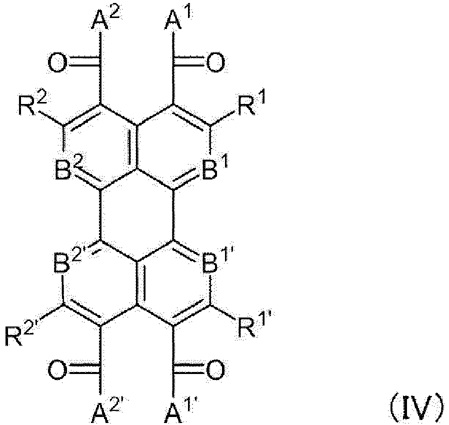

A¹, A², A^{1'}, A^{2'}, R¹, R² R^{1'},R^{2'}, B¹, B², B^{1'}, and B^{2'} in Formula (IV) are respectively the same as A¹, A², A^{1'}, A^{2'}, R¹, R² R^{1'},R^{2'}, B¹, B², B^{1'}, and B^{2'} in Formula (II), and the suitable aspects thereof are also the same.

In Formula (IV), in each of the combination of A¹ and A² and the combination of A^{1'} and A^{2'}, the groups may be the same or different from each other, but it is preferable that the groups are the same, and it is more preferable that, in both of the combination of A¹ and the A² and the combination of A^{1'}and A^{2'}, the groups are integrated to form -N(R⁶)-.

From the viewpoint of improved carrier mobility, durability, and material stability, the molecular weight of the specific azaperylene compound is preferably 350 or more, more preferably 400 or more, and still more preferably 500 or more. In addition, from the viewpoint of excellent solubility, the molecular weight of the specific azaperylene compound is preferably 3000 or less, more preferably 2000 or less, and still more preferably 1000 or less.

The specific azaperylene compound produced by using the intermediate II exhibits properties as a semiconductor, and by using the specific azaperylene compound for an organic semiconductor film included in an organic thin film transistor, the carrier mobility of the organic semiconductor film can be improved, and the amount of carrier mobility decrease in the atmosphere with time can be suppressed.

### <Method for producing specific azaperylene compound>

The method for producing the specific azaperylene compound includes a step of ring-closing the intermediate II. More specifically, the specific azaperylene compound can be produced by performing a ring closure reaction in which the 6-membered aromatic ring containing B² and the 6-membered aromatic ring containing B^{2'} in the molecule of the intermediate II are bonded to each other at the closest positions to each other, thereby forming an azaperylene skeleton structure.

Such a ring closure reaction of the intermediate II can be performed according to a known method, for example, a method described in Peter D. Frischmann et. al., Org. Lett., vol. 15, No. 18, 2013, p. 4674.

The ring closure reaction of the intermediate II is preferably performed in the presence of a base.

The type of the base used for the ring closure reaction of the intermediate II is not particularly limited, and the bases exemplified as the bases which can be used for the coupling reaction described above can be used. Among these, an inorganic base is preferable, a hydroxide or a carbonate is more preferable, and sodium hydroxide, potassium hydroxide, sodium carbonate, or potassium carbonate is still more preferable.

The base may be used alone or in combination of two or more thereof.

The amount of the base used is not particularly limited, and is preferably 5 to 30 mol and more preferably 10 to 20 mol with respect to 1 mol of the intermediate II.

The ring closure reaction of the intermediate II may be performed in the presence of a solvent. The solvent may be used alone or in combination of two or more thereof.

The solvent is not particularly limited, and examples thereof include an amine solvent, a hydrocarbon solvent, an ether solvent, and a sulfoxide solvent.

Examples of the amine solvent include dimethylaminoethanol, ethylenediamine, methylamine, dimethylamine, trimethylamine, ethylamine, diethylamine, triethylamine, pyridine, and N,N-dimethylformamide.

Examples of the hydrocarbon solvent include pentane, hexane, octane, decane, toluene, xylene, mesitylene, ethylbenzene, amylbenzene, decalin, 1-methylnaphthalene, 1-ethylnaphthalene, 1,6-dimethylnaphthalene, and tetralin.

Examples of the ether solvent include diethyl ether, dibutyl ether, dioxane, and tetrahydrofuran.

Examples of the sulfoxide solvent include dimethyl sulfoxide and sulfolane.

From the viewpoint of being used for the ring closure reaction under heating conditions, a solvent having a boiling point of 70°C or higher is preferable, and a solvent having a boiling point of 90°C or higher is more preferable. Among the above-described solvents, examples of the solvent having a boiling point of 90°C or higher include dimethylaminoethanol, toluene, xylene, chlorobenzene, dichlorobenzene, dimethyl formamide, dimethyl acetamide, dimethyl sulfoxide, and sulfolane.

The reaction temperature of the ring closure reaction of the intermediate II is not particularly limited, but is preferably 70°C to 200°C and more preferably 100°C to 150°C.

The reaction time of the ring closure reaction of the intermediate II varies depending on the solvent used and reaction conditions including the reaction temperature, but is, for example, 1 to 24 hours, preferably 3 to 20 hours.

After completion of the ring closure reaction of the intermediate II, as necessary, the obtained specific azaperylene compound may be purified by a separation and purification method including washing, extraction, drying, filtration, concentration, recrystallization, and column purification.

In the intermediate II, in a case where at least one of A¹, A², A^{1'}, or A^{2'} represents -OR³ or -NR⁴R⁵ (in other words, in a case where any one of A¹ and A², or A^{1'}and A^{2'} is not integrated to form -O- or -N(R⁶)-), along with the ring closure reaction of the intermediate II, a reaction of forming a 6-membered ring containing an oxygen atom or a nitrogen atom, in which A¹ and A², and A^{1'}and A^{2'} are integrated to form -O- or -N(R⁶)-, may be performed.

For example, by performing the ring closure reaction of the intermediate II in which both A¹ and A² represent -OR³ in the presence of an organic amine represented by NH₂R⁶, a specific azaperylene compound having -N(R⁶)- which is formed by integrating A¹ and A² can be synthesized.

The amount of the organic amine having an R⁶ group, used in a case of performing the ring formation reaction of A¹ and A², and A^{1'}and A^{2'} in the intermediate II, is not particularly limited, but is preferably 1 to 10 molar equivalents and more preferably 1.5 to 3 molar equivalents with respect to the total of a combination of A¹ and A², and A^{1'}and A^{2'}, in which no ring is formed.

In a case where any one of A¹ and A², or A^{1'}and A^{2'} in the intermediate II does not form a ring, the above-described ring formation reaction may be performed on such an intermediate II before the ring closure reaction is performed. The solvent used in this case and the reaction conditions may be set according to the ring closure reaction of the intermediate II.

As described later, the specific azaperylene compound obtained by the ring closure reaction of the intermediate II is used as a material for forming an organic semiconductor film included in an organic thin film transistor. In addition, the specific azaperylene compound can also be used, for example, in an organic semiconductor film included in a non-luminescent organic semiconductor device. The non-luminescent organic semiconductor device means a device which does not have a purpose of emitting light.

Examples of such a device include an organic thin film transistor which controls the amount of current or the amount of voltage, an organic photoelectric conversion element which converts light energy into electric power (for example, a solid-state imaging element for light sensors and a solar cell for energy conversion), an organic thermoelectric conversion element which converts thermal energy into electric power, a gas sensor, an organic rectifying element, an organic inverter, and an information recording element.

### [Composition for organic thin film transistor]

Next, a composition for an organic thin film transistor will be described.

The composition for an organic thin film transistor (also simply referred to as an "organic semiconductor composition" in the present specification) contains a specific azaperylene compound and is used for forming an organic semiconductor film of an organic thin film transistor.

The specific azaperylene compound contained in the organic semiconductor composition is as described above, and may be used alone or in combination of two or more thereof.

The content of the specific azaperylene compound in the organic semiconductor composition can be represented by a solid content excluding the solvent described later, and for example, it is preferable that the content of the specific azaperylene compound with respect to the total mass of the solid content in the organic semiconductor composition is included in a suitable range of the content of the specific azaperylene compound with respect to the total mass of the organic semiconductor film described later.

### <Binder polymer>

The organic semiconductor composition may contain a binder polymer. From the viewpoint that an organic semiconductor film having high film quality can be obtained, the organic semiconductor composition preferably contains a binder polymer.

The type of the binder polymer is not particularly limited, and a known binder polymer can be used. Examples of the binder polymer include an insulating polymer including polystyrene, poly(α-methylstyrene), polycarbonate, polyarylate, polyester, polyamide, polyimide, polyurethane, polysiloxane, polysulfone, polymethyl methacrylate, polymethyl acrylate, cellulose, polyethylene, and polypropylene, and a copolymer thereof.

In addition to these, examples of the binder polymer include a rubber including an ethylene-propylene rubber, an acrylonitrile-butadiene rubber, a hydrogenated nitrile rubber, a fluoro-rubber, a perfluoro elastomer, a tetrafluoroethylene-propylene copolymer, an ethylene-propylene-diene copolymer, a styrene-butadiene rubber, polychloroprene, polyneoprene, a butyl rubber, a methylphenyl silicone resin, a methylphenylvinyl silicone resin, a methylvinyl silicone resin, a fluorosilicone resin, an acryl rubber, an ethylene acryl rubber, chlorosulfonated polyethylene, chloropolyethylene, an epichlorohydrin copolymer, a polyisoprene-natural rubber copolymer, a polyisoprene rubber, a styrene-isoprene block copolymer, a polyester-urethane copolymer, a polyether-urethane copolymer, a polyether ester thermoplastic elastomer, and a polybutadiene rubber, and a thermoplastic elastomer polymer.

Furthermore, examples of the binder polymer include a photoconductive polymer including polyvinylcarbazole and polysilane, a conductive polymer including polythiophene, polypyrrole, polyaniline, and poly p-phenylenevinylene, and a semiconductive polymer described in Chemistry of Materials, 2014, 26, p. 647.

In consideration of charge mobility, it is preferable that the binder polymer has a structure not including a polar group. Here, the polar group refers to a functional group having a heteroatom other than carbon atoms and hydrogen atoms. Since it has a structure not including a polar group, polystyrene or poly(α-methylstyrene) is preferable as the binder polymer. In addition, a semiconductive polymer is also preferable.

The glass transition temperature of the binder polymer is not particularly limited, and is appropriately set according to the use. For example, in a case of imparting firm mechanical strength to the organic semiconductor film, it is preferable that the glass transition temperature is set to be high. On the other hand, in a case of imparting flexibility to the organic semiconductor film, it is preferable that the glass transition temperature is set to be low.

The binder polymer may be used singly, and two or more kinds thereof may be used in combination.

The content of the binder polymer in the organic semiconductor composition is not particularly limited, but from the viewpoint that the carrier mobility and durability of the organic semiconductor film of the organic thin film transistor are further improved, it is preferable that the content of the binder polymer with respect to the total mass of the solid content in the organic semiconductor composition is included in a suitable range of the content of the binder polymer with respect to the total mass of the organic semiconductor film described later.

The weight-average molecular weight of the binder polymer is not particularly limited, and is preferably 1,000 to 10,000,000, more preferably 3,000 to 5,000,000, and still more preferably 5,000 to 3,000,000. The weight-average molecular weight of the binder polymer can be obtained by gel permeation chromatography (GPC).

In the organic semiconductor composition, the specific azaperylene compound may be uniformly mixed with the binder polymer, or a part or all of the specific azaperylene compound may be phase-separated from the binder polymer. From the viewpoint of ease of application or uniformity of application, it is preferable that the specific azaperylene compound and the binder polymer are uniformly mixed at least in a case of application.

### <Solvent>

The organic semiconductor composition may contain a solvent, and from the viewpoint of improving coatability thereof, the organic semiconductor composition preferably contains a solvent. Such a solvent is not particularly limited as long as the solvent dissolves or disperses the above-described compounds, examples thereof include an inorganic solvent and an organic solvent, and an organic solvent is preferable. The solvent may be used alone or in combination of two or more kinds thereof.

The organic solvent is not particularly limited, and examples thereof include hydrocarbon solvents including hexane, octane, decane, toluene, xylene, mesitylene, ethylbenzene, amylbenzene, decalin, 1-methylnaphthalene, 1-ethylnaphthalene, 1,6-dimethylnaphtalene, and tetralin; ketone solvents including acetone, methyl ethyl ketone, methyl isobutyl ketone, cyclohexanone, acetophenone, propiophenone, and butyrophenone; halogenated hydrocarbon solvents including dichloromethane, chloroform, tetrachloromethane, dichloroethane, trichloroethane, tetrachloroethane, chlorobenzene, 1,2-dichlorobenzene, 1,2,4-trichlorobenzene, chlorotoluene, and 1-fluoronaphthalene; heterocyclic solvents including pyridine, picoline, quinoline, thiophene, 3-butylthiophene, and thieno[2,3-b]thiophene; halogenated heterocyclic solvents including 2-chlorothiophene, 3-chlorothiophene, 2,5-dichlorothiophene, 3,4-dichlorothiophene, 2-bromothiophene, 3-bromothiophene, 2,3-dibromothiophene, 2,4-dibromothiophene, 2,5-dibromothiophene, 3,4-dibromothiophene, and 3,4-dichloro-1,2,5-thiadiazole; ester solvents including ethyl acetate, butyl acetate, amyl acetate, 2-ethylhexyl acetate, γ-butyrolactone, and phenyl acetate; alcohol solvents such as methanol, propanol, butanol, pentanol, hexanol, cyclohexanol, methyl cellosolve, ethyl cellosolve, and ethylene glycol; ether solvents including dibutyl ether, tetrahydrofuran, dioxane, dimethoxyethane, anisole, ethoxybenzene, propoxybenzene, isopropoxybenzene, butoxybenzene, 2-methylanisole, 3-methylanisole, 4-methylanisole, 4-ethylanisole, dimethylanisole (any one of 2,3-, 2,4-, 2,5-, 2,6-, 3,4-, 3,5-, or 3,6-), and 1,4-benzodioxane; amide or imide solvents including N,N-dimethylformamide, N,N-dimethylacetamide, 1-methyl-2-pyrrolidone, 1-methyl-2-imidazolidinone, and 1,3-dimethyl-2-imidazolidinone; sulfoxide solvents including dimethyl sulfoxide; phosphoric acid ester solvents including trimethyl phosphoric acid; nitrile solvents including acetonitrile and benzonitrile; and nitro solvents including nitromethane and nitrobenzene.

Among these, a hydrocarbon solvent, a ketone solvent, a halogenated hydrocarbon solvent, a heterocyclic solvent, a halogenated heterocyclic solvent, or an ether solvent is preferable; toluene, xylene, mesitylene, amylbenzene, tetraline, acetophenone, propiophenone, butyrophenone, dichlorobenzene, anisole, ethoxybenzene, propoxybenzene, isopropoxybenzene, butoxybenzene, 2-methylanisole, 3-methylanisole, 4-methylanisole, 1-fluoronaphthalene, 3-chlorothiophene, or 2,5-dibromothiophene is more preferable; and toluene, xylene, tetraline, acetophenone, propiophenone, butyrophenone, anisole, ethoxybenzene, propoxybenzene, butoxybenzene, 2-methylanisole, 3-methylanisole, 4-methylanisole, 1-fluoronaphthalene, 3-chlorothiophene, or 2,5-dibromothiophene is still more preferable.

From the viewpoint of film quality, and viewpoint that crystals of the above-described compounds can be enlarged, as the solvent contained in the organic semiconductor composition is preferably a solvent having a boiling point of 100°C or higher.

Examples of the solvent having a boiling point of 100°C or higher include toluene, xylene, mesitylene, tetraline, acetophenone, propiophenone, butyrophenone, dichlorobenzene, anisole, ethoxybenzene, propoxybenzene, isopropoxybenzene, butoxybenzene, 2-methylanisole, 3-methylanisole, and 4-methylanisole. Among these, toluene, xylene, tetraline, acetophenone, propiophenone, butyrophenone, anisole, ethoxybenzene, propoxybenzene, butoxybenzene, 2-methylanisole, 3-methylanisole, or 4-methylanisole is preferable.

In addition, from the viewpoint of environmental load and toxicity to humans, a non-halogen solvent (solvent having no halogen atom in the molecule) is preferable as the solvent having a boiling point of 100°C or higher.

In a case where the organic semiconductor composition contains a solvent, the content of the solvent is preferably 90% to 99.9% by mass, more preferably 95% to 99.9% by mass, and still more preferably 96% to 99.5% by mass with respect to the total mass of the organic semiconductor composition.

### <Other components>

The organic semiconductor composition may contain a component other than the specific azaperylene compound, the binder polymer, and the solvent. Examples of such a component include various additives.

As the additive, an additive used in the organic semiconductor composition can be used, and more specific examples thereof include a surfactant, an antioxidant, a crystallization control agent, and a crystal orientation control agent. As the surfactant and the antioxidant, paragraphs 0136 and 0137 of JP2015-195362A can be incorporated, and the contents thereof are incorporated in the present specification.

The content of the additive in the organic semiconductor composition is not particularly limited, but from the viewpoint that the organic semiconductor composition has excellent film forming property, and the carrier mobility and heat resistance are further improved, it is preferable that the content of the additive with respect to the total mass of the solid content in the organic semiconductor composition is included in a suitable range of the content of the additive with respect to the total mass of the organic semiconductor film described later.

From the viewpoint of high water resistance, the viscosity of the organic semiconductor composition is preferably 10 mPa·s or more.

### <Preparation method>

The method of preparing the organic semiconductor composition is not particularly limited, and a known preparation method can be adopted. For example, it is possible to prepare the organic semiconductor composition by adding respective components in a predetermined amount to an organic solvent and stirring the mixture appropriately.

The respective components can be heated during or after stirring as necessary. The heating temperature is not particularly limited, and is determined, for example, in a range of 40°C to 150°C. In a case of using the solvent, the heating temperature is determined to be a temperature in the above-described range and lower than the boiling point of the solvent.

### [Organic thin film transistor]

Next, an organic thin film transistor (hereinafter, also referred to as an "organic TFT"), which is a preferred form among the above-described organic semiconductor devices using the specific azaperylene compound, will be described.

The organic TFT includes an organic semiconductor film described later. As a result, the organic TFT exhibits high carrier mobility and can effectively suppress a decrease over time even in the atmosphere, thereby driving stably. The ambient temperature or humidity in the atmosphere is not particularly limited as long as a temperature or humidity in the usage environment of the organic TFT, and examples thereof include room temperature (20°C) as a temperature and 10 to 90 RH% as a humidity.

The organic TFT is preferably used as an organic field effect transistor (FET) and more preferably used as an insulated gate type FET in which the gate and the channel are insulated.

The thickness of the organic TFT is not particularly limited, but in a case of a thinner transistor, for example, the thickness of the entire organic TFT is preferably 0.1 to 0.5 µm.

The organic TFT includes an organic semiconductor film (also referred to as an organic semiconductor layer or a semiconductor active layer) including the specific azaperylene compound, and can further include a source electrode, a drain electrode, a gate electrode, and a gate insulating film.

It is preferable that the organic TFT includes a gate electrode, an organic semiconductor film, a gate insulating film provided between the gate electrode and the organic semiconductor film, and a source electrode and a drain electrode which are provided in contact with the organic semiconductor film and are linked to each other through the organic semiconductor film, on a substrate. In the organic TFT, the organic semiconductor film and the gate insulating film are provided to be adjacent to each other.

The structure of the organic TFT is not particularly limited as long as the above-described respective layers are provided. For example, the organic TFT may have any structures of a bottom gate-bottom contact type, a top gate-bottom contact type, a bottom gate-top contact type, or a top gate-top contact type. The organic TFT is preferably a bottom gate type (bottom gate-bottom contact type or bottom gate-top contact type) in which the gate electrode is provided between the substrate and the organic semiconductor film.

Hereinafter, an example of the organic TFT will be described with reference to the drawings.

### <Bottom gate-bottom contact type organic TFT>

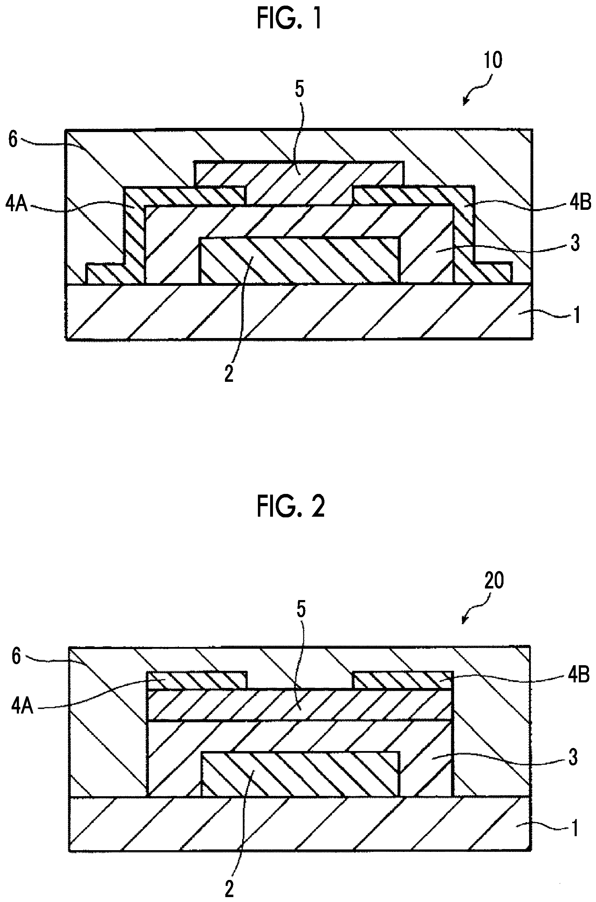

Fig. 1 is a schematic cross-sectional view showing a structure of a bottom gate-bottom contact type organic TFT 10 which is an example of the organic TFT.

As illustrated in Fig. 1, the organic TFT 10 has a substrate (base material) 1, a gate electrode 2, a gate insulating film 3, a source electrode 4A and a drain electrode 4B, an organic semiconductor film 5, and a sealing layer 6, in this order.

Hereinafter, a substrate (base material), a gate electrode, a gate insulating film, a source electrode, a drain electrode, an organic semiconductor film, a sealing layer, and a manufacturing method thereof will be described in detail.

### (Substrate)

The substrate acts as supporting the gate electrode, the source electrode, the drain electrode, and other layers.

The type of the substrate is not particularly limited, and examples thereof include a plastic substrate, a silicon substrate, a glass substrate, and a ceramic substrate. Among these, from the viewpoint of applicability to each device and costs, a glass substrate or a plastic substrate is preferable.

The thickness of the substrate is not particularly limited. The upper limit of the thickness of the substrate is preferably 10 mm or less, more preferably 2 mm or less, and still more preferably 1.5 mm or less. The lower limit of the thickness of the substrate is preferably 0.01 mm or more and more preferably 0.05 mm or more.

### (Gate electrode)

As the gate electrode, an electrode which is used as a gate electrode of an organic TFT can be applied without particular limitation.

A material (electrode material) for forming the gate electrode is not particularly limited, and examples thereof include metals including gold, silver, aluminum, copper, chromium, nickel, cobalt, titanium, platinum, magnesium, calcium, barium, and sodium; conductive oxides including InO2, SnO2, and indium tin oxide (ITO); conductive polymers including polyaniline, polypyrrole, polythiophene, polyacetylene, and polydiacetylene; semiconductors including silicon, germanium, and gallium arsenide; and carbon materials including fullerene, carbon nanotube, and graphite. Among these, the above-described metals are preferable, and silver or aluminum is more preferable.

The thickness of the gate electrode is not particularly limited, but is preferably 20 to 200 nm.

The gate electrode may function as the substrate, and in this case, the above-described substrate may not be provided.

The method of forming the gate electrode is not particularly limited, and examples thereof include a method of performing vacuum deposition (hereinafter, simply referred to as "vapor deposition") of or sputtering the above-described electrode material on the substrate, and a method of applying or printing a composition for forming an electrode, which contains the above-described electrode material, on the substrate. In addition, in a case of patterning the gate electrode, examples of the patterning method include printing methods including inkjet printing, screen printing, offset printing, and relief printing (flexographic printing), a photolithography method, and a mask vapor deposition method.

### (Gate insulating film)

The gate insulating film is not particularly limited as long as the gate insulating film is a layer having insulating properties, and may be a single layer or a multilayer.

The material for forming the gate insulating film is not particularly limited, and examples thereof include polymers including polymethyl methacrylate, polystyrene, polyvinyl phenol, melamine resin, polyimide, polycarbonate, polyester, polyvinyl alcohol, polyvinyl acetate, polyurethane, polysulfone, polybenzoxazole, polysilsesquioxane, epoxy resin, and phenol resin; inorganic oxides including silicon dioxide, aluminum oxide, and titanium oxide; and nitrides including silicon nitride. Among these, from the viewpoint of compatibility with the organic semiconductor film, the above-described polymers are preferable, and from the viewpoint of uniformity of the film, the above-described inorganic oxides are preferable and silicon dioxide is more preferable.

These materials may be used singly, and two or more kinds thereof may be used in combination.

The film thickness of the gate insulating film is not particularly limited, but is preferably 100 to 1,000 nm.

The method of forming the gate insulating film is not particularly limited, and examples thereof include a method of applying a composition for forming a gate insulating film, which contains the above-described material, on the substrate on which the gate electrode is formed, and a method of performing vapor deposition of or sputtering the above-described material.

### (Source electrode and drain electrode)

In the organic TFT, the source electrode is an electrode in which a current flows from the outside through a wiring. In addition, the drain electrode is an electrode in which a current is sent to the outside through a wiring.

As materials for forming the source electrode and the drain electrode, the same materials as the electrode material for forming the above-described gate electrode can be used. Among them, metal is preferable, and gold or silver is more preferable.

The thicknesses of the source electrode and the drain electrode are not particularly limited, but are respectively preferably 1 nm or more and more preferably 10 nm or more. In addition, the upper limit of the thicknesses of the source electrode and the drain electrode is preferably 500 nm or less and more preferably 300 nm or less.

The distance (gate length L) between the source electrode and the drain electrode may be appropriately determined, but for example, the distance is preferably 200 µm or less and more preferably 100 µm or less. In addition, the gate width W may be appropriately determined, but the gate width W is preferably 5000 µm or less and more preferably 1000 µm or less. A ratio of the gate width W to the gate length L is not particularly limited, but for example, the ratio W/L is preferably 10 or more and more preferably 20 or more.

The method of forming the source electrode and the drain electrode is not particularly limited, and examples thereof include a method of performing vacuum deposition of or sputtering the electrode material on the substrate on which the gate electrode and the gate insulating film are formed, and a method of applying or printing a composition for forming an electrode on the substrate. In a case of patterning the source electrode and the drain electrode, the patterning method is the same as the method of the gate electrode described above.

### (Organic semiconductor film)

As the organic semiconductor film in the organic TFT, an organic semiconductor film containing the specific azaperylene compound is used. The specific azaperylene compound contained in the organic semiconductor film may be one kind or two or more kinds.

In a case where the organic semiconductor film contains the specific azaperylene compound, the carrier mobility of the organic semiconductor film can be improved, and the high carrier mobility can be maintained even in a case of being used or stored (left) in the atmosphere. The reason is not clear, but it is considered that the orbital energy of the lowest unoccupied molecular orbital of the specific azaperylene compound is low.

The content of the specific azaperylene compound in the organic semiconductor film can be appropriately set without particular limitation. For example, the content of the specific azaperylene compound with respect to the total mass of the organic semiconductor film is preferably 10% by mass or more, more preferably 30% by mass or more, still more preferably 50% by mass or more. The upper limit thereof is not particularly limited, and the content of the specific azaperylene compound with respect to the total mass of the organic semiconductor film may be 100% by mass. In a case where the organic semiconductor film contains the binder polymer or other components, the upper limit of the content of the specific azaperylene compound with respect to the total mass of the organic semiconductor film is preferably 90% by mass or less and more preferably 80% by mass or less.

The organic semiconductor film may contain the above-described binder polymer, in addition to the specific azaperylene compound. The binder polymer may be used singly, and two or more kinds thereof may be used in combination.

In the organic semiconductor film, a state of the specific azaperylene compound and the binder polymer contained is not particularly limited, but from the viewpoint of carrier mobility, it is preferable that the specific azaperylene compound and the binder polymer are phase-separated from each other along a film thickness direction.

The content of the binder polymer in the organic semiconductor film can be appropriately set without particular limitation. In a case where the organic semiconductor film contains the binder polymer, the content of the binder polymer with respect to the total mass of the organic semiconductor film is preferably 90% by mass or less and more preferably 70% by mass or less. The lower limit thereof is not particularly limited, and the content of the binder polymer with respect to the total mass of the organic semiconductor film may be 0% by mass or more, preferably 10% by mass or more, and more preferably 20% by mass or more.

The organic semiconductor film may contain the above-described additive, in addition to the specific azaperylene compound. The additive may be used singly or in combination of two or more kinds thereof.

In a case where the organic semiconductor film contains the additive, the content of the additive with respect to the total mass of the organic semiconductor film is preferably 10% by mass or less, more preferably 5% by mass or less, and still more preferably 1% by mass or less.

The film thickness of the organic semiconductor film is appropriately determined depending on the organic TFT to be applied, but is preferably 10 to 500 nm, more preferably 20 to 200 nm.

This organic semiconductor film can be formed by applying the above-described organic semiconductor composition. Details of a method for forming the organic semiconductor film will be described later.

The use of the organic semiconductor film containing the specific azaperylene compound is not limited to the organic semiconductor film for the organic TFT, and the organic semiconductor film can be used as an organic semiconductor film included in each of the above-described organic semiconductor devices.

### (Sealing layer)

Since the organic TFT provided with the above-described organic semiconductor film is stably driven even in the atmosphere, it is not necessary to seal the entire organic TFT and block either the atmosphere (oxygen gas) or moisture, but for the purpose of stable driving for a longer period of time, the entire organic TFT may be sealed with a metal sealing can, or a sealing layer may be formed using a sealing agent.

As the sealing layer, a sealing agent (composition for forming a sealing layer) used for an organic TFT can be used. Examples of the sealing agent include inorganic materials including glass and silicon nitride, polymer materials including parylene, and low molecular weight materials.

The sealing layer can be formed by a known method such as coating and drying, using the above-described sealing agent.

The thickness of the sealing layer is not particularly limited, but is preferably 0.2 to 10 µm.

### <Bottom gate-top contact type Organic TFT>

Fig. 2 is a schematic cross-sectional view showing a structure of a bottom gate-top contact type organic TFT 20 which is an example of the organic TFT.

As illustrated in Fig. 2, the organic TFT 20 has a substrate 1, a gate electrode 2, a gate insulating film 3, an organic semiconductor film 5, a source electrode 4A and a drain electrode 4B, and a sealing layer 6, in this order.

The organic TFT 20 is the same as the organic TFT 10, except that the layer configuration (lamination aspect) is different. Accordingly, the substrate, the gate electrode, the gate insulating film, the source electrode, the drain electrode, the organic semiconductor film, and the sealing layer are the same as those of the bottom gate-bottom contact type organic TFT, and thus descriptions thereof are omitted.

### <Method for producing organic TFT>

The method for producing the organic TFT is not particularly limited as long as the method includes a step of coating the substrate with the organic semiconductor composition to form an organic semiconductor film.

The gate electrode, the gate insulating film, the source electrode, the drain electrode, and the sealing layer can all be manufactured or formed by the method described above.

Hereinafter, the step of forming the organic semiconductor film will be described.

In this step, the above-described organic semiconductor composition is used.

In the present disclosure, the "coating the substrate with the organic semiconductor composition" includes an aspect of applying the organic semiconductor composition over the substrate through another layer provided on the substrate, in addition to an aspect of directly applying the organic semiconductor composition to the substrate. Another layer (a layer which is in contact with the organic semiconductor film and is a base of the organic semiconductor film) to be coated with the organic semiconductor composition is inevitably determined according to the structure of the organic TFT. For example, in a case where the organic TFT is a bottom gate type, the organic semiconductor composition is applied at least to the surface of the gate insulating film.

In a case of forming the organic semiconductor film, the substrate may be heated or cooled. By changing the temperature of the substrate, it is possible to control film quality or packing of the specific azaperylene compound in the film.

The temperature of the substrate is not particularly limited. For example, the temperature of the substrate is preferably set a range of 0°C to 200°C, more preferably in a range of 15°C to 100°C, and still more preferably in a range of 20°C to 95°C.

The method for forming the organic semiconductor film is not particularly limited as long as the organic semiconductor film including the specific azaperylene compound can be formed, and examples thereof include a vacuum process and a solution process. Among these, a solution process is preferable.

Examples of the vacuum process include a physical vapor deposition method including a vacuum evaporation method, a sputtering method, an ion plating method, and a molecular beam epitaxy (MBE) method, and a chemical vapor deposition (CVD) method including a plasma polymerization. Among these, a vacuum evaporation method is preferable.

As the solution process, it is preferable to use the above-described organic semiconductor composition.

The specific azaperylene compound is stable even in the atmosphere as described above. Therefore, the solution process can be performed in the atmosphere, and the organic semiconductor composition can be applied over a large area.

As a method for applying the organic semiconductor composition in the solution process, a known method can be used. Examples thereof include coating methods including a drop casting method, a casting method, a dip coating method, a die coater method, a roll coater method, a bar coater method, and a spin coating method; various printing methods including an inkjet method, a screen printing method, a gravure printing method, a flexography printing method, an offset printing method, and a microcontact printing method; and a Langmuir-Blodgett (LB) method. Among these, a drop casting method, a casting method, a spin coating method, an inkjet method, a gravure printing method, a flexography printing method, an offset printing method, or a microcontact printing method is preferable.

As a method for applying the organic semiconductor composition in the preferred aspect of the solution process described later, an inkjet method, a gravure printing method, a flexography printing method, an offset printing method, or a microcontact printing method is preferable, and a flexography printing method, a microcontact printing method, or an inkjet method is more preferable.

In the solution process, it is preferable to dry the organic semiconductor composition coated on the substrate, and it is more preferable to perform the drying gradually. By drying the organic semiconductor composition coated on the substrate, crystals of the specific azaperylene compound can be precipitated to form the organic semiconductor film.

From the viewpoint of film quality, as a method for drying the organic semiconductor composition, it is preferable that the organic semiconductor composition is naturally dried or dried by heating on a heated substrate and then dried under reduced pressure. The temperature of the substrate during natural drying or heat drying is preferably 20°C to 100°C and more preferably 50°C to 80°C. The time for natural drying or heat drying is preferably 0.5 to 20 hours and more preferably 1 to 10 hours.

The temperature of the substrate during drying under reduced pressure is preferably 20°C to 100°C and more preferably 40°C to 80°C. The time for drying under reduced pressure is preferably 1 to 20 hours and more preferably 2 to 10 hours. The pressure during drying under reduced pressure is preferably 10⁻⁶ to 10⁻² Pa and more preferably 10⁻⁵ to 10⁻³ Pa.

The organic semiconductor composition dried as described above may be shaped into a predetermined shape or a pattern shape as necessary.

### (Aspect of solution process)

Hereinafter, preferred aspects of the solution process will be described with reference to the drawings, but the solution process is not limited to the following aspects.

Examples of one aspect of the solution process include a method in which the organic semiconductor composition (hereinafter, also referred to as a "coating liquid") is dropped (applied) to a part of a surface of the substrate so as to be in contact with the substrate and a member (hereinafter, simply referred to as a "member") disposed on the substrate, and then the dropped coating liquid is dried. The substrate and member used in this aspect will be described later.

In this aspect, the member maintains a state of being in contact with the substrate, or is not fixed to the substrate and maintains a constant distance from the substrate.

As long as the substrate and the member maintain the above-described state, in a case where the coating liquid is dropped or dried, the relative positional relationship between the substrate and the member may be fixed or changed. From the viewpoint of production efficiency, it is preferable to move the member with respect to the substrate to change the relative positional relationship between the substrate and the member. In addition, from the viewpoint of film quality and crystal size of the obtained organic semiconductor film, it is preferable that the member is stationary with respect to the substrate to fix the relative positional relationship between the substrate and the member.

A method of dropping the coating liquid in this aspect is not particularly limited. From the viewpoint that the thickness of the film of the coating liquid on the substrate tends to be thin and the drying easily proceeds from the edge of the film of the coating liquid, in a case of dropping the coating liquid, it is preferable to drop one drop of the coating liquid, or it is preferable to drop one drop at a time in a case where two or more drops are dropped. In a case of dropping the coating liquid, the volume of one drop of the coating liquid is preferably 0.01 to 0.2 mL and more preferably 0.02 to 0.1 mL.

By dropping the coating liquid onto a part of the surface of the substrate, so as to be in contact with both the substrate and the member, the thickness of the film of the coating liquid at the edge can be reduced.

The contact angle (25°C) of the coating liquid with respect to the substrate is not particularly limited, but is preferably 0° to 90° and more preferably 10° to 20°. The contact angle of the coating liquid with respect to the substrate can be obtained by measuring an angle between the liquid droplet after 1 second has passed by dropping the coating liquid (concentration of solid content: 0.1% by mass, solvent: anisole), and the substrate. For example, the liquid volume is set to 1.0 µL or more, and the static contact angle is measured by a liquid droplet method using a Teflon (registered trademark) needle. In this way, different substrates obtained by the same treatment are measured a plurality of times (for example, 5 times), and an average value thereof is calculated used as the contact angle.

The coating liquid preferably forms a meniscus with respect to the member, and more preferably forms a concave meniscus with respect to the member in terms of film quality.

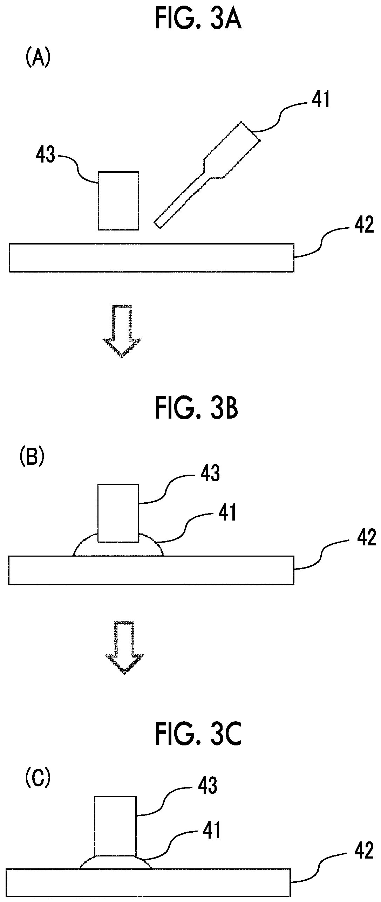

Hereinafter, a method of applying the coating liquid while maintaining a constant distance between the substrate and the member in this aspect will be described with reference to Figs. 3A to 3C. Figs. 3A to 3C are schematic views showing an example of the method of forming an organic semiconductor film of the organic TFT.

In this method, first, the substrate 42 and the member 43 are arranged at predetermined positions. For example, before dropping a coating liquid 41 onto a substrate, each of a substrate 42 and a member 43 is arranged at the position shown in Fig. 3A. In this case, the distance between the substrate 42 and the member 43 which is not in contact with the substrate 42 is kept constant. The distance between the substrate 42 and the member 43 cannot be unconditionally determined because it varies depending on the coating amount and viscosity of the coating liquid, but can be appropriately set.

Next, as shown in Fig. 3B, the coating liquid 41 is dropped onto a part (near a facing portion between the substrate 42 and the member 43) of the surface of the substrate 42, so as to be in contact with both the substrate 42 and the member 43.

Thereafter, the coating liquid 41 is dried while the relative positional relationship between the substrate 42 and the member 43 is fixed (Fig. 3C). The drying method is not particularly limited, but the above-described method for drying the organic semiconductor composition is preferable. As a result, the coating liquid 41 dries inward from both edges (ends) having a thin film thickness on the substrate 42, and the specific azaperylene compound is crystallized. As a result, the specific azaperylene compound can be arranged at a predetermined position as a crystal having a large size.

After the coating liquid 41 is dried, for example, the member 43 is pulled away from the substrate 42 by pulling up the member 43 perpendicularly to the main surface of the substrate 42. As a result, an organic semiconductor film having good film quality can be formed without leaving a trace of the member 43 on the formed crystals.

In this way, an organic semiconductor film formed of crystals of the specific azaperylene compound can be formed.

Hereinafter, a method of applying the coating liquid in a state in which the substrate and the member are in contact with each other in this aspect will be described with reference to Figs. 4A to 4D. Figs. 4A to 4D are schematic views showing another example of the method of forming an organic semiconductor film of the organic TFT.

In this method, first, the substrate 42 and the member 43 are arranged in contact with each other. For example, before dropping a coating liquid 41 onto the substrate 42, each of the substrate 42 and the member 43 is arranged at the position shown in Fig. 4A.

Next, as shown in Fig. 4B1 and Fig. 4B2, the coating liquid 41 is dropped onto a part (near a contact portion between the substrate 42 and the member 43) of the surface of the substrate 42, so as to be in contact with both the substrate 42 and the member 43. In this case, as shown in Fig. 4B2, it is preferable that the coating liquid 41 surrounds the contact portion between the substrate 42 and the member 43. Fig. 4B1 is a front view of the substrate coated with the coating liquid, and Fig. 4B2 is a plan view of the substrate coated with the coating liquid. Three-dimensional coordinates (X, Y, Z) are indicated in Fig. 4B1 and Fig. 4B2.

Thereafter, the coating liquid 41 is dried while the relative positional relationship between the substrate 42 and the member 43 is fixed, preferably as described above (Fig. 4C). The drying method is not particularly limited, but the above-described method for drying the organic semiconductor composition is preferable. As a result, the coating liquid 41 dries inward from both ends having a thin film thickness on the substrate 42, and the specific azaperylene compound is crystallized. As a result, the specific azaperylene compound can be arranged at a predetermined position as a crystal having a large size.

After the coating liquid 41 is dried, for example, the member 43 is pulled away from the substrate 42 by pulling up the member 43 perpendicularly to the main surface of the substrate 42. As a result, as shown in Fig. 4D, an organic semiconductor film 5 which is formed of crystals of the specific azaperylene compound and has good film quality can be formed without leaving a trace of the member 43 on the crystals of the specific azaperylene compound.

From the viewpoint of film quality, and viewpoint that a mechanism for holding the member 43 is unnecessary and the distance (contact state) of the member 43 to the substrate 42 can be maintained, the method of applying the coating liquid in a state in which the substrate 42 and the member 43 are in contact with each other is preferable to the method of applying the coating liquid in a state in which the distance between the substrate 42 and the member 43 is kept constant.

Hereinafter, another method of applying the coating liquid in a state in which the substrate and the member are in contact with each other in this aspect will be described with reference to Figs. 5A to 5C. Fig. 5A to 5C are schematic views showing another example of the method of forming an organic semiconductor film of the organic TFT.

This method differs from the method shown in Figs. 4A to 4D in that the crystallization of the specific azaperylene compound is promoted by moving the member 43 with respect to the substrate 42 while keeping the distance between the substrate 42 and the member 43 constant.

In this method, first, the substrate 42 and the member 43 are arranged in contact with each other. For example, before dropping a coating liquid 41 onto the substrate 42, each of the substrate 42 and the member 43 is arranged at the position shown in Fig. 5A.

Next, as shown in Fig. 5B, the coating liquid 41 is dropped onto a part (near a contact portion between the substrate 42 and the member 43) of the surface of the substrate 42, so as to be in contact with both the substrate 42 and the member 43. In this case, as shown in Fig. 4B2, it is preferable that the coating liquid 41 surrounds the contact portion between the substrate 42 and the member 43.

Thereafter, while keeping the distance between the substrate 42 and the member 43 constant, the member 43 is moved with respect to the substrate 42, and the coating liquid 41 is dried. For example, the member 43 is moved with respect to the substrate 42 in an arrow direction (X-axis negative direction) in Fig. 5C. Drying of the coating liquid 41 progresses from the edge (X-axis positive direction) opposite to the moving direction of the member 43 toward the moving direction (X-axis negative direction), and the specific azaperylene compound crystallizes. As a result, the specific azaperylene compound can be arranged at a predetermined position as a crystal having a large size.

After the coating liquid 41 is dried, for example, the member 43 is pulled away from the substrate 42 by pulling up the member 43 perpendicularly to the main surface of the substrate 42. As a result, an organic semiconductor film which is formed of the specific azaperylene compound and has good film quality can be formed without leaving a trace of the member 43 on crystals of the specific azaperylene compound.

The substrate 42 used in these aspects corresponds to the substrate of the organic TFT, and a substrate on which a gate insulating film is formed is preferable.

The member 43 used in these aspects is not particularly limited, but as a material of the member 43, an inorganic material (more preferably, glass, quartz, or silicon) or plastic (more preferably, Teflon (registered trademark), polyethylene, or polypropylene) is preferable, and glass is still more preferable.

The shape of the member 43 used in these aspects is not particularly limited as long as the member 43 has a smooth surface facing the substrate 42, but a rectangular parallelepiped is preferable.

Fig. 6 is a schematic view showing an example of the substrate 42 and the member 43 used in these aspects. In Fig. 6, the shape of the member 43 is a rectangular parallelepiped, d and w respectively represent lengths of one side and the other side of the member 43 on the surface facing the substrate 42, and h represents the height of the member 43.

The size of the member 43 used in these aspects is not particularly limited. In a case where the member 43 is a rectangular parallelepiped shown in Fig. 6, the lower limit value of the lengths (d and w in Fig. 6) of one side and the other side of the member 43 on the surface facing the substrate 42 is preferably 0.1% or more, more preferably 1% or more, still more preferably 10% or more, and particularly preferably 20% or more with respect to a length of one side of the main surface (surface on which the coating liquid is applied) of the substrate 42. In addition, the upper limit value of the above-described lengths of one side and the other side is preferably 80% or less, more preferably 70% or less, and still more preferably 50% or less with respect to the length of one side of the main surface of the substrate 42. The height (h in Fig. 6) of the member 43 is preferably 1 to 50 mm and more preferably 5 to 20 mm. Furthermore, a ratio h/d of the height h to the length d of the member 43 is preferably 0.01 to 10, and from the viewpoint of arrangement stability of the member 43, is more preferably 0.1 to 5. In addition, a ratio w/d of the length w to the length d of the member 43 is preferably 1 to 1000, and from the viewpoint of being capable of crystallizing the specific azaperylene compound in a wide range, is more preferably 5 to 100.

In this way, the crystals of the specific azaperylene compound can be precipitated to form an organic semiconductor film. Whether or not the crystals of the specific azaperylene compound are precipitated can be confirmed by observing the organic semiconductor film, using a polarizing microscope (trade name: Eclipse LV100N POL (transmission/reflection lighting type), manufactured by Nikon Corporation, eyepiece: 10x magnification, objective lens: 5 to 20x magnification).

### <Use of organic TFT>

The above-described organic TFT is not particularly limited in its use, and can be used for, for example, electronic paper, display devices, sensors, and electronic tags. Examples

Hereinafter, the present invention will be described in more detail with reference to Examples. The materials, the amounts of materials to be used, the proportions, the treatment details, and the treatment procedure shown in the examples below may be modified appropriately as long as the modifications do not depart from the spirit of the present invention. Therefore, the scope of the present invention should not be construed as being limited to the following specific examples.

### [Example 1]

According to the synthesis procedure shown in the following scheme (S1), a compound 39, which is the intermediate I, a compound 40, which is the intermediate II, and a compound 41, which is the specific azaperylene compound, were synthesized.

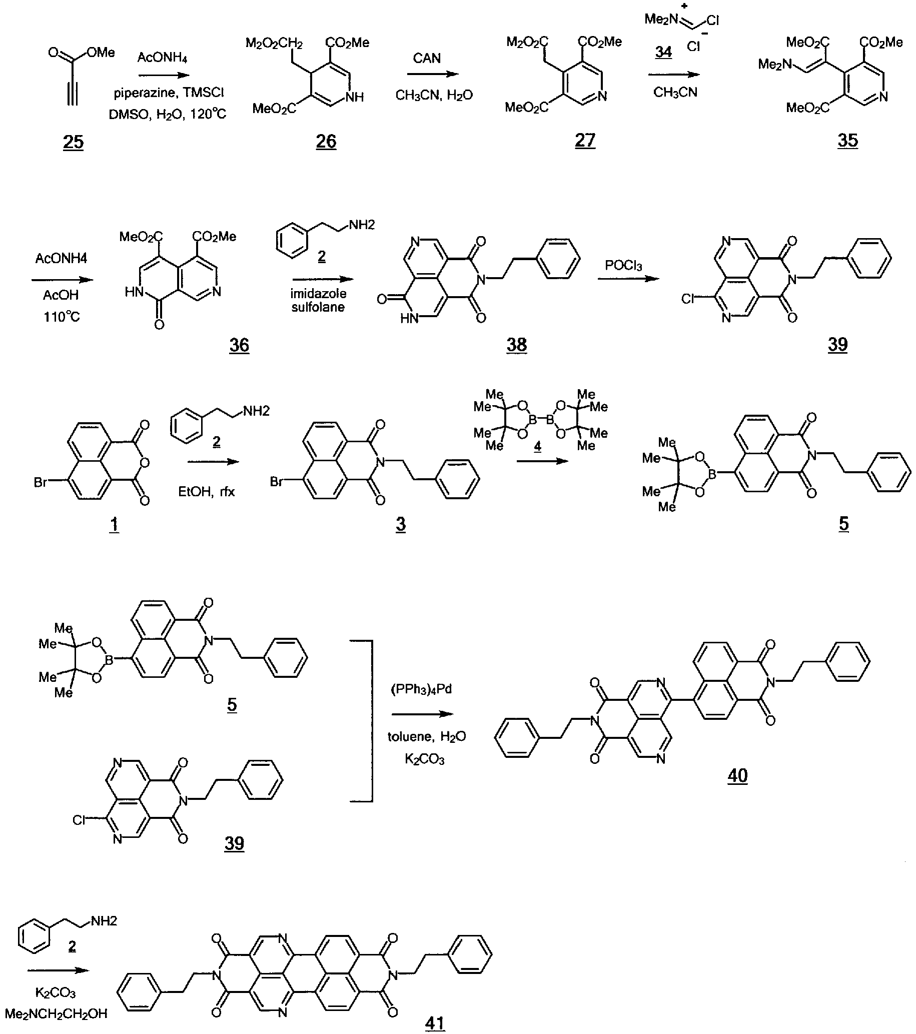

Hereinafter, each synthesis procedure will be described in detail.

### <Synthesis Example 1: synthesis of intermediate I>

The compound 39 (N-(2-Phenylethyl) 1-Chloro-[2,7]-naphthyridine-4,5-dicarboximide), which is the intermediate I, was synthesized by the following method.

First, a compound 26 (Methyl-2-{3,5-Bis(methoxycarbonyl)-1,4-dihydropyridin-4-yl}acetate) was synthesized with reference to a method described in Hu, Deqing et. al., [Tetrahedron, 2015, vol. 71, #36, pp. 6094 to 6098].

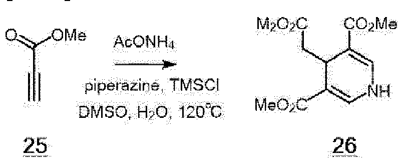

200 mL of dimethyl sulfoxide (DMSO), 200 mL of water, and 44.3 g (1.25 molar equivalents) of ammonium acetate (AcONH₄) were added to 118.5 g (1.38 mol) of methyl propiolate (compound 25), and the mixture was stirred. 13.4 g (0.33 molar equivalents) of piperazine and 19.5 mL of trimethylchlorosilane (TMSCl) were added to the obtained mixture, and the reaction was performed for 1 hour while setting the outside temperature to 90°C. Thereafter, the outside temperature was set to 125°C, and the reaction was further performed for 6 hours. After cooling the reaction product, 200 mL of water was added to the reaction product. The mixture was extracted 3 times with 200 mL of methylene chloride, and then concentrated and purified by silica gel column chromatography to obtain the compound 26 (yielding amount: 47.0 g, yield: 37.9%). The structure of the obtained compound 26 was identified by ¹H Nuclear Magnetic Resonance (NMR).
¹H NMR (DMSO): 9.16 (1H, bt, J=5.2Hz), 7.27 (2H, d, J=5.2Hz), 4.02 (1H, t, J=5.6Hz), 3.63 (6H, s), 3.48 (3H, s), 2.21 (2H, d, J=5.6Hz)
¹H NMR (CDCl₃): 7.29 (2H, d, J=5.6Hz), 6.69 (1H, bs), 4.22 (1H, t, J=5.2Hz), 3.73 (6H, s), 3.60 (3H, s), 2.51 (2H, d,J=5.2Hz)

Using the obtained compound 26, a compound 27 (Methyl 2-(3,5-Bis(methoxycarbonyl)pyridine-4-yl)acetate) was synthesized by the following method.

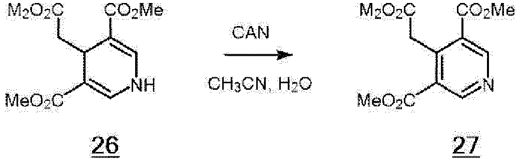

300 mL of acetonitrile and 80 mL of water were added to 47.0 g (174.6 mol) of the compound 26, and the mixture was stirred. Next, 40 g of cerium(IV) ammonium nitrate (Ceric Ammonium Nitrate; CAN) was added to this solution. The liquid temperature increased by 6°C to 7°C. Subsequently, 40 g of CAN was added thereto four times every 20 minutes. After visually confirming the dissolution of the reactant, the solvent was distilled off under reduced pressure. 200 mL of water and 400 mL of ethyl acetate were added to the obtained reaction product, and sodium hydrogen carbonate was added thereto little by little with stirring to neutralize the mixture. After separating into an organic phase and a water phase, the water phase was further extracted twice with 200 mL of ethyl acetate. The collected organic phase was washed with 10% saline and dehydrated with anhydrous magnesium sulfate. The organic phase was concentrated and solidified, hexane was added thereto, and the solid in the mixture was collected by filtration to obtain crystals of the compound 27 (yielding amount: 44.0 g, yield: 94.3%). In addition, by concentrating immediately before a timing of moment in which the oxidation by CAN was completed, crystals of the nitrate of the compound 27 were precipitated. The structure of the obtained compound 27 was identified by ¹H NMR
¹H NMR (CDCl₃) (salt free): 9.24 (2H, s), 4.53 (2H, s), 3.94 (6H, s), 3.73 (3H, s)
¹H NMR (CDCl₃) (nitrate): 13.88 (1H, bs), 9.35 (2H, s), 4.72 (2H, s), 4.01 (6H, s), 3.75 (3H, s)

Using the obtained compound 27, a compound 35 (Methyl 3-(Dimethylamino)-2-{3,5-bis(methoxycarbonyl)pyridine-4-yl}acrylate) was synthesized by the following method.

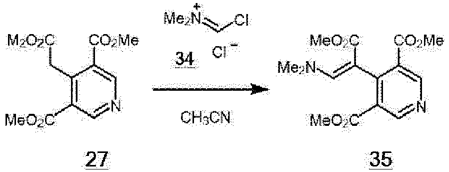

60 mL of acetonitrile was added to 10.0 g (37.4 mmol) of the compound 27, and the mixture was stirred. 5.7 g (1.2 molar equivalents) of commercially available chloromethylene dimethyliminium chloride was added thereto. The reaction solution was colored orange. Three hours after the addition, 1.43 g (0.3 molar equivalents) of chloromethylene dimethyliminium chloride was further added thereto, and four hours later, 1.43 g (0.3 molar equivalents) of chloromethylene dimethyliminium chloride was further added thereto. Thereafter, the mixture was left overnight. Ethyl acetate and sodium hydrogen carbonate aqueous solution were mixed and stirred, and the reaction solution was poured into the mixed solution. The solution was separated into an organic phase and a water phase, and the organic phase was dehydrated with anhydrous magnesium sulfate. The obtained organic phase was concentrated and crystallized using a mixed solvent of ethyl acetate and hexane to obtain crystals of the target compound 35 (yielding amount: 10.3 g, yield: 85.4%). The structure of the obtained compound 35 was identified by ¹H NMR.
¹H NMR (CDCl₃): 8.98 (2H, s), 7.59 (1H, s), 3.86 (6H, s), 3.58 (3H, s), 2.70 (6H, s)

Using the obtained compound 35, a compound 36 (Dimethyl 1,2-Dihydro-1-oxo-2H-isoquinoline-4,5-dicarboxylate) was synthesized by the following method.

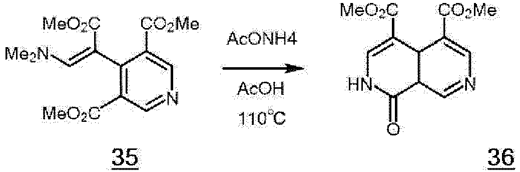

80 mL of acetic acid (AcOH) was added to 11.0 g (34.1 mmol) of the compound 35, and 11.0 g (4.6 molar equivalents) of ammonium acetate (AcONH₄) was further added thereto. The outside temperature was set to 110°C, and the reaction was performed for 4 hours. After completion of the reaction, 200 mL of water was added thereto, and then precipitated crystals were collected by filtration, washed with water, and dried to obtain the target compound 36 (yielding amount: 9.0 g, yield: 100%). The structure of the obtained compound 36 was identified by ¹H NMR.
¹H NMR (DMSO): 12.39 (1H, bs), 9.47 (1H, s), 8.97 (1H, s), 8.04 (1H, s), 3.80 (3H, s), 3.75 (3H, s)
¹H NMR (MeOH-d4): 9.54 (1H, s), 8.99 (1H, s), 8.11 (1H, s), 3.90 (3H, s), 3.86 (3H, s)

Using the obtained compound 36, a compound 38 (N-(2-Phenylethyl)-1,2-dihydro-1(2H)-oxo-[2,7]-naphthyridine-4,5-dicarboximide) was synthesized.

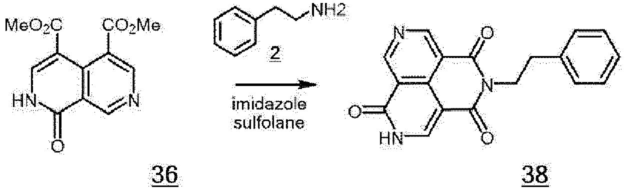

8 mL of sulfolane, 10.4 g (5 molar equivalents) of imidazole, and 4.07 g (1.1 molar equivalents) of 2-phenylethylamine were added to 8.0 g (30.5 mmol) of the compound 36, and the reaction was performed for 6 hours in a nitrogen atmosphere while setting the outside temperature to 130°C. After completion of the reaction, 100 mL of methanol was added to the reaction solution, and precipitated crystals were collected by filtration. The obtained crystals were washed with methanol and dried to obtain the target compound 38 (yielding amount: 4.4 g, yield: 45.2%). The structure of the obtained compound 38 was identified by ¹H NMR.
¹H NMR (DMSO): 12.90 (1H, bs), 9.49 (1H, s), 9.37 (1H, s), 8.47 (1H, s), 7.35-8.19 (5H, m), 4.17 (1H, d, J=16.0Hz),4.17 (1H, t, J=2.0Hz), 2.87 (1H, d, J=16.0), 2.87 (1H, t, J=2.0Hz)

Using the obtained compound 38, the compound 39, which is the intermediate I, was synthesized by the following method.

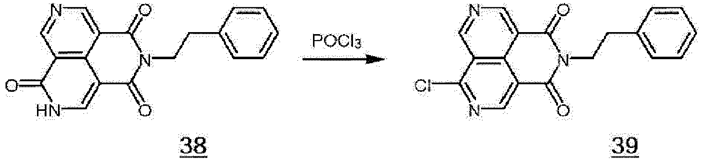

25.0 g of phosphorus oxychloride was added to 2.0 g (6.38 mmol) of the compound 38, and the reaction was performed for 5 hours while setting the outside temperature to 115°C. After completion of the reaction, the reaction solution was cooled to 80°C, and 70 mL of ethyl acetate was added thereto. A mixture of 30 mL of ethyl acetate and 100 g of ice was stirred, and the reaction solution which had been cooled to room temperature was poured into the mixture. The mixed solution was stirred to precipitate crystals, and the obtained crystals were collected by filtration, washed with water, and dried to obtain the target compound 39 (intermediate I) (yielding amount: 1.4 g, yield: 67.4%). The structure of the obtained compound 39 was identified by ¹H NMR.
¹H NMR (DMSO): 9.97 (1H, s), 9.69 (1H, s), 9.36 (1H, s), 7.35-7.20 (5H, m), 4.39 (1H, d, J=16.0Hz), 4.39 (1H, t, J=2,4Hz), 3.01 (1H, d, J=16.0Hz), 3.01 (1H, t, J=2.4Hz)

### <Synthesis Example 2: synthesis of intermediate III>

A compound 5 (N-(2-Phenylethyl)-4-(4,4,5,5-tetramethyl-1,3,2-dioxaborolan-2-yl)naphthalene-1,8-dicarboxii mide) which is the intermediate III was synthesized by the following method.

First, a compound 3 (N-(2-Phenylethyl)-4-bromonaphthalene-1,8-dicarboxiimide) was synthesized by the following method.

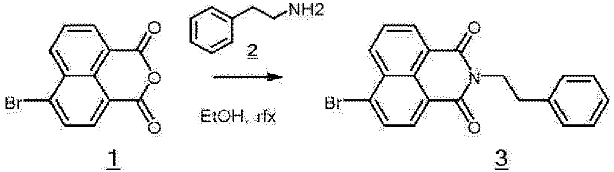

150 mL of ethanol and 10.0 g (1.52 molar equivalents) of 2-phenylethylamine (compound 2) was added to 15.0 g (54.1 mmol) of 4-bromonaphthalene-1,8-dicarboxylic acid anhydride (compound 1), and the mixture was heated under reflux. The reaction solution became uniform once, and then crystals began to precipitate. After heating under reflux for 1 hour, the mixture was cooled to room temperature and the crystals were collected by filtration. The obtained crystals were washed with ethanol and dried to obtain the compound 3 (yielding amount: 20.5 g, yield: 100%). The structure of the obtained compound 3 was identified by ¹H NMR.
¹H NMR (CDCl₃): 8.66 (1H, dd, J=1.2Hz, 7.2Hz), 8.58 (1H, dd, J=1.2Hz, 8.4Hz), 8.42 (2H, d, J=7.6Hz), 8.49 (2H, d,J=7.6Hz), 7.86 (1H, dd, J=7.2Hz, 7.6Hz), 7.38-7.20 (5H, m), 4.40 (1H, d, J=16.4Hz), 4.40 (1H, t, J=2.4Hz), 3.02 (1H,d, J=16.4Hz), 3.02 (1H, t, J=2.4Hz)

Using the obtained compound 3, the compound 5 which is the intermediate III was synthesized by the following method.

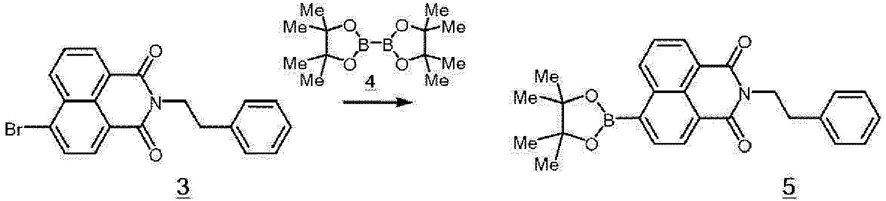

15.0 g (39.4 mmol) of the compound 3, 29.4 g (2.87 molar equivalents) of bis(pinacolato)diboron (compound 4), 11.9 g (3.0 molar equivalents) of potassium acetate, and 1.4 g (4.9 mol%) of a palladium complex catalyst (1,1'-Bis(diphenylphosphino)ferrocene)palladium(II) Dichloride Dichloromethane Complex) was mixed. Next, 300 mL of dioxane was added to the mixture, and after degassing, the reaction was performed for 2 hours in a nitrogen atmosphere while setting the outside temperature to 90°C. After completion of the reaction, the reaction solution was concentrated under reduced pressure, and then ethyl acetate and water were added to the concentrated reaction solution for extraction. Activated carbon was added to the obtained liquid, the activated carbon was filtered off, the filtrate was concentrated to the extent that crystals did not precipitate, and the concentrated liquid was left to stand. After standing overnight, precipitated crystals were filtered, washed with a mixed solution of hexane and ethyl acetate, and dried (yielding amount: 10.0 g). The crystals precipitated from the filtrate were also collected by filtration and treated in the same manner (yielding amount: 2.3 g). The obtained crystals were mixed to obtain the compound 5 (total yielding amount: 12.3 g, yield: 73.2%). The structure of the obtained compound 5 was identified by ¹H NMR.
¹H NMR (CDCl₃): 9.12 (1H, dd, J=1.2Hz, 8.4Hz), 8.61 (1H, dd, J=1.2Hz, 7.2Hz), 8.57 (1H, d, J=7.2Hz), 8.30 (1H, d,J=7.6Hz), 7.79 (1H, dd, J=7.2Hz, 8.4Hz), 7.40-7.20 (5H, m), 4.40 (1H, d, J=16.0), 4.40 (1H, t, J=2.8Hz), 3.04 (1H, d,J=16.0Hz), 3.04 (1H, t, J=2.8Hz), 1.456 (12H, s)

### <Synthesis Example 3: synthesis of intermediate II>

Using the compound 39 (intermediate I) obtained in Synthesis Example 1 and the compound 5 (intermediate III) obtained in Synthesis Example 2, the compound 40 (6-(1,3-dioxo-2-phenethyl-2,3,3a1,6a-tetrahydro-1H-benzo[de]isoquinolin-6-yl)-2-phenethyl-3 a1,6a-dihydro-1H-pyrido[3,4,5-de][2,7]naphthyridine-1,3(2H)-dione) which is the intermediate II was synthesized by the following method.

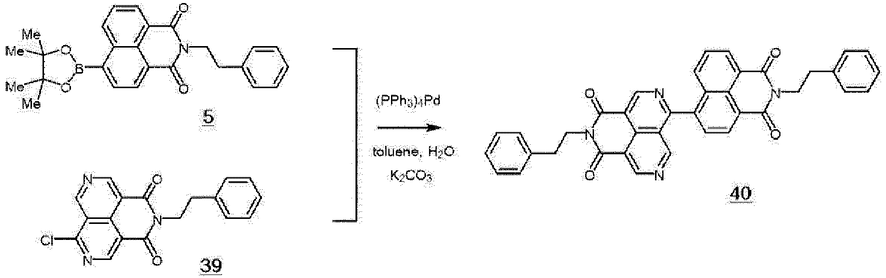

1.0 g (3 mmol) of the compound 39 (intermediate I) and 1.35 g (1.1 molar equivalents) of the compound 5 (intermediate III) were mixed. To the mixture, 30 mL of toluene, 15 mL of water, 350 mg (10 mol%) of tetrakis(triphenylphosphine)palladium(0) ((PPh₃)₄Pd), and 1.3 g of potassium carbonate was added, and the mixture was heated under reflux for 5 hours with vigorous stirring in a nitrogen atmosphere. After cooling the reaction solution to room temperature, an organic phase obtained by extracting the reaction solution with methylene chloride three times was concentrated and purified by silica gel column chromatography to obtain the target compound 40 (intermediate II) (yielding amount: 870 mg, yield: 47.9%). The structure of the obtained compound 40 was identified by ¹H NMR.
¹H NMR (CDCl₃): 9.81 (1H, s), 9.69 (1H, s), 9.41 (1H, s), 8.81 (1H, d, J=7.6Hz), 8.71 (1H, dd, J=1.2Hz, 7.6Hz), 8.00(1H, dd, J=0.8Hz, 8.4Hz), 7.96 (1H, d, 7.2Hz), 7.76 (1H, dd, J=7.2Hz, 8.4Hz), 7.42-7.23 (10H, m), 4.47 (4H, m), 3.09(2H, t, J=8.0Hz), 3.07 (2H, t, J=8.0Hz)

### <Synthesis Example 4: synthesis of specific azaperylene compound>

Using the obtained compound 40 (intermediate II), a compound 41 (2,9-Diphenethyl-3a1,5b1,5a1,7a1-tetrahydropyrido[3,4,5-de]pyrido[3',4',5':6,7]phenaleno[1,2 ,3-ij][2,7]naphthyridine-1,3,8,10(2H,9H)-tetraone), which is the specific azaperylene compound, was synthesized by the following method.

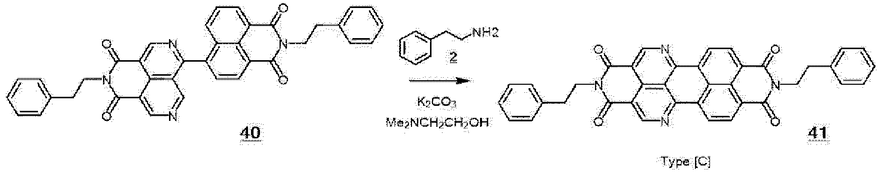

15 mL of N,N-dimethylethanolamine, 2.0 g of potassium carbonate, and 6.0 mL (47.7 mmol) of 2-phenylethylamine were added to 870 mg (1.11 mmol) of the compound 40, and the reaction was performed for 5 hours while setting the outside temperature to 95°C. After cooling the reaction solution, water was added to the reaction solution, and extraction was performed with chloroform/hexafluoro-2-propanol (hexafluoro isopropanol; HFIP). The organic phase was concentrated and purified by silica gel column chromatography (eluate: chloroform/HFIP = 1/19). High-purity fractions were collected to obtain the compound 41 (yielding amount: 115 mg, yield: 13%), which was a specific azaperylene compound that was pure at the NMR spectrum level. The structure of the obtained compound 41 was identified by ¹H NMR.
¹H NMR (CDCl₃/HOC(CF₃)₃=3/1): 9.68 (2H, s), 9.47 (2H, d, J=8.0Hz), 8.84 (2H, d, J=8.0Hz), 7.38-7.25 (10H, m),4.43 (2H, t, J=6.0Hz), 4.41 (2H, t, J=6.0Hz), 3.05 (2H, t, J=6.0Hz), 3.03 (2H, t, J=6.0)

### [Example 2]

According to the synthesis procedure shown in the following scheme (S2), a compound 24, which is the intermediate I, and a compound 42, which is the intermediate II, were synthesized.

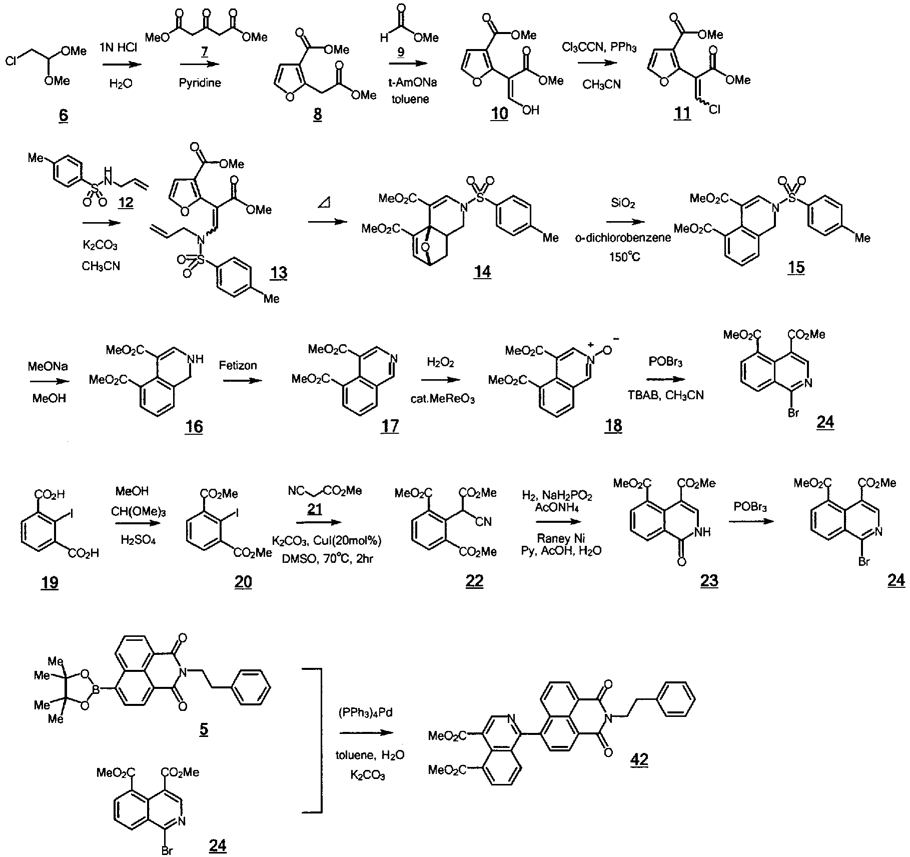

Hereinafter, each synthesis procedure will be described in detail.

### <Synthesis Example 5: synthesis of intermediate I>

The compound 24 (Dimethyl 1-Bromoisoquinoline-4,5-dicarboxylate), which is Intermediate I, was synthesized by the following method.

First, a compound 8 (Methyl 3-(Methoxycarbonylfuran-2-yl)acetate) was synthesized by the following method.

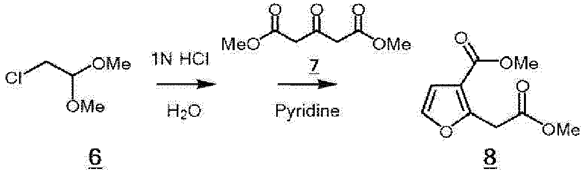

117 mL of water was added to 147 g (1.18 mol) of 2,2-dimethylchloroacetal (compound 6), and the mixture was stirred. 16.9 mL of 1 M dilute hydrochloric acid was added to the obtained solution, and the reaction was performed at 90°C for 2 hours. After cooling the reaction solution with ice water, 16.9 mL of 1 M sodium hydroxide aqueous solution was added thereto to neutralize the mixture. The obtained solution was added to 174 g (1.0 mol) of dimethyl acetonedicarboxylic acid, 370 mL of pyridine was added to the mixed solution, and the reaction was performed for 12 hours while setting the outside temperature to 55°C. This reaction solution was concentrated, ethyl acetate was added thereto, and extraction was performed twice. The obtained organic phase was washed with saturated saline, and dehydrated and concentrated with anhydrous magnesium sulfate. The residue was purified by silica gel column chromatography to obtain the compound 8 as a colorless liquid (yielding amount: 117 g, yield: 59.0%). The structure of the obtained compound 8 was identified by ¹H NMR.
¹H NMR (CDCl₃): 7.34 (1H, d, J=2Hz), 6.70 (1H, d, J=2Hz), 4.09 (2H, s,), 3.83 (3H, s), 3.73 (3H, s)

Using the obtained compound 8, a compound 11 (Methyl 3-Chloro-2-(3-methoxycarbonylfuran-2-yl)acrylate) was synthesized by the following method.

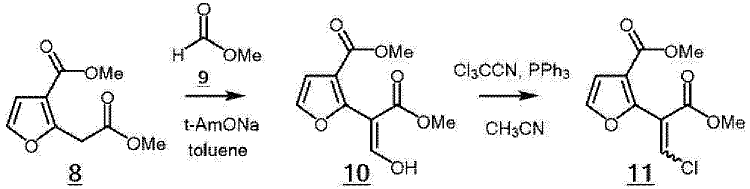

50.0 g (454 mmol) of sodium tert-pentoxide was dissolved in 300 mL of toluene, and the mixture was stirred. A mixed solution of 30.0 g (151.4 mmol) of Methyl 3-(Methoxycarbonylfuran-2-yl)acetate (compound 8) and 29.0 g (483 mmol) of methyl formate was added dropwise thereto. Due to heat production, the temperature of the reaction solution increased from 22°C to 36°C. Since residual raw material was confirmed 2.5 hours after the start of the reaction, 10 g of sodium tert-pentoxide and 10 g of methyl formate were added to the reaction solution, and the reaction was further performed for 1.5 hours. The above-described reaction solution was poured into a mixed solution obtained by stirring 100 mL of concentrated hydrochloric acid, 200 mL of water, and 300 mL of ethyl acetate. After the liquid separation, the water phase was extracted twice more with 200 mL of ethyl acetate. The obtained organic phase was washed with saturated saline, dehydrated with anhydrous magnesium sulfate, and concentrated to obtain a concentrate (weight: approximately 40 g) containing a hydroxymethylene compound (compound 10) as a main component.

250 mL of methylene chloride was added to this concentrate, and 41.7 g of triphenylphosphine was further added thereto and dissolved by stirring. 18.3 mL of trichloroacetonitrile was added dropwise to this reaction solution, and the mixture was left to stand for 2 days. After removing the precipitated solid by filtration through Celite, the filtrate was concentrated and subjected to silica gel column chromatography as it was to obtain a chloromethylene compound (compound 11) as a mixture of E/Z isomers (yielding amount: 17.4 g, yield: 46.9% (2 steps)). The structure of the obtained compound 11 was identified by ¹H NMR.
¹H NMR of one isomer (CDCl3): 7.69 (1H, s), 7.50 (1H, d, J=2Hz), 6.82 (1H, d, J=2Hz), 3.80 (3H, s), 3.78 (3H, s)
¹H NMR of the other isomer (CDCl₃): 7.38 (1H, d, J=2.0Hz), 7.23 (1H, s), 6.78 (1H, d, J=2.0Hz), 3.86 (3H, s), 3.81 (3H,s)

Using the obtained compound 11, a compound 14 (Dimethyl 2-Tosyl-1,7,8,8a-tetrahydro-2H-4a,7-epoxyisoquinoline-4,5-dicarboxylate) was synthesized by the following method.

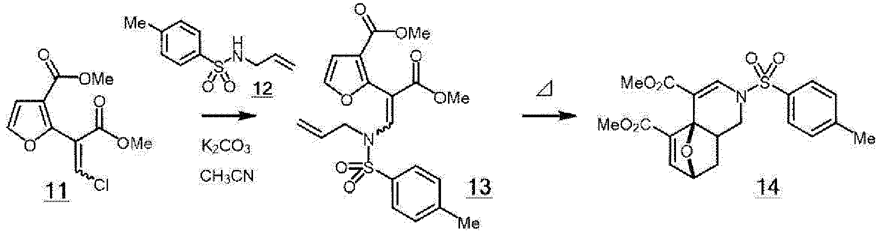

100 mL of acetonitrile is added to 32.0 g (130.8 mmol) of the compound 11 and 32.0 g of potassium carbonate, and 28.0 g (1.01 equivalents) of N-allyl-p-toluenesulfonamide (compound 12) was added thereto, and the reaction was performed while setting the outside temperature to 60°C. At the beginning of the reaction, the reaction solution was colored dark green. It was found from analysis by thin-layer silica gel chromatography that the reaction solution at the beginning of the reaction mainly included the compound 13 and the compound 14. After performing the reaction for 4 hours, the outside temperature was set to 95°C, and the reaction was further performed for 6 hours. It was confirmed from analysis by thin-layer silica gel chromatography that, while the compound 13 disappeared in the reaction solution, the compound 14 was included in the reaction solution as a main product. Subsequently, the reaction was terminated. After concentrating the reaction solution, ethyl acetate and water were added for extraction. The organic phase was washed successively with dilute hydrochloric acid and sodium hydrogen carbonate aqueous solution, dehydrated with anhydrous magnesium sulfate, and concentrated to obtain a crude compound including the compound 14 (crude yielding amount: 53 g, crude yield: 97%). The structures of the intermediate compound 13 and the target compound 14 were identified by ¹H NMR.
¹H NMR of Z isomer of compound 13 (CDCl₃): 7.78 (2H, d, 8.0Hz), 7.55 (1H, s), 7.45 (2H, d, J=8.0Hz), 7.32 (1H, d,J=2.0Hz), 6.75 (1H, d, J=2.0Hz), 5.45 (1H, m), 5.12-5.04 (2H, m), 4.44 (2H, m), 3,77 (3H, s), 3.63 (3H, s), 2.45 (3H, s)
¹H NMR of compound 14 (CDCl₃): 8.36 (1H, s), 7.72 (2H, d, J=8.0Hz), 7.34 (1H, d, J=8.0Hz), 7.04 (1H, d, J=2.0Hz), 4.93 (1H, dd,J=2.0Hz, 4.8Hz), 4.11 (1H, dd, J=3.2Hz, 12.0Hz), 3.72 (3H, s), 3.68 (3H, s), 2.59 (1H, t, J=12.0Hz), 2.44 (3H, s), 1.82(1H, m), 1.66 (1H, dd, J=7.6Hz, 12.0Hz), 1.47 (1H, ddd, J=3.2Hz, 4.8Hz, 12.0Hz)

Using the obtained compound 14, a compound 15 (Dimethyl 2-(p-Toluenesulfonyl)-1,2-dihydroisoquinoline-4,5-dicarboxylate) was synthesized by the following method.

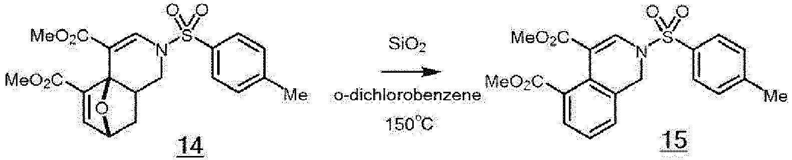

150 g of silica gel ("Wako C200" manufactured by FUJIFILM Wako Pure Chemical Corporation) and 200 mL of o-dichlorobenzene were added to approximately 53 g of the crude compound including the compound 14, and the mixture was stirred. The outside temperature was set to 150°C, and the reaction was performed for 1 hour. The silica gel was filtered from the reaction solution, and the filtrate was washed with ethyl acetate, and then concentrated under reduced pressure. The concentrated residue was purified by silica gel column chromatography, dissolved by heating in ethyl acetate, and then crystallized by adding hexane to the solution. The precipitated crystals were collected by filtration and dried to obtain the compound 15 (yielding amount: 39.5 g, yield: 77.8%). The structure of the obtained compound 15 was identified by ¹H NMR.
¹H NMR (CDCl₃): 7.88 (1H, s), 7.77 (2H, d, J=8.4Hz), 7.64 (1H, dd, J=1.6Hz, 7.6Hz), 7.36 (1H, d, J=8.4Hz), 7.25 (1H,t, J=7.6Hz), 7.19 (1H, dd, J=1.6Hz, 7.6Hz), 4.44 (2H,s), 3.80 (3H, s), 3.77 (3H, s)

Using the obtained compound 15, a compound 17 (Dimethyl Isoquinoline-4,5-dicarboxylate) was synthesized by the following method.

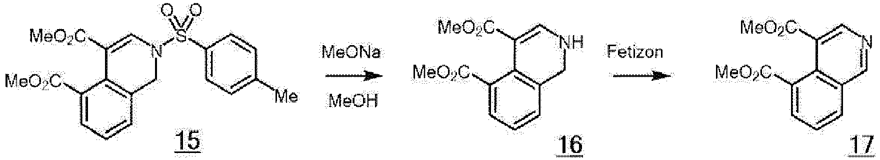

60 mL of methanol was added to 29.0 g (72.2 mmol) of the compound 15, and the mixture was stirred. 85 mL of a 28% methanol solution of sodium methoxide was added thereto. The mixture was heated under reflux for 3 hours and cooled to room temperature. 100 g of silica gel was added thereto, and the mixture was concentrated to adsorb the product, and then purified by silica gel column chromatography to obtain the compound 16 (Dimethyl1,2-Dihydroisoquinoline-4,5-dicarboxylate) (yielding amount: 14 g (56.6 mmol)). 150 mL of acetone and 22 g of silver carbonate were added to 14 g of the obtained compound 16, and the reaction was performed at an outside temperature setting of 55°C for 5 hours. The inorganic substance was filtered off by Celite filtration, and the filtrate was concentrated, and then purified by silica gel column chromatography to obtain the compound 17 (yielding amount: 10.8 g, yield: 77.8%). The structure of the obtained compound 17 was identified by ¹H NMR.
¹H NMR (CDCl₃): 9.38 (1H, s), 8.96 (1H, s), 8.22 (1H, dd, J=1.2Hz, 6.8Hz), 8.17 (1H, dd, J=1.2Hz, 8.4Hz), 7.72 (1H,dd, J=6.8Hz, 8.4Hz), 3.96 (3H, s), 3.94 (3H, s)

Using the obtained compound 17, a compound 18 (Dimethyl Isoquinoline-4,5-dicarboxylate N-Oxide) was synthesized by the following method.

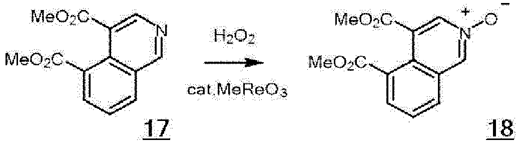

7.0 g (28.5 mmol) of the compound 17 was dissolved in 70 mL of methylene chloride, and the mixture was stirred. 400 mg of methyltrioxorenium (MeReO₃) was added to this solution. Next, 14 mL of 30% hydrogen peroxide was added thereto, and the mixture was stirred at room temperature for 8 hours. The organic phase was separated and subjected to silica gel column chromatography to obtain the compound 18 (yielding amount: 5.3 g, yield: 71.1%). The structure of the obtained compound 18 was identified by ¹H NMR.
¹H NMR (CDCl₃): 8.82 (1H, d, J=2.0Hz), 8.52 (1H, d, J=2.0Hz), 8.05 (1H, dd, J=1.2Hz, 7.2Hz), 7.87 (1H, dd, J=0.8Hz, 8.4Hz), 7.69 (1H, dd, J=7.2Hz, 8.4Hz)

Using the obtained compound 18, the compound 24 (Dimethyl 1-Bromoisoquinoline-4,5-dicarboxylate) was synthesized by the following method.

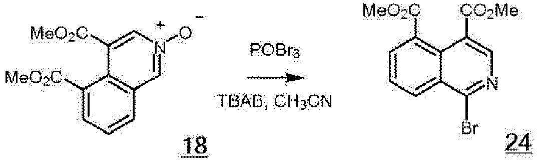

After dissolving 250 mg (0.96 mmol) of the compound 18 in 5 mL of methylene chloride, 322 mg of tetrabutylammonium bromide was added to this solution, and the mixture was stirred. 500 mg of phosphorus oxybromide was added to this reaction solution, and the reaction was performed at room temperature for 4 hours. After adding ice to the reaction solution to inactivate the phosphorus oxybromide, the organic phase was purified by a short silica gel column to obtain the target compound 24 (yielding amount: 210 mg, yield: 67.7%). The structure of the obtained compound 18 was identified by ¹H NMR.
¹H NMR (CDCl₃): 8.67 (1H, s), 8.58 (1H, dd, J=1.2Hz, 8.4Hz), 8.25 (1H, dd, J=1.2Hz, 7.2Hz), 7.78 (1H, dd, J=7.2Hz,8.4Hz), 7.72 (1H, dd, J=6.8Hz, 8.4Hz), 3.95 (3H, s), 3.93 (3H, s)

### <Synthesis Example 6: synthesis of intermediate I>

The compound 24, which is the intermediate I, was synthesized by the following method different from that of Synthesis Example 5.

First, a compound 20 (Dimethyl 2-Iodoisophthalate) was synthesized by the following method.

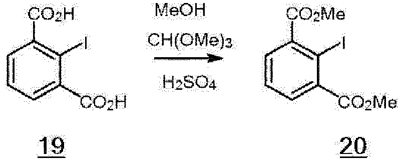

70 mL of methanol was added to 5.3 g (18.1 mmol) of 2-iodoisophthalic acid (compound 19), and the mixture was stirred. 2.6 mL of concentrated sulfuric acid was added dropwise thereto, 1.9 g of methyl orthoformate was added thereto, and the mixture was heated under reflux for 10 hours. 1 g of methyl orthoformate was added to the reaction solution, and the reaction was further performed for 3 hours. After cooling the reaction solution, the reaction solution was poured into a sodium bicarbonate water. After confirming that the mixed solution was weakly basic, the mixed solution was extracted with ethyl acetate. The obtained organic phase was dehydrated with anhydrous magnesium sulfate and concentrated to obtain the compound 20 (yielding amount: 5.66 g, yield: 97.7%). The structure of the obtained compound 20 was identified by ¹H NMR.
¹H NMR (CDCl₃): 7.62 (2H, d, J=7.6Hz), 7.45 (1H, dd, J=7.6Hz, 11.6Hz), 3.96 (6H, s)

Using the obtained compound 20, a compound 22 (Methyl 2-(2,6-Bis(methoxycarbonyl)phenyl)-2-cyanoacetate) was synthesized by the following method.

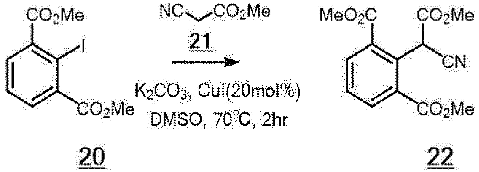

11.4 g (35.6 mmol) of dimethyl 2-iodoisophthalate (compound 20), 1.36 g (20 mol%) of cuprous iodide, 15.0 g (3.0 molar equivalents) of potassium carbonate, and 3.9 g (1.1 molar equivalents) of methyl cyanoacetate (compound 21) were mixed, and 57 mL of DMSO was added thereto and stirred. The outside temperature was set to 80°C, the reaction was performed for 3 hours, and the reaction solution was cooled to room temperature. After adding ethyl acetate and water to the reaction solution, dilute hydrochloric acid was added thereto to acidify the reaction solution. This mixed solution was filtered through Celite, and the filtrate was extracted twice with ethyl acetate, and then dehydrated with magnesium sulfate. The obtained organic phase was concentrated, and the residue was purified by silica gel column chromatography to obtain the compound 22 (yielding amount: 8.66 g, yield: 83.5%). The structure of the obtained compound 22 was identified by ¹H NMR.
¹H NMR (CDCl₃): 8.25 (2H, d, J=7.6Hz), 7.59 (1H, t, J=7.6Hz), 6.97 (1H, s), 3.96 (6H, s), 3.86 (3H, s)

Using the obtained compound 22, a compound 23 (Dimethyl 1,2-Dihydro-2-oxo-2H-isoquinoline-4,5-dicarboxylate) was synthesized by the following method.

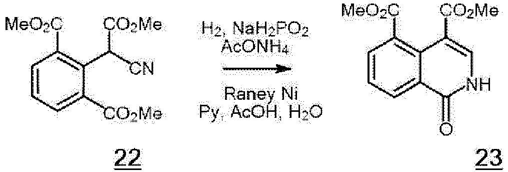

4 mL of pyridine, 2 mL of acetic acid, and 2 mL of water were added to a mixture of 280 mg (0.96 mmol) of the compound 22, 820 mg (8 molar equivalents) of monosodium hypophosphate, and 600 mg (8.1 molar equivalents) of ammonium acetate, and hydrogenation was performed in the presence of Raney nickel. Here, the hydrogen pressure was set to 0.8 MPa, and the outside temperature was set to 80°C. After reacting for 5 hours, the reaction solution was cooled to room temperature, and the catalyst was removed by filtration through Celite. After extraction by adding ethyl acetate and water to the filtrate, the organic phase was washed with dilute hydrochloric acid and sodium bicarbonate aqueous solution in this order, and then concentrated to precipitate crystals. The crystals were washed with a mixed solution of ethyl acetate and hexane, and dried to obtain the compound 23 (yielding amount: 150 mg, yield: 59.8%). The structure of the obtained compound 23 was identified by ¹H NMR.
¹H NMR (DMSO): 11.98 (1H, bs), 8.42 (1H, dd, J=1.6Hz, 8.0Hz), 8.04 (1H, dd, J=1.6Hz, 8.0Hz), 7.78 (1H, bs), 7.65(1H, t, J=8.0Hz) 3.76 (3H, s), 3.73 (3H, s)

Using the obtained compound 23, the compound 24 (Dimethyl 1-Bromoisoquinoline-4,5-dicarboxylate) was synthesized by the following method.

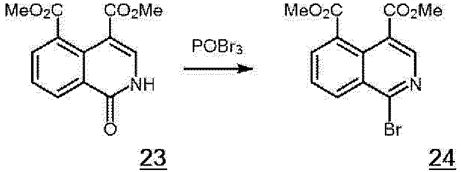

3.7 mL of phosphorus oxybromide was added to 730 mg (2.79 mmol) of the compound 23, and the mixture was heated and stirred at 110°C for 10 minutes. The reaction solution was cooled to room temperature, and the reaction solution was added dropwise to a 1 M sodium hydroxide aqueous solution. Chloroform was added to this mixed solution for extraction, and the organic phase was dehydrated with magnesium sulfate. The organic phase was concentrated, and the residue was purified by silica gel column chromatography to obtain the target compound 24 (yielding amount: 550 mg, yield: 70%). The structure of the obtained compound 24 was identified by ¹H NMR.
¹H NMR (CDCl₃): 8.67 (1H, s), 8.58 (1H, dd, J=1.2Hz, 8.4Hz), 8.25 (1H, dd, J=1.2Hz, 7.2Hz), 7.78 (1H, dd, J=7.2Hz, 8.4Hz), 7.72 (1H, dd, J=6.8Hz, 8.4Hz), 3.95 (3H, s), 3.93 (3H, s)

### <Synthesis Example 7: synthesis of intermediate II>

Using the compound 24 (intermediate I) obtained in Synthesis Example 5 or 6 and the compound 5 (intermediate III) obtained in Synthesis Example 2, the compound 42 (Dimethyl 1-(1,3-dioxo-2-phenethyl-2,3,3a1,6a-tetrahydro-1H-benzo[de]isoquinolin-6-yl)-4a,8adihydrois oquinoline-4,5-dicarboxylate), which is the intermediate II, was synthesized by the following method.

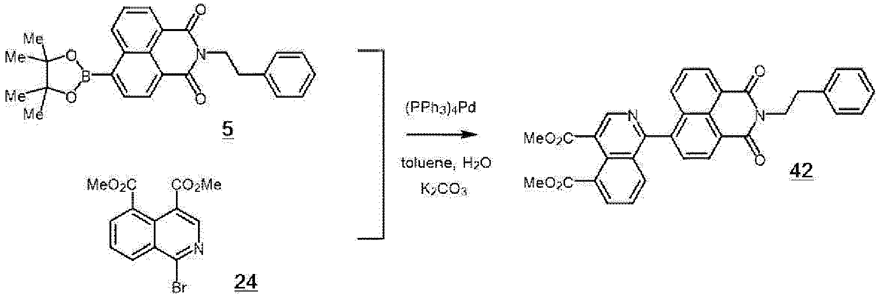

137 mg (0.423 mmol) of the compound 24 (intermediate I) and 184 mg (1.02 molar equivalents) of the compound 5 (intermediate III) were mixed. 5 mL of toluene and 5 mL of water were added to the mixture, 50 mg (10 mol%) of tetrakis(triphenylphosphine)palladium(0) and 300 mg of potassium carbonate was further added thereto, and the mixture was heated under reflux for 15 hours with vigorous stirring in a nitrogen atmosphere. The reaction solution was cooled to room temperature, and then extracted with ethyl acetate. The obtained organic phase was dehydrated with anhydrous magnesium sulfate, and then concentrated to precipitate crystals. The precipitated crystals were added to a mixed solution of hexane and ethyl acetate, and the mixture was collected by filtration under reduced pressure to obtain the target compound 42 (intermediate II) (yielding amount: 160 mg, yield: 69.5%). The structure of the obtained compound 42 was identified by ¹H NMR.
¹H NMR (CDCl₃): 9.14 (1H, s), 8.76 (1H, d, J=7.6Hz), 8.65 (1H, dd, J=1.2Hz, 7.2Hz), 8.24 (1H, 1.2Hz, 7.2Hz), 7.84(1H, d, J=7.6Hz), 7.74 (1H, m), 7.65 (1H, t, J=7.2Hz), 7.55 (1H, t, J=7.2Hz), 7.40-7.23 (5H, m), 4.47 (1H, d, 16.0Hz),4.47 (1H, t, J=2.4Hz), 4.04 (3H, s), 4.00 (3H, s), 3.09 (1H, d, 16.0Hz), 3.09 (1H, t, J=2.4Hz)

### [Example 3]

According to the synthesis procedure shown in the following scheme (S3), a compound 32 and 33, which are the intermediate I, a compound 45, which is the intermediate II, and a compound 46, which is the specific azaperylene compound, were synthesized.

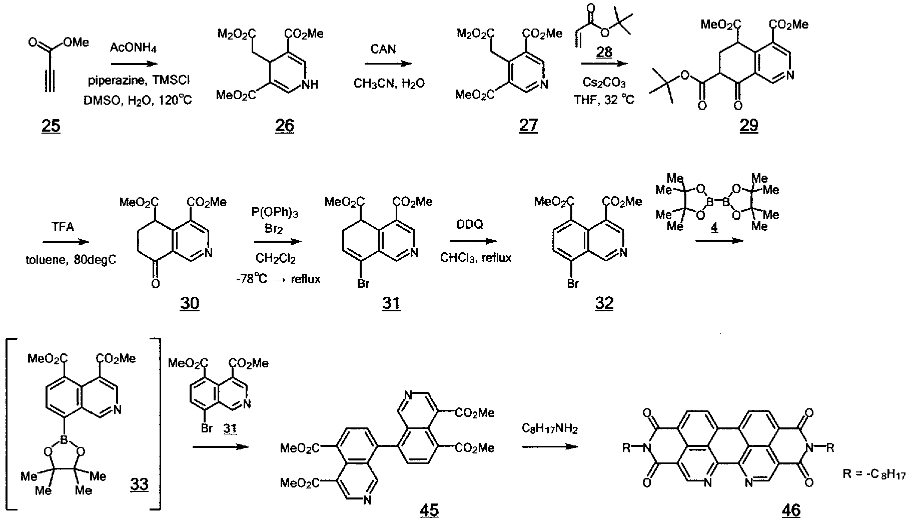

Hereinafter, each synthesis procedure will be described in detail.

### <Synthesis Example 8: synthesis of intermediate I>

The compound 32 (Dimethyl 8-Bromo-5,6-dihydroisoquinoline-4,5-dicarboxylate), which is Intermediate I, was synthesized by the following method.

First, a compound 27 was synthesized according to the method described in Synthesis Example 1 of Example 1.

Using the obtained compound 27, a compound 29 (tert-Butyl 4,5-Bis(methoxycarbonyl)-5,6,7,8-tetrahydro-8-oxo-7H-isoquinoline-7-carboxylate) was synthesized by the following method.

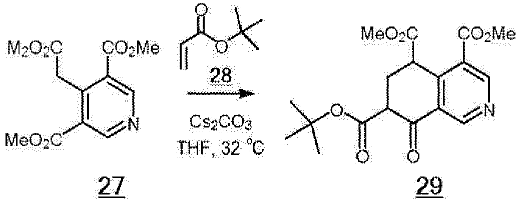

10.0 g (37.4 mmol) of the compound 27 was dissolved in 100 mL of THF, and the mixture was stirred. 19.2 g (4 molar equivalents) of tert-butyl acrylate (compound 28) and 24.4 g (2 molar equivalents) of cesium carbonate were added thereto, and the mixture was stirred for 6 hours at an outside temperature of 35°C. The precipitated solid was collected by filtration, a mixed solution of dilute hydrochloric acid and ethyl acetate was added to the collected solid, and the solid was dissolved and then separated. The resulting organic phase was washed with water and then with saturated saline. The organic phase was dehydrated with anhydrous magnesium sulfate and then concentrated to obtain the compound 29 (yielding amount: 5.5 g, yield: 40.0%). The structure of the obtained compound 29 was identified by ¹H NMR.
¹H NMR (CDCl₃): 12.48 (1H, bs), 9.18 (1H, s), 9.15 (1H, s), 4.88 (1H, dd, J=2.0Hz, 7.6Hz), 3.95 (3H, s), 3.64 (3H, s), 3.28 (1H, dd, J=2.0Hz, 16.8Hz), 2.60 (1H, dd, J=7.6Hz, 16.8Hz), 1.60 (9H, s)

Using the obtained compound 29, a compound 30 (Dimethyl 8-Oxo-5,6,7,8-tetrahydroidoquinoline-4,5-dicarboxylate) was synthesized.

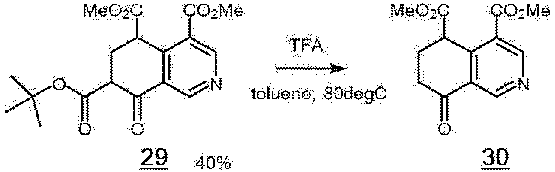

92 mL of toluene and 11.5 mL of trifluoroacetic acid were added to 7.8 g (21.5 mmol) of the compound 29, and the mixture was stirred. The outside temperature was set to 70°C, and the reaction was performed for 9 hours. The obtained reaction solution was concentrated under reduced pressure, and the residue was dissolved in ethyl acetate and passed through an alumina short column. After concentration again, purification was performed by silica gel column chromatography to obtain the compound 30 (yielding amount: 5.0 g, yield: 88.5%). The structure of the obtained compound 30 was identified by ¹H NMR.
¹H NMR (CDCl₃): 9.38 (1H, s), 9.31 (1H, s), 4.83 (1H, m), 3.96 (3H, s), 3.75 (3H, s), 2.76-2.58 (3H, m), 2.37 (1H, m)

Using the obtained compound, a compound 31 (Dimethyl 8-Bromo-5,6-dihydroisoquinoline-4,5-dicarboxylate) was synthesized by the following method.

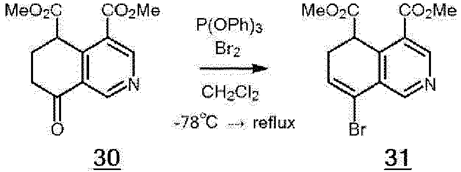

10.3 g (33.1 mmol) of triphenyl phosphate was dissolved in 50 mL of methylene chloride, and cooled to ―78°C under a nitrogen atmosphere. While keeping this solution below ―60°C, 1.9 mL of bromine was added dropwise to the solution. 5.1 g (19.4 mmol) of the compound 30 and 3.3 mL of diisopropylethylamine were added to this solution, and the mixture was left overnight. Next, the reaction solution was heated under reflux for 3 hours, and the solvent was distilled off under reduced pressure. Ethyl acetate and water were added to the residue, and the solution was neutralized with sodium hydrogen carbonate while stirring. The organic phase obtained by the liquid separation was washed with sodium hydrogen carbonate aqueous solution, dehydrated with anhydrous magnesium sulfate, and then concentrated under reduced pressure. The obtained concentrated liquid was purified by silica gel column chromatography to obtain the compound 31 (yielding amount: 4.5 g, yield: 71%). The structure of the obtained compound 31 was identified by ¹H NMR.
¹H NMR (CDCl₃): 9.11 (1H, bs), 6.59 (1H, dd, J=2.7Hz, 7.2Hz), 5.02 (1H, d, J=6.9), 3.96 (3H, s), 3.65 (3H, s), 3.09(1H, ddd, J=1.5Hz, 6.9Hz, 18.0Hz), 2.63 (1H, ddd, J=2.7Hz, 7.8Hz, 18.0Hz)

Using the obtained compound, the compound 32 (Dimethyl 8-Bromoisoquinoline-4,5-dicarboxylate) was synthesized by the following method.

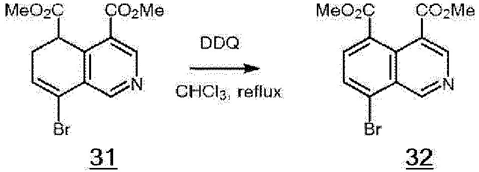

After adding 50 mL of chloroform to 4.5 g (13.8 mmol) of the compound 31, 4.2 g of 2,3-dichloro-5,6-dicyano-1,4-benzoquinone (DDQ) was further added thereto, and the mixture was heated under reflux for 3 hours. The precipitated solid was separated by filtration, the filtrate was concentrated, and the obtained concentrated liquid was purified by silica gel column chromatography to obtain the target compound 32 (intermediate I) (yielding amount: 3.9 g, yield: 87.2%). The structure of the obtained compound 32 was identified by ¹H NMR.
¹H NMR (CDCl₃): 9.34 (1H, bs), 9.03 (1H, bs), 8.07 (1H, d, J=7.8Hz), 8.00 (1H, d, J=7.8Hz), 3.97 (3H, s), 3.94 (3H,s)

### <Synthesis Example 9: synthesis of intermediate II>

Using the obtained compound 32 (intermediate I) obtained in Synthesis Example 8, the compound 45 (tetramethyl [8,8'-biisoquinoline]-4,4',5,5'-tetracarboxylate), which is the intermediate II, was synthesized by the following method.

First, the compound 45 (tetramethyl [8,8'-biisoquinoline]-4,4',5,5'-tetracarboxylate) was synthesized by the following method.

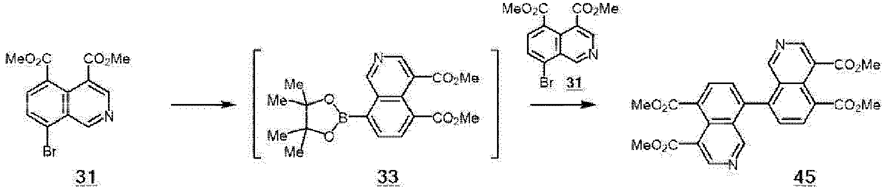

190 mg (0.59 mmol) of the compound 31, 160 mg (1.17 mmol) of potassium carbonate, 82 mg (0.32 mmol) of bis(pinacolato)diboron, and molecular sieve 5A were dispersed in 2.0 mL of n-propyl acetate under a nitrogen atmosphere. The reaction solution was heated at 100°C for 5 minutes, 21 mg (5 mol%) of bis(di-tert-butyl(4-dimethylaminophenyl)phosphine)dichloropalladium(II) was added thereto, and the mixture was heated and stirred for 3.5 hours. It was found from analysis by thin-layer silica gel chromatography that, in the system, the produced compound 33 immediately reacted with the raw material compound 31 to produce the compound 45. After cooling the reaction solution to room temperature, the reaction solution was and purified by silica gel column chromatography to obtain the target compound 45 (intermediate II) (yielding amount: 146 mg, yield: 81%). The structure of the obtained compound 45 was identified by ¹H NMR.
¹H NMR (CDCl₃): 9.00 (2H, bs), 9.88 (2H, bs), 8.34 (2H, d, J=7.4Hz), 7.69 (2H, d, J=7.4Hz), 4.01 (6H, s), 4.00 (6H, s)

### <Synthesis Example 10: synthesis of specific azaperylene compound>

Using the obtained compound 45 (intermediate II) obtained in Synthesis Example 9, a compound 46 (2,9-dioctyldiisoquinolino[4,5,6-cde:6',5',4'-ghi][1,10]phenanthroline-1,3,8,10(2H,9H)-tetraon e), which is the specific azaperylene compound, was synthesized by the following method.

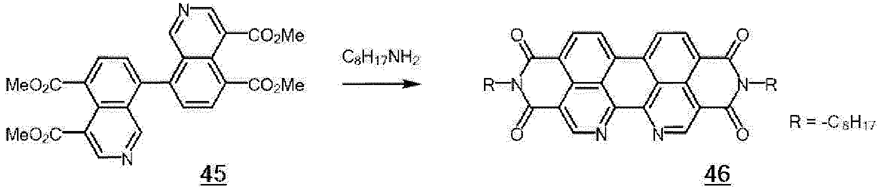

185 mg (0.38 mmol) of the compound 45 was mixed with 0.19 mL (1.14 mmol) of n-octylamine, 103 mg (0.76 mmol) of zinc (II) chloride, and 1.85 g of imidazole in a nitrogen atmosphere, and the mixed solution was heated and stirred at 140°C for 2 hours. The reaction solution was cooled to room temperature, and 5 mL of chloroform was added to the reaction solution. By filtering insoluble matter, the compound 46, which is the specific azaperylene compound, was obtained (yielding amount: 76 mg, yield: 31%). The obtained compound 46 was dispersed in tetrahydrofuran (THF), and the supernatant in which the compound 46 was partially dissolved was identified by a high performance liquid chromatography-mass spectrometry (HPLC-MS) method.

As a result, the calculated value of the molecular weight [M+H] of the compound 46 was 616.8, and the measured value of the molecular weight of the compound 46 by HPLC-MS was 616.7. Since the two values were substantially in agreement, it was confirmed that the compound 46 was synthesized by this example.

### [Example 4]

A compound 47, which is the specific azaperylene compound, was synthesized according to the following synthesis procedure.

### <Synthesis Example 11: synthesis of specific azaperylene compound>

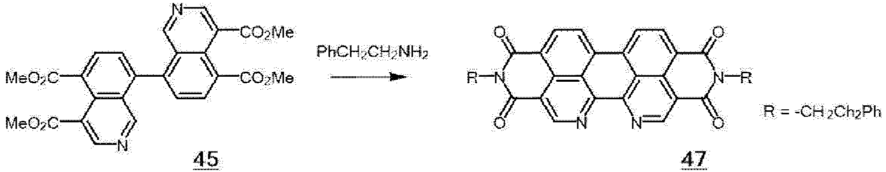

The compound 47 (2,9-diphenethyldiisoquinolino[4,5,6-cde:6',5',4'-ghi][1,10]phenanthroline-1,3,8,10(2H,9H)-tet raone), which is the specific azaperylene compound, was synthesized according to the method described in Synthesis Example 10 of Example 3, except that 138 mg (1.13 mmol) of phenethylamine was used instead of n-octylamine (yielding amount: 75 mg:, yield: 33 %). The obtained compound 47 was dispersed in THF, and the supernatant in which the compound 47 was partially dissolved was identified by the HPLC-MS method.

As a result, the calculated value of the molecular weight [M+H] of the compound 47 was 601.2, and the measured value of the molecular weight of the compound 47 by HPLC-MS was 601.2. Since the two values were substantially in agreement, it was confirmed that the compound 47 was synthesized by this example.

### Explanation of References

1: substrate
2: gate electrode
3: gate insulating film
4A: source electrode
4B: drain electrode
5: organic semiconductor film (organic semiconductor layer)
6: sealing layer
10, 20: organic thin film transistor (organic TFT)
42: substrate
43: member

## Claims

1. A compound represented by Formula (I), 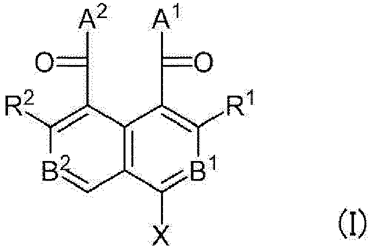 in Formula (I), A¹ and A² each independently represent -OR³ or -NR⁴R⁵,
R³ to R⁵ each independently represent a hydrogen atom, an aliphatic hydrocarbon group which may have a substituent, an aromatic hydrocarbon group which may have a substituent, or a heterocyclic group which may have a substituent,
A¹ and A² may be integrated to form -O- or -N(R⁶)-,
R⁶ represents an aliphatic hydrocarbon group which may have a hydrogen atom or a substituent, an aromatic hydrocarbon group which may have a substituent, or a heterocyclic group which may have a substituent,
B¹ and B² each independently represent -CH= or -N=, where at least one of B¹ or B² represents -N=,
X represents a leaving group or an atomic group having a metal atom or a metalloid atom, which is bonded to a carbon atom in an azanaphthalene ring,
R¹ and R² each independently represent a hydrogen atom or a substituent, and
R¹ and B¹, and R² and B² may be bonded to each other to form a ring.

2. The compound according to claim 1,
wherein A¹ and A² each independently represent -OR³, or are integrated to form -O- or -N(R⁶)-.

3. The compound according to claim 1 or 2,
wherein R¹ and R² represent hydrogen atoms.

4. A compound represented by Formula (II), 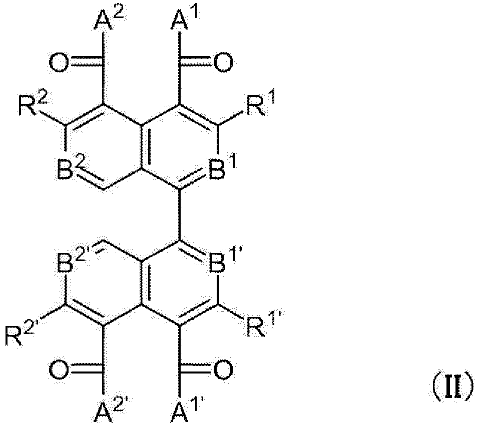 in Formula (II), A¹, A², A^{1'}, and A^{2'} each independently represent -OR³ or -NR⁴R⁵,
R³ to R⁵ each independently represent a hydrogen atom, an aliphatic hydrocarbon group which may have a substituent, an aromatic hydrocarbon group which may have a substituent, or a heterocyclic group which may have a substituent,
A¹ and A², and A^{1'} and A^{2'} may be integrated to form -O- or -N(R⁶)-,
R⁶ represents an aliphatic hydrocarbon group which may have a hydrogen atom or a substituent, an aromatic hydrocarbon group which may have a substituent, or a heterocyclic group which may have a substituent,
B¹, B², B^{1'}, and B^{2'} each independently represent -CH= or -N=, where at least one of B¹ or B² represents -N=,
R¹, R², R^{1'}, and R^{2'} each independently represent a hydrogen atom or a substituent,
and
R¹ and B¹, R² and B², R^{1'} and B^{1'}, and R^{2'} and B^{2'} may be bonded to each other to form a ring.

5. The compound according to claim 4,
wherein A¹, A², A^{1'}, and A^{2'} each independently represent -OR³, or A¹ and A², and A^{1'} and A^{2'} are each independently integrated to form -O- or -N(R⁶)-.

6. The compound according to claim 4 or 5,
wherein R¹, R², R^{1'}, and R^{2'} represent hydrogen atoms.

7. The compound according to any one of claims 4 to 6,
wherein the compound represented by Formula (II) is a compound represented by any one of Formula (II-1), (II-2), (II-3), (II-4), (II-5), (II-6), (II-7), or (II-8), 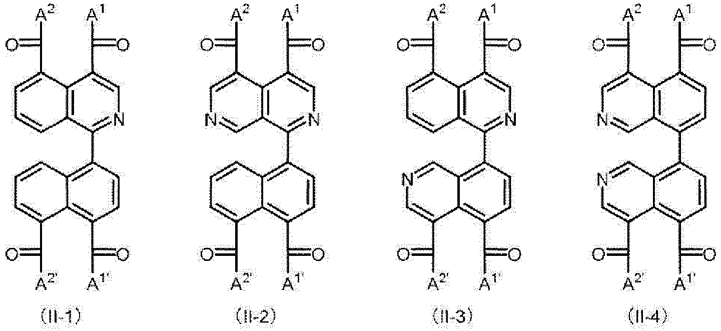 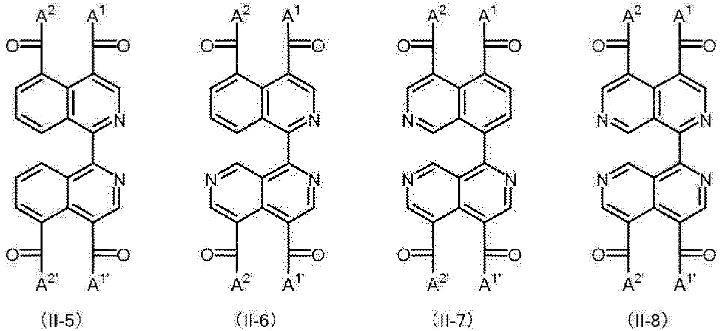 where A¹, A², A^{1'}, and A^{2'} in Formulae (II-1) to (II-8) are the same as A¹, A², A^{1'}, and A^{2'} in Formula (II).

8. A method for producing a compound, comprising:
a step of ring-closing the compound represented by Formula (II) according to any one of claims 4 to 7 to produce an azaperylene compound represented by Formula (IV), 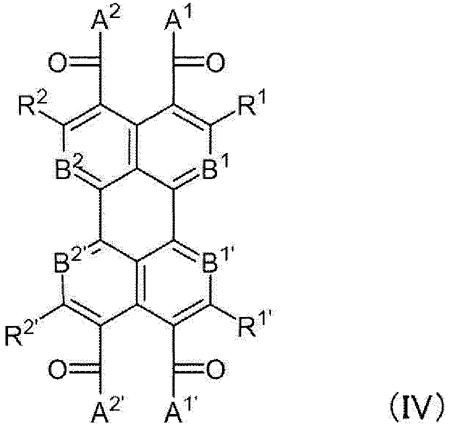 where A¹, A², A^{1'}, A^{2'}, B¹, B², B^{1'}, B^{2'}, R¹, and R² in Formula (IV) are the same as A¹, A², A^{1'}, A^{2'}, B¹, B², B^{1'}, B^{2'}, R¹, and R² in Formula (II).

9. The production method according to claim 8,
wherein the compound represented by Formula (II) is ring-closed in the presence of a base.

10. The production method according to claim 8 or 9, further comprising:
a step of reacting a compound represented by Formula (I) with a compound represented by Formula (III) in the presence of a transition metal catalyst to produce the compound represented by Formula (II), 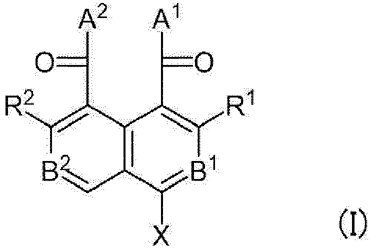 where A¹, A², B¹, B², R¹, and R² in Formula (I) are the same as A¹, A², B¹, B², R¹, and R² in Formula (II), and
X represents a leaving group or an atomic group having a metal atom or a metalloid atom, which is bonded to a carbon atom in an azanaphthalene ring, 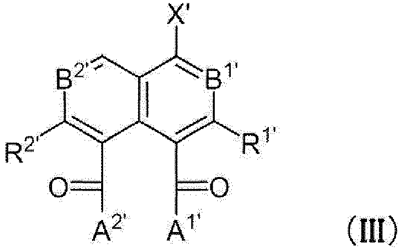 where A^{1'}, A^{2'}, R^{1'}, R^{2'}, B^{1'}, and B^{2'} in Formula (III) are the same as A^{1'}, A^{2'}, R^{1'}, R^{2'}, B^{1'}, and B^{2'} in Formula (II), and
X' represents a hydrogen atom, a leaving group, or an atomic group having a metal atom or a metalloid atom, which is bonded to a carbon atom in an azanaphthalene ring.

11. A method for producing a compound, comprising:
a step of reacting a compound represented by Formula (I) with a compound represented by Formula (III) in the presence of a transition metal catalyst to produce the compound represented by Formula (II) according to any one of claims 4 to 6, 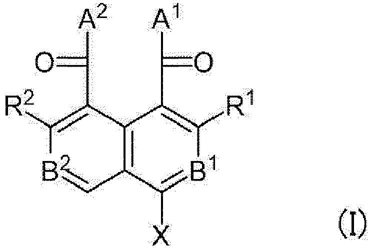 where A¹, A², B¹, B², R¹, and R² in Formula (I) are the same as A¹, A², B¹, B², R¹, and R² in Formula (II), and
X represents a leaving group or an atomic group having a metal atom or a metalloid atom, which is bonded to a carbon atom in an azanaphthalene ring, 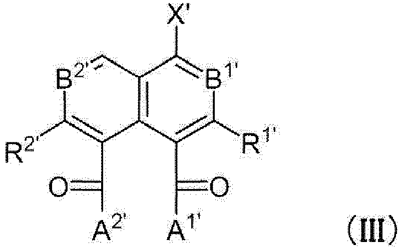
where A^{1'}, A^{2'}, R^{1'}, R^{2'}, B^{1'}, and B^{2'} in Formula (III) are the same as A^{1'}, A^{2'}, R^{1'}, R^{2'}, B^{1'}, and B^{2'} in Formula (II), and
X' represents a hydrogen atom, a leaving group, or an atomic group having a metal atom or a metalloid atom, which is bonded to a carbon atom in an azanaphthalene ring.

12. The production method according to claim 10 or 11,
wherein, in a case where X in Formula (I) represents a leaving group, X' in Formula (III) represents a hydrogen atom or an atomic group having a metal atom or a metalloid atom, which is bonded to a carbon atom in an azanaphthalene ring, and
in a case where X in Formula (I) represents an atomic group having a metal atom or a metalloid atom, which is bonded to a carbon atom in an azanaphthalene ring, X' in Formula (III) represents a leaving group.
